# EUROPEAN PATENT SPECIFICATION

(11) **EP 3 850 004 B1**
(45) Date of publication and mention of the grant of the patent: **18.02.2026**
(21) Application number: 19859765.0
(22) Date of filing: 11.09.2019
(51) Int. Cl.: A61K 40/00, A61K 40/42, A61K 40/15, A61K 40/31, A61K 31/44, A61K 38/17, C07K 14/705, C12N 5/00, A61P 35/04, A61K 45/06, A61K 35/17, A61K 35/00, A61K 39/00, C12N 5/0783, A61K 38/05, A61P 35/00

(54) **NATURAL KILLER CELL COMPOSITIONS AND IMMUNOTHERAPY METHODS FOR TREATING A LIVER TUMOR**
NATÜRLICHE KILLERZELLENZUSAMMENSETZUNGEN UND IMMUNTHERAPIEVERFAHREN ZUR BEHANDLUNG EINES LEBERTUMORS
COMPOSITIONS DE CELLULES TUEUSES NATURELLES ET MÉTHODES D'IMMUNOTHÉRAPIE POUR TRAITER UNE TUMEUR DE FOIE

(30) Priority: 13.09.2018 US 201862730939 P; 20.03.2019 US 201962821360 P; 01.05.2019 US 201962841768 P; 18.07.2019 US 201962875893 P
(43) Date of publication of application: 21.07.2021
(73) Proprietor: Nkarta, Inc., South San Francisco, CA 94080 (US)
(72) Inventor: TRAGER, James Barnaby, South San Francisco, California 94080 (US); SHEN, Angela Jean, South San Francisco, California 94080 (US)
(74) Representative: Witte, Weller & Partner Patentanwälte mbB
(86) International application number: PCT/US2019/050679
(87) International publication number: WO 2020/056045

(56) References cited:
- WO-A1-2016/044605
- WO-A1-2017/079705
- WO-A1-2018/049248
- WO-A1-2018/160993
- WO-A2-2017/069958
- WO-A2-2018/124766
- US-A1- 2005 255 118
- US-A1- 2015 273 089
- US-A1- 2016 030 659
- US-A1- 2016 333 108
- US-A1- 2017 073 638
- US-A1- 2017 107 178
- US-A1- 2017 260 594
- US-A1- 2018 135 015
- US-A1- 2018 186 878
- KAWALEKAR OMKAR UDAY ET AL: "Distinct Signaling By Chimeric Antigen Receptors (CARs) Containing CD28 Signaling Domain Versus 4-1BB In Primary Human T Cells", BLOOD, AMERICAN SOCIETY OF HEMATOLOGY, US, vol. 122, no. 21, 15 November 2013 (2013-11-15), pages 2902, XP086753236, ISSN: 0006-4971, [retrieved on 20210707], DOI: 10.1182/BLOOD.V122.21.2902.2902
- JENNIFER T. GRIER ET AL: "Human immunodeficiency-causing mutation defines CD16 in spontaneous NK cell cytotoxicity", THE JOURNAL OF CLINICAL INVESTIGATION, vol. 122, no. 10, 1 October 2012 (2012-10-01), GB, pages 3769 - 3780, XP055218451, ISSN: 0021-9738, DOI: 10.1172/JCI64837
- PITTARI GIANFRANCO ET AL: "Revving up Natural Killer Cells and Cytokine-Induced Killer Cells Against Hematological Malignancies", FRONTIERS IN IMMUNOLOGY, vol. 6, 13 May 2015 (2015-05-13), XP055911635, DOI: 10.3389/fimmu.2015.00230

## Description

### RELATED CASES

### BACKGROUND

Liver cancers are common, among the leading types of cancers. Hepatocellular carcinoma is one of the most common types of liver cancer. In contrast to certain types of liver cancer that are secondary (e.g., originating in another tissue and migrating to the liver, hepatocellular carcinoma is a primary cancer (e.g., originates in the liver). Other types of liver cancer, such as a liver tumor due to metastasis from colorectal carcinoma are also common.

WO 2018/160993 A1 discloses a composition for inducing an immune response comprising a first effector module, said effector module comprising a first stimulus response element (SRE) operably linked to at least one immunotherapeutic agent, for example a chimeric antigen receptor (CAR). Upon stimulation of the SRE, which can be a destabilizing domain (DD), the immunotherapeutic agent takes action in inducing an immune response.

Kawalekar Omkar Uday et al. (Kawalekar Omkar Uday et al. "Distinct Signaling By Chimeric Antigen Receptors (CARs) Containing CD28 Signaling Domain Versus 4-1BB In Primary Human T Cells", Blood, American Society of Hematology, US, vol. 122, no. 21, 15 November 2013, page 2902, XP086753236, ISSN: 0006-4971, DOI: 10.1182/BLOOD.V122.21.2902.2902) discloses that CARs with CD28 show stronger activation of T cells when compared to CARs with 4-1BB or CD3z alone and the antigen-specific activation threshold for CAR-T cells is greatly reduced when the CD28 is included as well as direct interaction of CARs with pZAP70 and TRAF proteins.

Jennifer T. Grier et al. (Jennifer T. Grier et al.: "Human immunodeficiencycausing mutation defines CD16 in spontaneous NK cell cytotoxicity", The Journal of Clinical Inverstigation, vol. 122, no. 10, 1 October 2012, pages 3769-3780, XP055218451, GB ISSN: 0021-9738, DOI: 10.1172/JCI64837) discloses the FcγRIIIA, also denoted CD16, to associate with CD2 on NK cells, is localized to the immunological synapse, and is important to maintain surfaceexpression of CD2. The complex from CD2 and CD16 is disclosed to play an important role in spontaneous NK cell cytotoxicity.

US 2018/135015 A1 discloses NK cells engineered to express a polynucleotide comprising (a) an extracellular ligand binding domain coding for natural killer (NK) cell activation, (b) the transmembrane and stimulatory domains of DNAX-activating protein 10 (DAP10) and (c) the signalling domain CD3zeta, which boosts the anti-cancer potential of NK cells. Such NK cells can be administered locally, also in combination with radiation therapy or chemotherapy.

WO 2018/124766 A2 discloses NK cells engineered to express a CAR comprising an intracellular signaling domain comprising all or a part of OX40, which enhances anti-cancer activity of the NK cells.

WO 2017/079705 A1 discloses T cells engineered to express CAR, which receptors comprise a TRAF signaling inducing intracellular signaling domain, as for example the one from CD40.

US 2017/073638 A1 discloses cells, especially NK cells, engineered to express a membrane bound IL15 or a membrane-bound derivative of IL15 and its use in treating cancer. Usually, IL2 is administered together with NK cells that exhibit anti-cancer activity to prolong the life span of the NK cells. However, IL2 has a lot of negative side-effects. Such engineered NK cells advantageously allow to lower the dose of IL2 to be administered together with the respective NK cells to a cancer patient or to fully refrain from administering IL2.

### MATERIAL IN ASCII TEXT FILE

It is referred to the Sequence Listing contained in the following ASCII text file being submitted concurrently herewith: File name: NKT025WO_ST25; created September 10, 2019, 90.0KB in size.

### SUMMARY

The present invention is defined in the appended claims. In several embodiments, there are provided for herein compositions for use in treating tumors. In particular, in several embodiments, the compositions are delivered locally to the tumor. Disclosed herein is also a method for treating a primary liver tumor, the method comprising, accessing the intra-hepatic artery of a patient with a primary liver tumor, and administering a population of natural killer (NK) cells engineered to express a cytotoxic receptor complex. In several embodiments, NK cells bearing engineered receptors, such as a CAR or chimeric receptor as disclosed herein bind to an NK ligand on a tumor cell. Cytotoxic signals are then generated which result in killing of tumor cells.

Several embodiments disclosed herein relate to combination therapy regimens for use in treating liver cancers. Several embodiments employ a combination of an engineered immune cell (e.g., a Natural Killer cell or a T cell) that expresses a chimeric receptor or chimeric antigen receptor and another anti-cancer agent. Depending on the embodiment, various anticancer agents are used, including, for example, such as a histone deacetylase inhibitor, kinase (e.g., tyrosine kinase) inhibitors, inhibitors of epidermal growth factor receptor signaling; inhibitors of vascular endothelial growth factor receptor signaling, chemotherapeutic agent, a proteasome inhibitor, radiation, demethylating agent, tyrosine kinase inhibitor, all trans retinoic acid, sodium butyrate, and the like. In several embodiments, synergistic anti-cancer effects result from the combination.

In several embodiments, there is provided a combination therapy regimen for use in the treatment of a liver tumor, the therapy regime comprising (i) a population of natural killer (NK) cells engineered to express a cytotoxic receptor complex, and (ii) at least one additional anticancer agent.

In several embodiments, the therapy regimen is configured for the population of NK cells to be administered locally to the liver tumor.

In several embodiments, the cytotoxic receptor complex comprises a functional fragment of a C-type lectin-like receptor, wherein the C-type lectin-like receptor comprises a Natural Killer Group 2D (NKG2D) receptor and a cytotoxic signaling complex comprising a transmembrane domain, a co-stimulatory domain, and a signaling domain. In several embodiments, the functional fragment of the C-type lectin-like receptor comprises SEQ ID NO. 24, a fragment thereof having a high degree of sequence identity (e.g., about 85%, about 90%, about 95%, about 96%, about 97%, about 98%, or about 99% sequence identity), or a fragment thereof that retains at least a portion of the ligand binding function the fragment of the C-type lectin-like receptor comprises SEQ ID NO. 24.

In several embodiments, the liver tumor to be treated by the therapy regimen results from hepatocellular carcinoma. In several embodiments, the liver tumor is secondary to a tumor in a different location. For example, in several embodiments, the liver tumor is a primary metastasis of colorectal carcinoma.

In several embodiments, the signaling domain of the cytotoxic signaling complex comprises an OX40 domain. In several embodiments the OX40 domain is at least 95% identical to an OX40 domain having the sequence of SEQ ID NO. 35. In several embodiments, the OX40 domain can be truncated to a sequence shorter than that of SEQ ID NO. 35, yet still retains stimulatory function. In several embodiments, the signaling construct comprises two, three, four or more OX40 domains, which advantageously enhances NK cell activation. In several embodiments, the signaling domain of the cytotoxic signaling complex further comprises a CD3zeta domain. In several embodiments, the CD3zeta domain is at least 95% identical to a CD3zeta domain having the sequence of SEQ ID NO. 32. In several embodiments, the CD3zeta domain is truncated or modified to enhance signaling and/or packaging of the cytotoxic receptor complex (e.g., in a vector). For example, in several embodiments, signaling domain comprises a hemi-ITAM domain. In several embodiments, the cytotoxic receptor complex further comprises IL15. In several embodiments, the IL15 comprises membrane-bound IL15 (mbIL15). In several embodiments, the mbIL15 is at least 95% identical to a mbIL15 having the sequence of SEQ ID NO. 39. In several embodiments, the mbIL15 is truncated and/or mutated, for example in order to enhance stimulation of the engineered NK cells. In several embodiments, the mbIL15 is encoded by a polynucleotide that also encodes the remainder of the cytotoxic receptor complex. In several embodiments, the mbIL15 is provided on a separate polynucleotide, that is separately introduced into NK cells along with the polynucleotide encoding the cytotoxic receptor complex.

In several embodiments, the cytotoxic receptor complex is encoded by the nucleic acid sequence of SEQ ID NO: 7. In several embodiments, the cytotoxic receptor complex is encoded by a nucleic acid sequence that is about 80%, about 85%, about 90%, about 91%, about 92%, about 93%, about 94%, or about 95% identical to SEQ ID NO: 7. In several embodiments, the cytotoxic receptor complex comprises the amino acid sequence of SEQ ID NO: 8. Depending on the embodiment the cytotoxic receptor complex is about 80%, about 85%, about 90%, about 91%, about 92%, about 93%, about 94%, or about 95% identical to SEQ ID NO: 8.

In several embodiments, the regimen is configured for the at least one additional anti-cancer agent to be administered prior to, concurrent with, or after the population of NK cells is administered. In several embodiments, the additional anti-cancer agent is administered more frequently than the engineered NK cells, for example, the anti-cancer agent is administered daily, while the NK cells are administered one, or weeks apart. In several embodiments, the additional anti-cancer agent works synergistically with the engineered NK cells as result of the additional anti-cancer agent inducing expression of one or more ligands of the receptor portion of the cytotoxic receptor complex and/or because it further stimulates NK cell activity or longevity.

In several embodiments, the additional anti-cancer agent is a tyrosine kinase inhibitor. In several embodiments, the agent is sorafenib. In some embodiments, the anti-cancer agent is sorafenib, nilotinib, imantinib, gefitinib, erlotinib, sunitinib, adavosertib, lapatinib, or combinations thereof (e.g., combinations of two, three, four or more).

In several embodiments, the additional anti-cancer agent is an antibody. For example, in several embodiments the antibody is a monoclonal antibody, scFv or antibody fragment. In several embodiments, the additional anti-cancer agent is cetuximab, daratumumab, rituximab, obinutuzumab, trastuzumab, or combinations thereof. In several embodiments, the additional anti-cancer agent is a histone deacetylase (HDAC) inhibitor. Various HDAC inhibitors can be used. For example, in several embodiments the HDAC inhibitor is valproic acid, FR901228, MS-275, phenylbutyrate, PDX101, sodium valproate, suberoylanilide hydroxamic acid, or combinations thereof.

Depending on the embodiment, more widely-used anti-cancer agents can be coupled with the engineered NK cells disclosed herein. In several embodiments, the additional anti-cancer agent is a chemotherapeutic agent. For example, in several embodiments, the chemotherapeutic agent is cisplatin, hydroxyurea, 5-fluorouracil, doxorubicin, melphalan, mitomycin C, etoposide, or combinations thereof. In addition to, or in place of, some embodiments, employ high dose ionizing radiation. In several embodiments, this is particularly effective when combined with local delivery of the engineered NK cells, as both treatments are focally delivered.

In several embodiments, the additional anti-cancer agent is a proteasome inhibitor. In several embodiments, the proteasome inhibitor is bortezomib. In several embodiments, the additional anti-cancer agent is all trans retinoic acid, sodium butyrate, or a combination thereof. In several embodiments, the additional anti-cancer agent is an inhibitor of a NK checkpoint pathway. In such embodiments, the inhibitor acts to block the immunosuppressive nature of NK checkpoint pathways, thereby enhancing the activity of NK cells (both endogenous and engineered). In several embodiments, the NK checkpoint inhibitor is an antagonist of CD73, an antagonist of CD39, an antagonist of an adenosine immunosuppressive signaling pathway, or combinations thereof. Likewise, other approaches are used to enhance NK cell activity, in several embodiments, such as antibodies directed to TIM3 and/or a TGF beta receptor. In still additional embodiments, additional anti-cancer agent is an inhibitor of indoleamine 2,3-dioxygenase. In several embodiments, combinations of additional agents are used, such as radiation in combination with NK checkpoint inhibitors and the engineered NK cells. In such embodiments, the utilization of different mechanisms of action and different signaling pathways leads to a synergistic cytotoxic effect on the target tumor cells. In some embodiments, two or more of the following are combined: tyrosine kinase inhibitors, antibodies, chemotherapeutic agents, HDAC inhibitors, demethylating agents, proteasome inhibitors, checkpoint inhibitors, and other anti-cancer or anti-proliferative agents.

In several embodiments, there is provided a use of the combination therapies disclosed herein for the treatment of a liver cancer. In several embodiments, there is provided a use of the combination therapies disclosed herein for the preparation of a medicament for the treatment of a liver cancer.

In several embodiments, the cytotoxic receptor complex comprises a functional fragment of a C-type lectin-like receptor, and a cytotoxic signaling complex comprising a transmembrane domain, a co-stimulatory domain, and a signaling domain. In several embodiments, the C-type lectin-like receptor comprises a Natural Killer Group 2D (NKG2D) receptor. In several embodiments, the functional fragment of the C-type lectin-like receptor comprises SEQ ID NO. 24.

In several embodiments, there is also provided a population of natural killer (NK) cells engineered to express a cytotoxic receptor complex for use in a method for treating a liver tumor, the method comprising administering to a patient with a liver tumor a composition comprising a population of NK cells engineered to express a cytotoxic receptor complex, wherein the cytotoxic receptor complex comprises an extracellular domain that binds a ligand of a Natural Killer Group 2D (NKG2D) receptor, a cytotoxic signaling complex comprising a transmembrane domain, a co-stimulatory domain, and a signaling domain, and wherein the administration comprises an injection into the intra-hepatic artery of the patient. In several embodiments, the extracellular domain is encoded by at least a portion of the polynucleotide of SEQ ID NO. 23. In several embodiments, the extracellular domain is a portion of the extracellular domain of a wild-type NKG2D receptor. Surprisingly, in several embodiments, the portion of the extracellular domain retains the ability to bind a ligand of the NKG2D receptor. Also, according to several embodiments, accessory proteins are not needed for efficient expression of the extracellular domain of the NKG2D receptor. For example, in several embodiments, DAP10 is not required for efficient NKG2D extracellular domain expression and/or function. Likewise, in several embodiments, DAP12 is not required for efficient NKG2D extracellular domain expression and/or function.

In several embodiments, the ligand of NKG2D is selected from MICA, MICB, ULBP1, ULBP2, ULBP3, ULBP4, ULBP5, ULBP6, and combinations thereof. Other tumor associated ligands can be bound, in some embodiments. In several embodiments, the NKG2D receptor is a fragment of a human NKG2D receptor. In alternative embodiments, non-human sequences may be used.

In several embodiments, there is provided a population of natural killer (NK) cells engineered to express a cytotoxic receptor complex for use in a method for treating hepatocellular carcinoma, the method comprising administering to a patient with a hepatocellular carcinoma a composition comprising a population of natural killer (NK) cells engineered to express a cytotoxic receptor complex, wherein the cytotoxic receptor complex comprises an extracellular domain coupled to a transmembrane domain, the transmembrane domain further coupled to a signaling domain, wherein the extracellular domain comprises a fragment of a Natural Killer Group 2D (NKG2D) receptor, wherein the fragment the NKG2D receptor is at least 95% homologous to the NKG2D receptor fragment encoded by the polynucleotide of SEQ ID NO. 24, and wherein the administration comprises an injection into the intra-hepatic artery of the patient.

In several embodiments, the signaling domain of the cytotoxic signaling complex comprises an OX40 domain. In several embodiments, the signaling domain of the cytotoxic signaling complex further comprises a CD3zeta domain. Depending on the embodiment, the CD3zeta domain comprises at least one immunoreceptor tyrosine-based activation motif (ITAM) motif. In some embodiments, no ITAM motif is present. In several embodiments, the co-stimulatory domain comprises IL-15. In several embodiments, the IL-15 is expressed by the NK cells as membrane-bound IL-15. In several embodiments, the membrane bound IL-15 is supplemented by expression of secreted IL-15. In several embodiments, alternative interleukins may be used in place of, or in addition to IL-15.

In several embodiments, the transmembrane domain of the cytotoxic signaling complex is derived from CD8 alpha. In several embodiments, the transmembrane domain comprises a first and a second subdomain. In some such embodiments, the first subdomain comprises a CD8 alpha transmembrane domain. In some such embodiments, the second subdomain comprises a CD8 alpha hinge.

In several embodiments, the cytotoxic receptor complex is at least 80% homologous to the cytotoxic receptor complex encoded by the polynucleotide of SEQ ID NO. 7. In several embodiments, the cytotoxic receptor complex is at least 85% homologous to the cytotoxic receptor complex encoded by the polynucleotide of SEQ ID NO. 7. In several embodiments, the cytotoxic receptor complex is at least 90% homologous to the cytotoxic receptor complex encoded by the polynucleotide of SEQ ID NO. 7. In several embodiments, the cytotoxic receptor complex is at least 95% homologous to the cytotoxic receptor complex encoded by the polynucleotide of SEQ ID NO. 7. In several embodiments, the cytotoxic receptor complex is at least 80% homologous to a cytotoxic receptor complex having the amino acid sequence of SEQ ID NO. 8. In several embodiments, the cytotoxic receptor complex is at least 90% homologous to a cytotoxic receptor complex having the amino acid sequence of SEQ ID NO. 8. In several embodiments, the cytotoxic receptor complex is at least 80% homologous to a cytotoxic receptor complex encoded by a polynucleotide of selected from the group consisting of SEQ ID NOs. 1, 3, 5, 9, 11, 13, 15, 17, 19, and 21. In still additional embodiments, the cytotoxic receptor complex may have less homology to the indicated sequences, but retains all or substantially all of the same functionality as the reference receptor complexes.

In several embodiments, the cells/compositions described herein are administered percutaneously. In several embodiments, the intra-hepatic artery is accessed percutaneously and wherein the population of cells is administered by infusion. In one embodiment, the liver is targeted via needle-puncture of the skin instead of using an open surgery approach. In one embodiment, a catheter system is used. Over-the-wire systems are used in some embodiments. Tumors (e.g., solid tumors) or other sites outside the hepatic system are targeted in several embodiments.

In one embodiment, a needle is placed through the skin and into a vessel, such as an artery or vein. An introducer such as a guide wire is introduced into the vessel. The needle is then exchanged for a conduit or sheath that is advanced over the introducer and into the vessel. The introducer is removed, and exchanged for a catheter or other medical device to be used to deliver the cells/compositions described herein. Catheters may be partially or wholly flexible to navigate tortuous vessels. Catheters may be adapted to the shape and size of the hepatic artery or portal vein, in some embodiments. In one embodiment, the cells/compositions are delivered over a period of time. In another embodiment, the cells/compositions are delivered in a single bolus. In yet other embodiments, the cells/compositions are delivered on a substrate. In some embodiments, image or other guidance is used to facilitate delivery. In some embodiments, the cells/compositions are used for targeted localized delivery such that at least 95%, 97%, 99% or 100% of the cells/compositions are delivered and remain at the target location. In one embodiment, only the hepatic artery is targeted, and not the portal vein. In several embodiments, the NK cells expressing chimeric receptors are administered via trans-arterial delivery (e.g., trans-arterial delivery to the hepatic artery).

Non-percutaneous administration is also provided in several embodiments. In some embodiments, cells and compositions are delivered intravenously, intramuscularly, intraperitoneally, though an artery, and/or via an open surgery approach. In some embodiments, tumors located in areas other than the liver are treated (such as the ovaries, pancreas, renal, and other organs). While NK cells and compositions are described herein, other cells or biologics/drugs may be delivered instead of in addition to NK cells (including but not limited to CAR T cells, antibodies, epidermal growth factor receptor inhibitors, antineoplastics, etc). In some embodiments, one or more of cetuximab, daratumumab, rituximab, obinutuzumab, and trastuzumab are provided in combination with NK cells. In several embodiments, one or more tyrosine kinase inhibitors are provided in combination with the engineered NK cells disclosed herein. For example, in several embodiments, sorafenib is administered in conjunction with the engineered NK cells disclosed herein. In several embodiments, sunitinib is administered in conjunction with the engineered NK cells disclosed herein. In several embodiments, one or more chemotherapy agents are administered in conjunction with the engineered NK cells disclosed herein. In several embodiments, one or more of cisplatin, hydroxyurea, 5-fluorouracil, doxorubicin, melphalan, mitomycin C, and/or etoposide are administered in conjunction with the engineered NK cells disclosed herein. In several embodiments, high dose ionizing radiation is administered in conjunction with the engineered NK cells disclosed herein. In several embodiments, one or more proteasome inhibitors are administered in conjunction with the engineered NK cells disclosed herein. In several embodiments, bortezomib (e.g., Velcade^{®}) is administered in conjunction with the engineered NK cells disclosed herein. In several embodiments, one or more HDAC inhibitors are administered in conjunction with the engineered NK cells disclosed herein. In several embodiments, one or more of remidepsin (e.g., FR901228), entinostat (e.g., MS-275), phenylbutyrate, belinostat (e.g., PDX101), sodium valproate, suberoylanilide hydroxamic acid, and/or valproic acid is administered in conjunction with the engineered NK cells disclosed herein. In several embodiments, one or more of all-trans retinoic acid and/or sodium butyrate is administered in conjunction with the engineered NK cells disclosed herein. In several embodiments, one or more demethylating agents is administered in conjunction with the engineered NK cells disclosed herein. For example, in several embodiments, azazytidine and/or decitabine is administered in conjunction with the engineered NK cells disclosed herein. In several embodiments, the additional agent administered renders the target tumor cells more susceptible to the engineered NK cells, for example by inducing upregulation of ligands of the engineered receptors disclosed herein.

In some embodiments, delivery of the cells/compositions described herein are administered with other diagnostic and/or therapeutic modalities. For example, tumor ablation (e.g., cryotherapy), chemotherapy, dissection etc. may be administered as additional therapies. In some embodiments, trans-arterial chemoembolization, ultrasound ablation, microwave ablation, laser therapy, gamma-knife radiosurgery, ethanol injection, and/or radioembolization are provided. In one embodiment, the cells/compositions enhance the efficacy of these other therapies, reduce the undesired side effects and/or reduce the treatment time. Techniques to increase delivery, localization and/or efficacy are provided in some embodiments, including but not limited to techniques that enhance cell membrane permeability, such as electroporation.

In several embodiments, the administered population of NK cells is autologous with respect to the patient. In alternative embodiments, the administered population of NK cells is allogeneic with respect to the patient. NK cells are innate immune sensors of virally infected, damaged, and malignant cells. NKs are not restricted to a single epitope; they read a wide variety of activating and inhibitory signals to spare healthy and kill diseased cells. Also, NK cell proliferation signals (e.g., in response to IL15, IL18, IL21, IL2, IL21) are distinct from the signals that drive cytotoxicity (e.g., signaling through NKG2D, NKG2C, CD16, DNAM-1, 2B4, NKp44, and/or NKp30). In several embodiments, the use of engineered NK cells according to embodiments disclosed herein is advantageous because such engineered NK cells: (i) are able to discriminate between healthy cells and cancer cells, (ii) are able to target multiple antigens, (iii) are able to target native ligands that are overexpressed by many tumor cells, (iv) are able to target checkpoint inhibitors, and/or (v) are able to reduce and/or eliminate risk of graft versus host disease. In several embodiments, the engineered cells according to embodiments disclosed herein have the ability to withstand cryopreservation with limited cell death. In several embodiments, this makes an off the shelf product possible. In still additional embodiments, the administered population is a mixed autologous/allogeneic population. In some embodiments, T cells are administered in conjunction with or in place of NK cells. In several embodiments, therapy regimens for use in the treatment methods further comprise administering IL-2 to the patient.

In several embodiments, the therapy regimens for use in the methods further comprise mapping the location of the intra-hepatic artery prior to accessing the intra-hepatic artery. Several embodiments employ steps to occlude blood vessels that do not supply blood to the liver. Depending on the embodiments, the administration of the population of NK cells is repeated once every 1 week, once every 2 weeks, once every 3 weeks, or once every 4 weeks. In several embodiments, the administered composition results in at least a 25% decrease in the tumor burden. In several embodiments, the reduced tumor burden is reduction in hepatocellular carcinoma tumor burden. In several embodiments, the administered composition comprises between about 107 and about 1010 NK cells.

In several embodiments, the signaling domain of the cytotoxic signaling complex comprises a CD3zeta domain and the extracellular domain is encoded by the polynucleotide of SEQ ID NO. 24. In several embodiments, the CD3zeta domain comprises at least one immunoreceptor tyrosine-based activation motif (ITAM) motif. In several embodiments, the signaling domain of the cytotoxic signaling complex further comprises an OX40 domain. In several embodiments, the cytotoxic signaling complex further comprises a 4-1BB domain. In several embodiments, the signaling domain of the cytotoxic signaling complex further comprises a CD28 domain. In several embodiments, the signaling domain of the cytotoxic signaling complex further comprises an NKp80 domain.

In several embodiments, the cytotoxic receptor complex is encoded by the nucleic acid sequence of SEQ ID NO. 1. In several embodiments, the cytotoxic receptor complex has the amino acid sequence of SEQ ID NO. 2. In several embodiments, the cytotoxic receptor complex is encoded by the nucleic acid sequence of SEQ ID NO. 3. In several embodiments, the cytotoxic receptor complex has the amino acid sequence of SEQ ID NO. 4. In several embodiments, the cytotoxic receptor complex is encoded by the nucleic acid sequence of SEQ ID NO. 5. In several embodiments, the cytotoxic receptor complex has the amino acid sequence of SEQ ID NO. 6. In several embodiments, the cytotoxic receptor complex is encoded by the nucleic acid sequence of SEQ ID NO. 7. In several embodiments, the cytotoxic receptor complex has the amino acid sequence of SEQ ID NO. 8. In several embodiments, the cytotoxic receptor complex is encoded by the nucleic acid sequence of SEQ ID NO. 9. In several embodiments, the cytotoxic receptor complex has the amino acid sequence of SEQ ID NO. 10. In several embodiments, the cytotoxic receptor complex is encoded by the nucleic acid sequence of SEQ ID NO. 11. In several embodiments, the cytotoxic receptor complex has the amino acid sequence of SEQ ID NO. 12. In several embodiments, the cytotoxic receptor complex is encoded by the nucleic acid sequence of SEQ ID NO. 13. In several embodiments, the cytotoxic receptor complex has the amino acid sequence of SEQ ID NO. 14. In several embodiments, the cytotoxic receptor complex is encoded by the nucleic acid sequence of SEQ ID NO. 15. In several embodiments, the cytotoxic receptor complex has the amino acid sequence of SEQ ID NO. 16. In several embodiments, the cytotoxic receptor complex is encoded by the nucleic acid sequence of SEQ ID NO. 17. In several embodiments, the cytotoxic receptor complex has the amino acid sequence of SEQ ID NO. 18. In several embodiments, the cytotoxic receptor complex is encoded by the nucleic acid sequence of SEQ ID NO. 19. In several embodiments, the cytotoxic receptor complex has the amino acid sequence of SEQ ID NO. 20. In several embodiments, the cytotoxic receptor complex is encoded by the nucleic acid sequence of SEQ ID NO. 21. In several embodiments, the cytotoxic receptor complex has the amino acid sequence of SEQ ID NO. 22.

The disclosure above and set forth in further detail below describe certain actions taken by a practitioner; however, it should be understood that they can also include the instruction of those actions by another party. Thus, actions such as "administering a population of engineered NK cells locally to the liver" include "instructing the administration of a population of engineered NK cells locally to the liver."

### BRIEF DESCRIPTION OF THE FIGURES

Figures 1A-1G represent schematic depictions of cytotoxic receptor complex constructs for expression in NK cells according to several embodiments disclosed herein.
Figures 2A-2D represent schematic depictions of additional cytotoxic receptor complex constructs for expression in NK cells according to several embodiments disclosed herein.
Figures 3A to 3C depict data related to the concentration of various factors released by cultured hepatocellular carcinoma cells when co-cultured with native NK cells (NT NK) or engineered NK cells according to embodiments disclosed herein (NKX101).

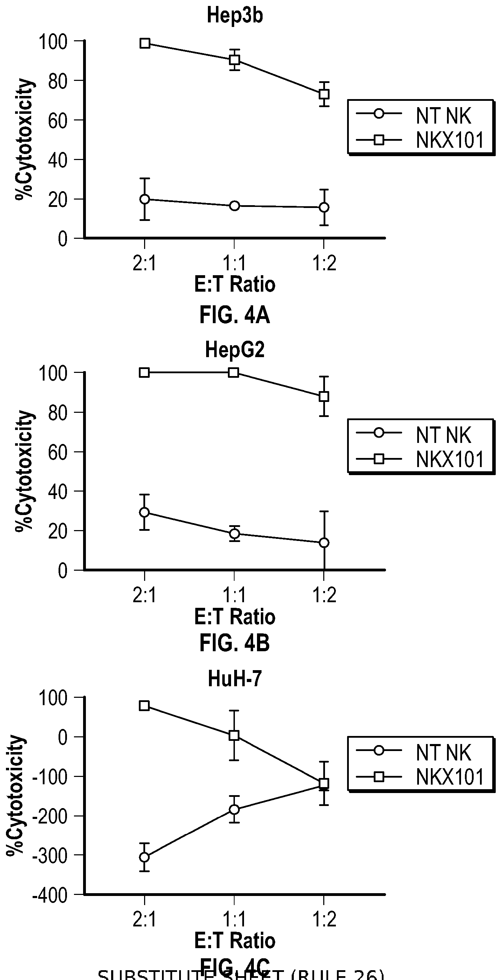

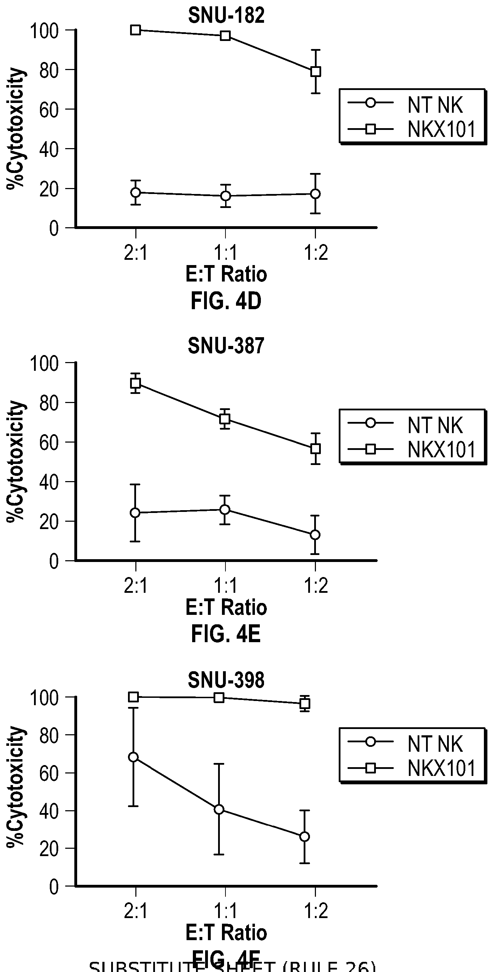

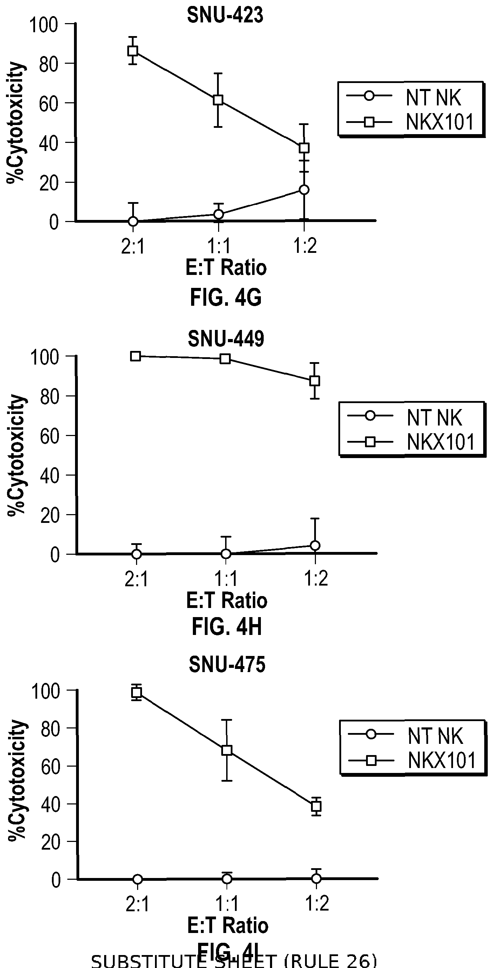

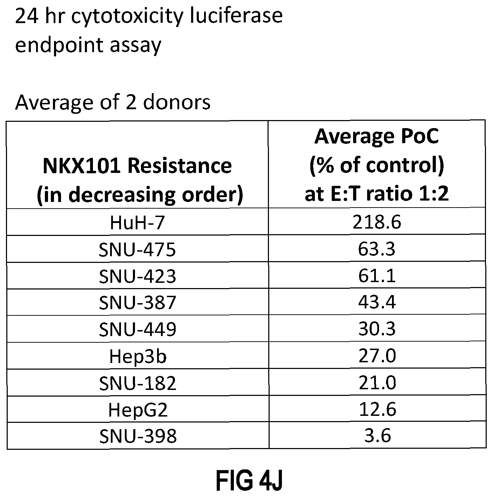

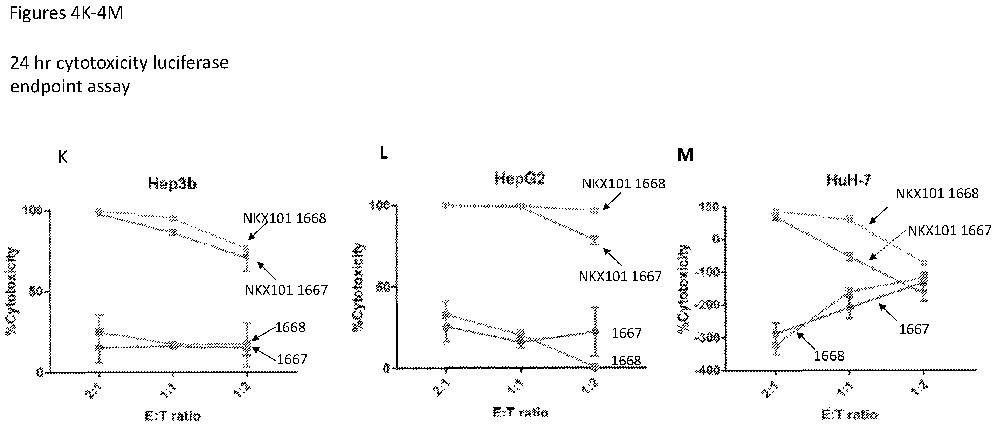

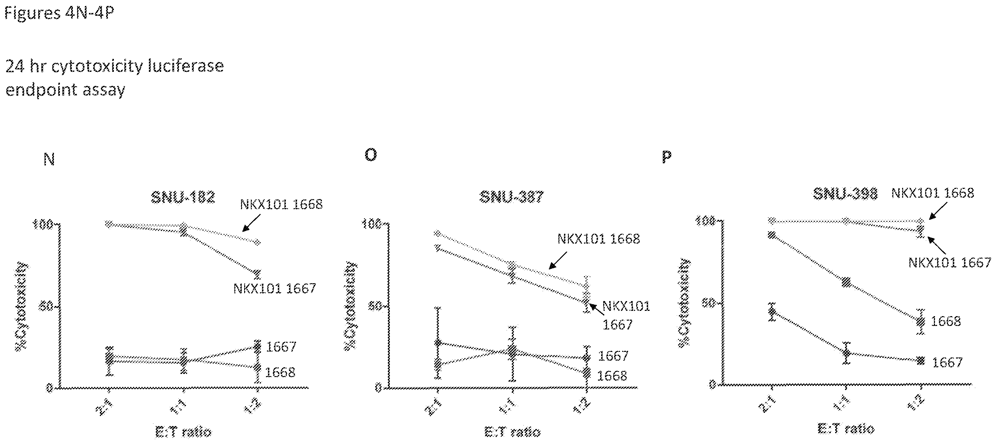

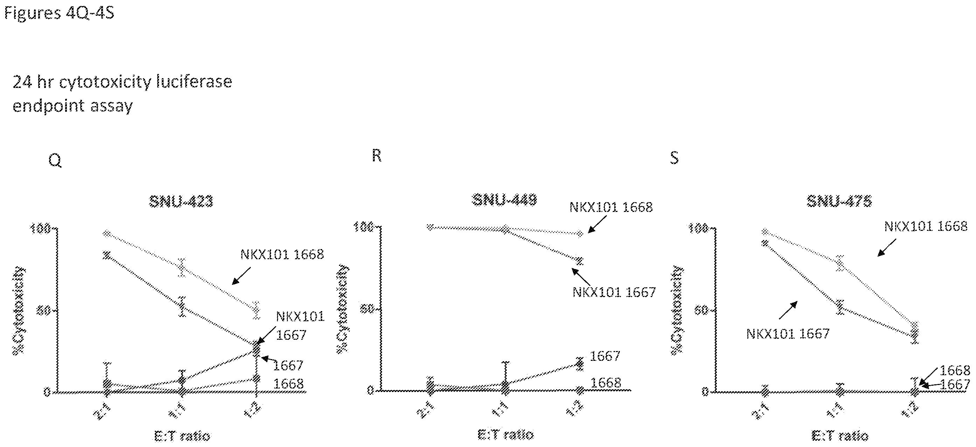

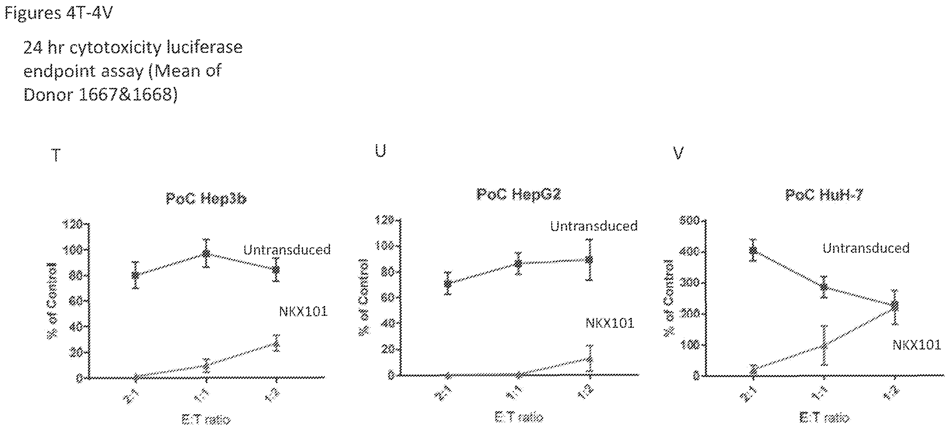

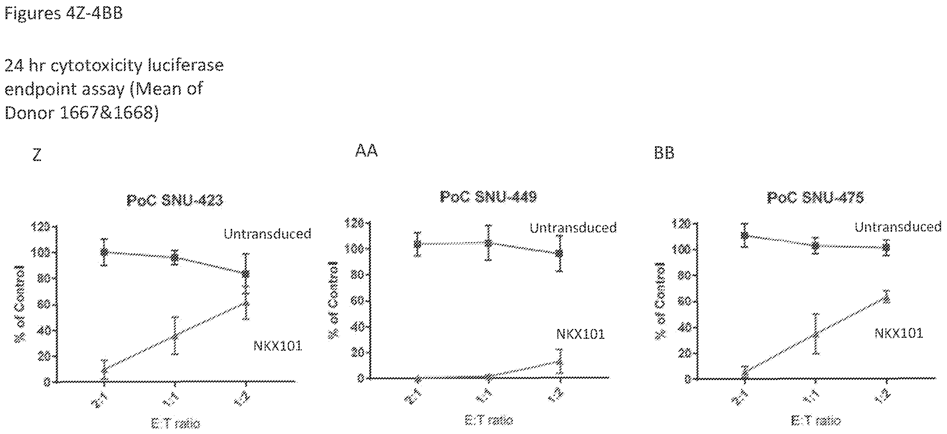

Figures 4A-4J show data related to the degree of cytotoxic effects induced by native NK cells (NT NK) or engineered NK cells according to embodiments disclosed herein (NKX101) when co-cultured with various hepatocellular carcinoma cell lines at varying effector:target ratios. Figure 4J summarizes the cell lines in terms of their resistance to NKX101 as measured by the percentage of control cells still viable at an effector to target ratio of 1:2. Figures 4K-4S depict additional data related to the degree of cytotoxic effects induced by native NK cells (donor number) or engineered NK cells according to embodiments disclosed herein (NKX101 (plus donor number)) when co-cultured with various hepatocellular carcinoma cell lines at varying effector:target ratios. Figures 4T-4BB reflect summary data for those traces shown in Figures 4F-4S. The Y-axis shows Percent of Control (PoC), based on the number of untreated HCC cells (calculated as [(mean experimental value)/(control mean)*100].
Figure 5 shows a schematic of a protocol for in vivo evaluation of NKX101 in reducing hepatocellular carcinoma tumor burden in mice. 3x10⁶ cells (either unmodified NK or NKX101) or PBS was administered IP to mice receiving hepatocellular carcinoma cells (SNU449 cells with firefly luciferase) at 8 days post-HCC administration. NKX101 transduction efficiency was ~57%, while residual CD3+ T cells were less than 2%.
Figures 6A-6F show data related to tumor burden in mice receiving PBS (A and D), unmodified NKs (B and E) or NKX101 (C and F). Shown in A, B, and C are bioluminescent images of mice having received the indicated cell (or PBS) and depicting tumor burden over time. Shown in D, E, and F are the corresponding line graphs depicting tumor burden over time.

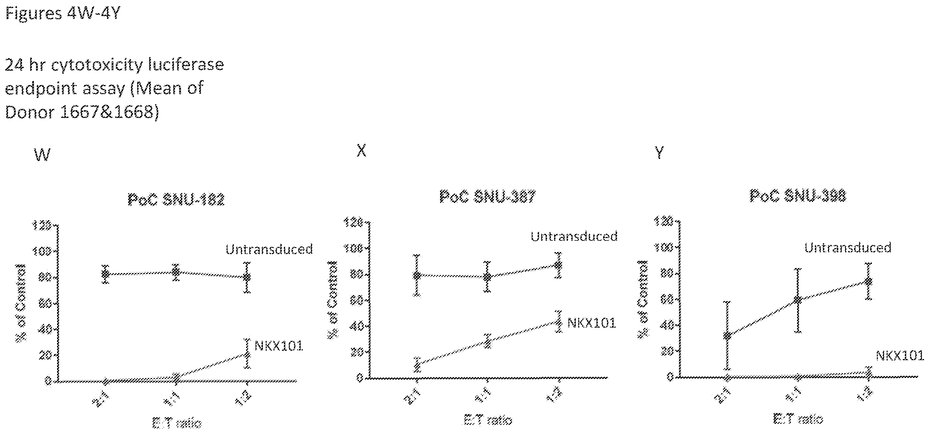

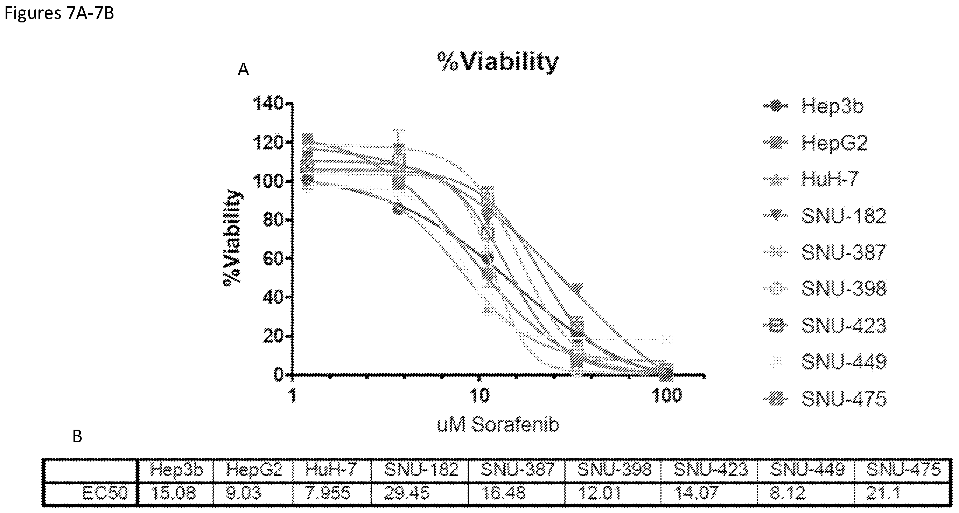

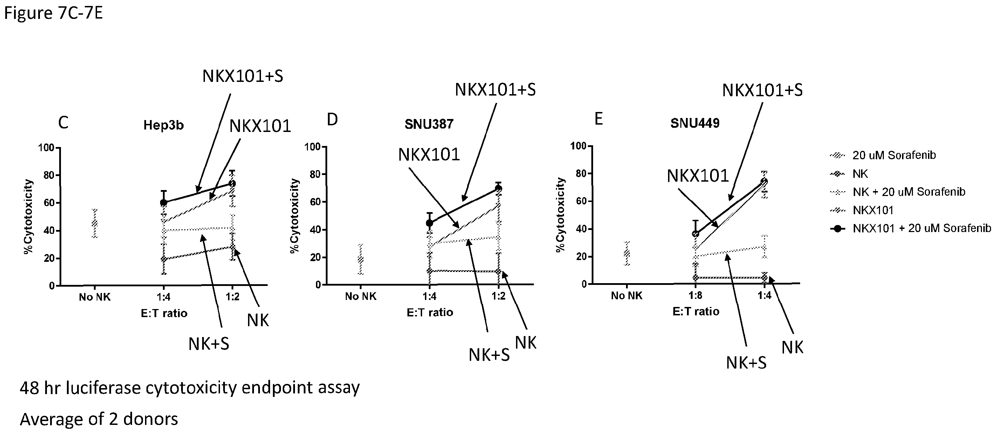

Figures 7A-7E depict data related to the efficacy of Sorafenib against various tumor cell lines when used alone or when used in conjunction with engineered NK cells provided for herein. 7A depicts data related to the percent survival of the indicated HCC cell lines at varying doses of the small molecule, sorafenib, which inhibits Raf-1, B-Raf and VEGFR-2 signaling pathways. Figure 7B is the tabulated EC50 dose for Sorafenib against the HCC cell lines. Figures 7C-7E depict the cytotoxicity of various combinations of a tyrosine kinase inhibitor (Sorafenib) with native NK cells or NKX101 at the indicated effector:target ratio.
Figures 8A-8I depict data related to the expression of various NKG2D receptor ligands and control markers in the indicated hepatocellular carcinoma cell type measured using Bamomab antibodies. NKG2D ligands are surrounded by the dashed box, while controls are surrounded by the solid box.
Figures 9A-9F depict data related the degree of cytotoxicity of the various HCC cell lines with respect to the degree of expression of the indicated NKG2D receptor ligand.
Figures 10A-10G depict data related to the degree of expression of NKG2D-Fc on various HCC cell lines (A and B) and to the degree of correlation between expression of the NKG2D-Fc and the indicated NKG2D receptor ligand.
Figures 11A-11B depict data related to the enhanced *in vitro* cytotoxicity of NKX101 cells at 4 hr endpoint measured in 2 model HCC cell lines engineered to express luciferase (11A shows Hep3b cells; 11B shows SNU449 cells). Averages shown for NKX101 and unmodified (NT NK) NK cells from 2 donors at day 21 post-expansion with K562-mbIL15-41BBL stimulatory cells. EC50 values calculated using Prism 4-parameter non-linear regression analysis.
Figures 12A-12I depict data related to the expression of various NKG2D receptor ligands and control markers in the indicated hepatocellular carcinoma cell type measured using PE conjugated antibodies (from R&D Systems). NKG2D ligands are surrounded by the dashed box, while controls are surrounded by the solid box.
Figures 13A-13I depict summary data of NKG2D ligand expression in the various indicated hepatocellular carcinoma cell lines indicated, using the PE-conjugated antibodies from Figure 12 and the BAMOmAb antibodies from Figure 8. Data are shown as dMFI (delta Mean Fluorescence Intensity, calculated as target MFI detected - antibody isotype MFI (e.g., background)).
Figures 14A-14F relate to an investigation of changes in specific NKG2D ligands in HCC. These data plot the expression level of all NKG2D ligands (Y-axis) as measured by binding of the ligands by NKG2D-Fc vs. the specific NKG2D ligand indicated (X-axis).
Figures 15A-15C depict cytotoxicity data for NKX101 cells at 4 hr endpoint measured in 3 model HCC cell lines engineered to express luciferase (15A shows Hep3b cells; 15B shows Huh-7 cells, and 15C shows SNU449 cells). Averages shown for NKX101 and unmodified (donor number) NK cells from 2 donors at day 21 post-expansion with K562-mbIL15-41BBL stimulatory cells.
Figures 16A-16G relate to engineering of various hepatocellar carcinoma cells to express luciferase. The vectors used to transduce the cell lines included a CD19 expression tag to allow for MACS CD19 positive selection of transduced cells. Figures 16A-16F show flow cytometry data for each of the indicated cell lines. Figure 16G summarizes the successful generation of the cells, as measured by their expression of luciferase (in Relative Light Units/10,000 cells).

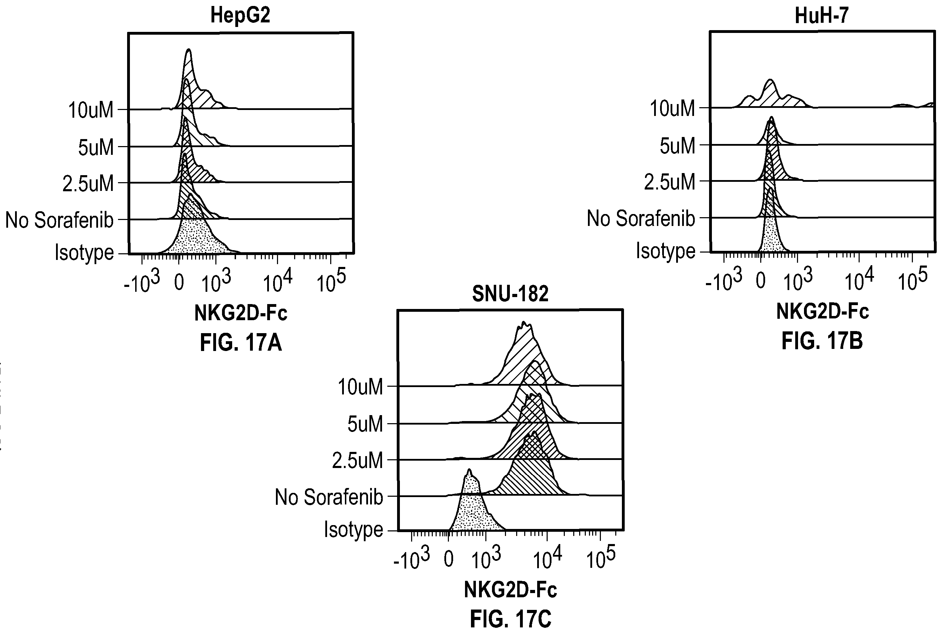

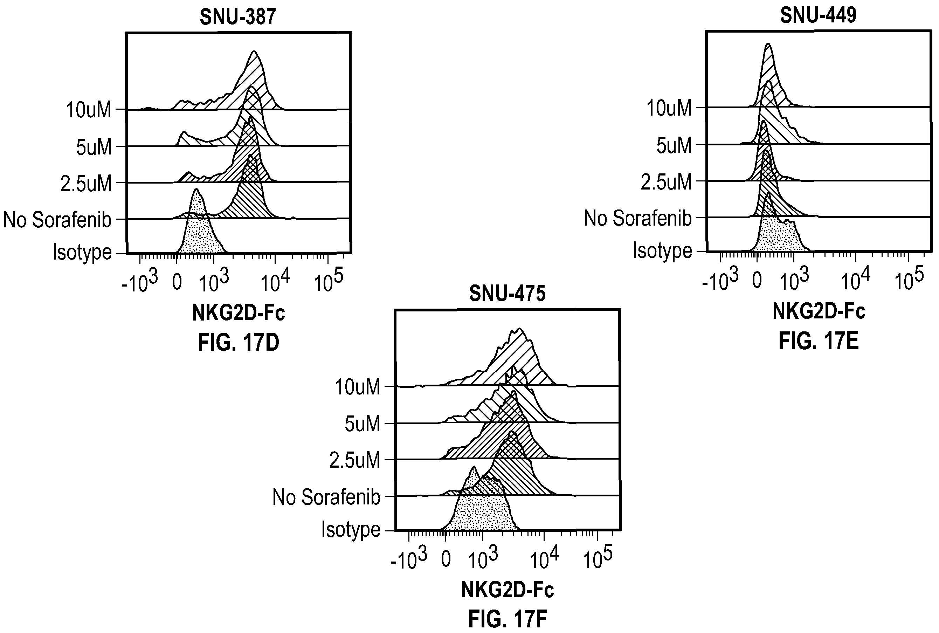

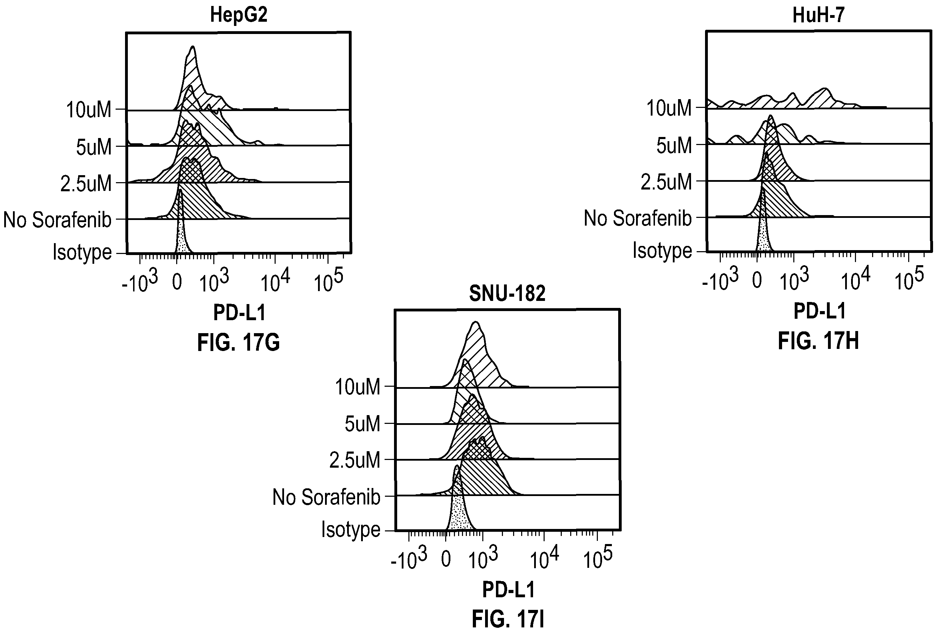

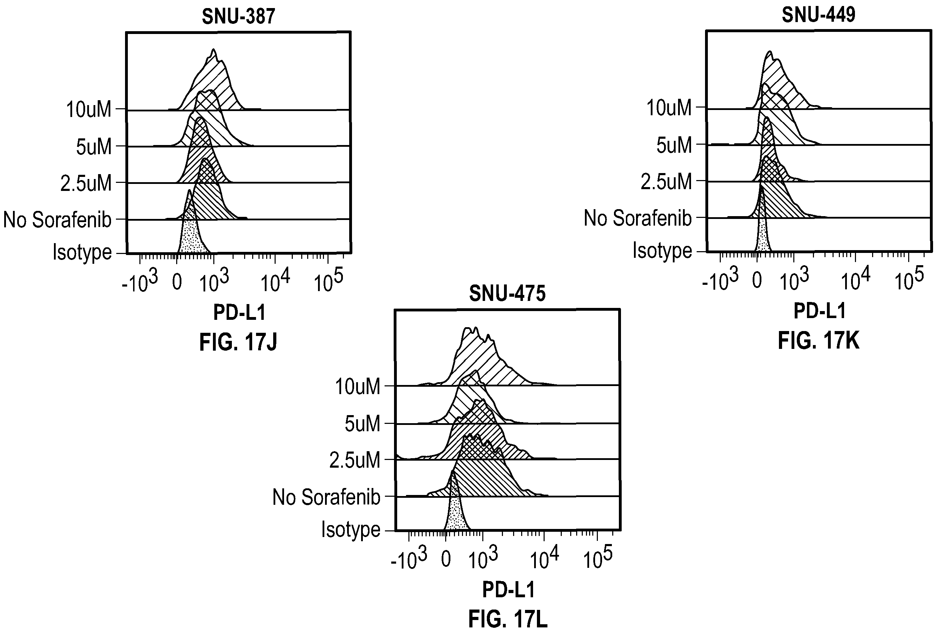

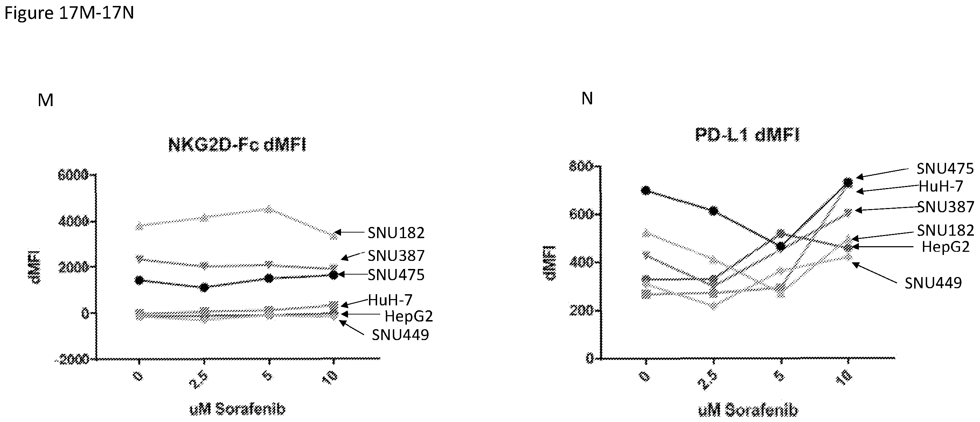

Figures 17A-17N relate to expression of total NKG2D ligands and PD-L1 in relation to sorafenib concentration. Figures 17A-17F depict flow cytometry data related to changes in total NKG2D ligand expression in the indicated tumor cell lines in response to various concentrations of sorafenib (total expression is based on the use of the NKG2D-Fc fusion, so detection is of any ligand NKG2D could bind). Figures 17G-17L depict flow cytometry data related to changes in PD-L1 expression in the indicated tumor cell lines in response to various concentrations of sorafenib. Figures 17M and 17N are line graphs that summarize the flow cytometry data.

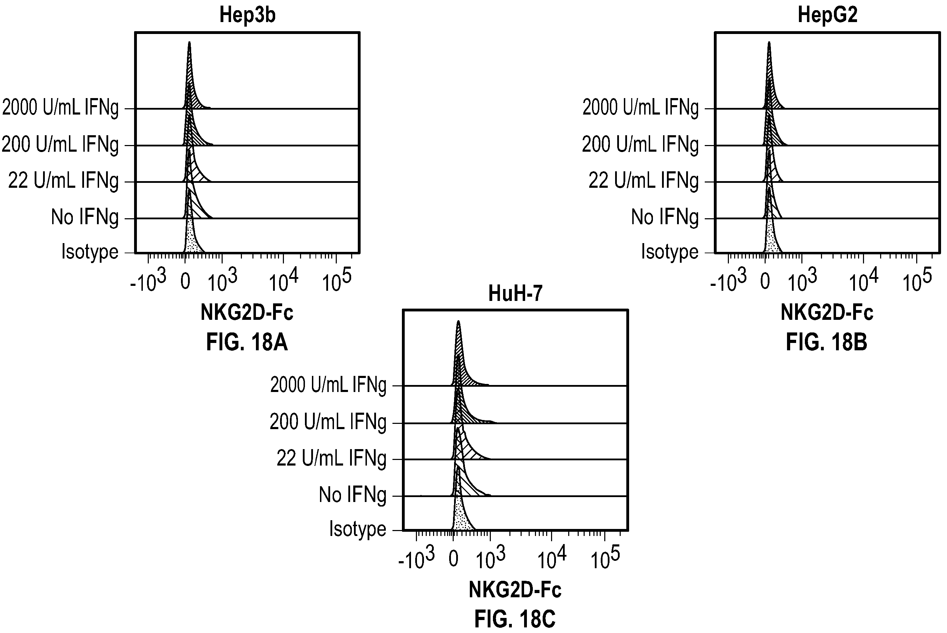

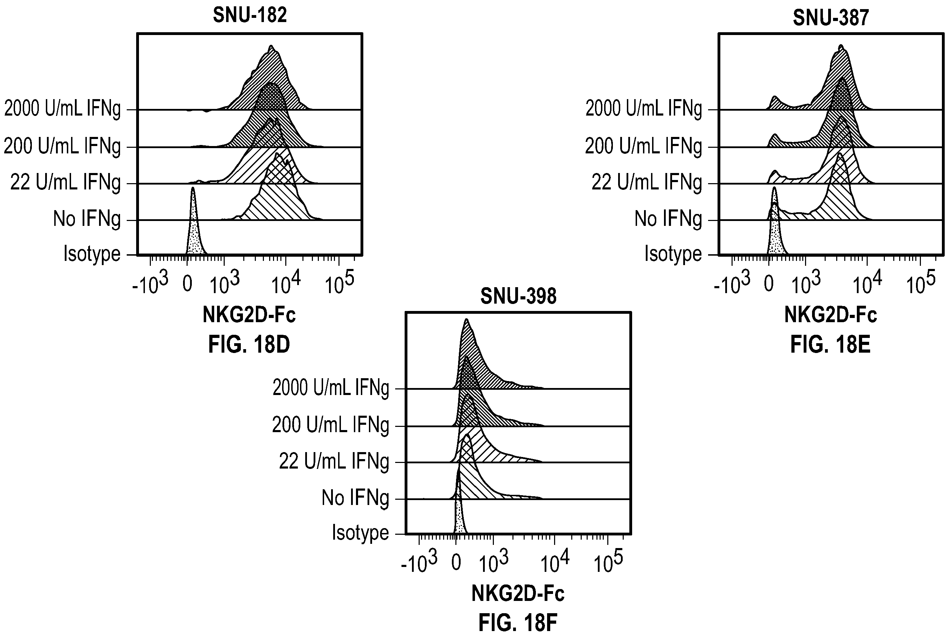

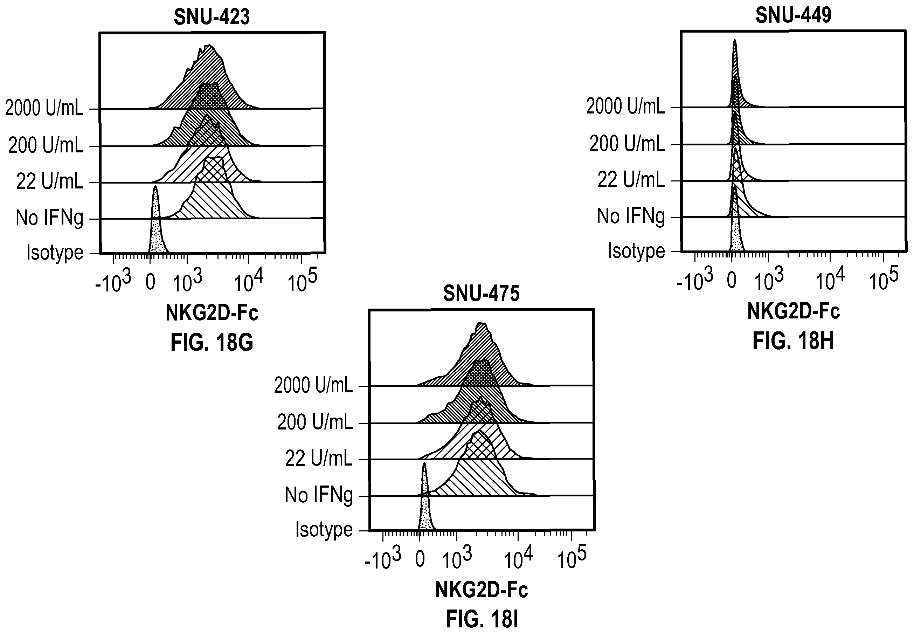

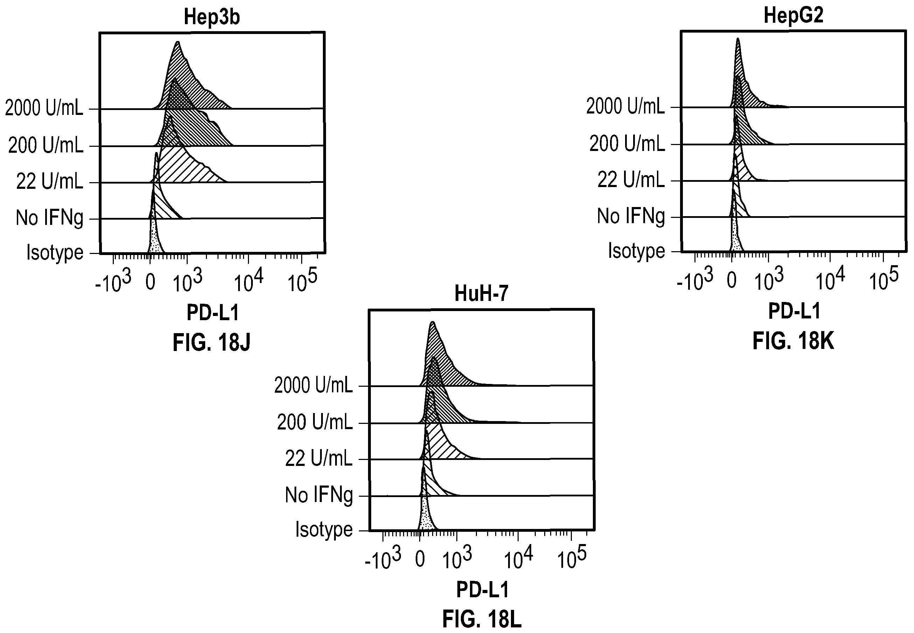

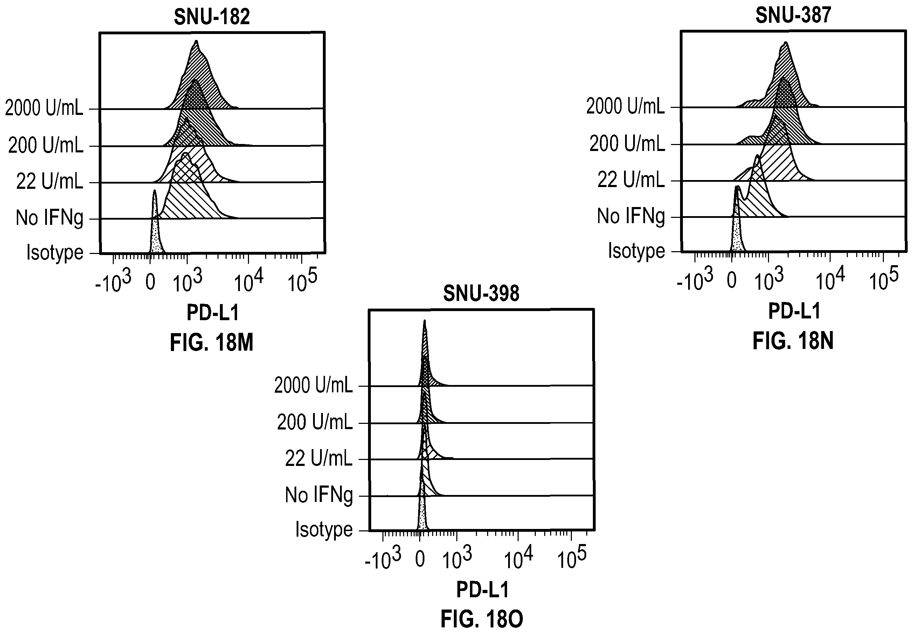

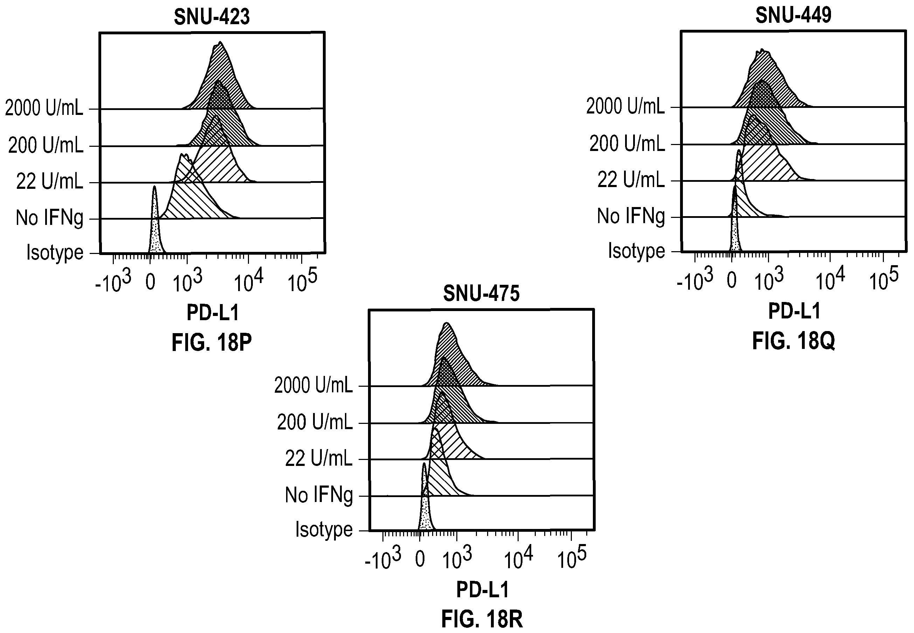

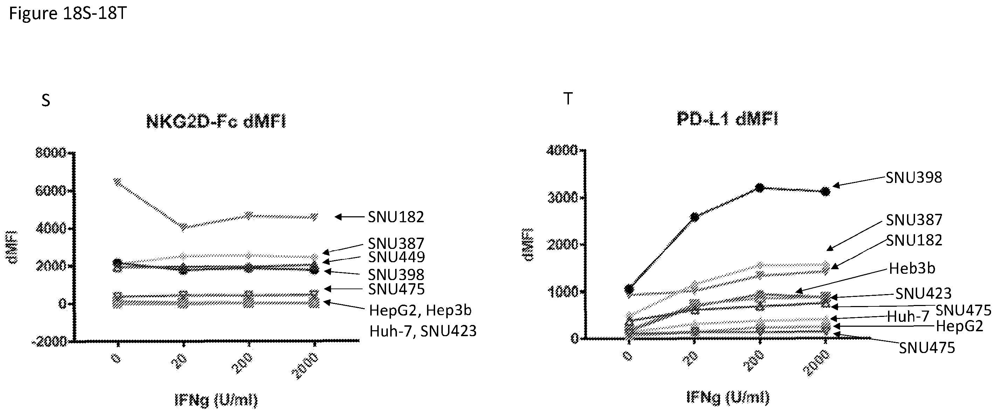

Figures 18A-18T relate to expression of total NKG2D ligands and PD-L1 in relation to Interferon gamma (IFNg) concentration. Figures 18A-18I depict flow cytometry data related to changes in total NKG2D ligand expression in the indicated tumor cell lines in response to various concentrations of IFNg (total expression is based on the use of the NKG2D-Fc fusion, so detection is of any ligand NKG2D could bind). Figures 18J-18S depict flow cytometry data related to changes in PD-L1 expression in the indicated tumor cell lines in response to various concentrations of IFNg. Figures 18S and 18T are line graphs that summarize the flow cytometry data.
Figures 19A-19D relate to expression of PD-1 by NK cells that are not transduced with a chimeric receptor construct or expression of PD-1 by NKX101 cells.
Figures 20A-20E relate to the expression of CD19 by engineered luciferaseexpressing colorectal cancer (CRC) cell lines. The vectors used to transduce the cell lines included a CD19 expression tag to allow for MACS CD19 positive selection of transduced cells.
Figures 21A-21E relate to the expression of various NKG2D ligands by the CRC cells engineered to express luciferase.
Figures 22A-22F show flow cytometry data relating to the expression by NK cells from 3 donors of CD56 before (A-C) and after (D-F) CD3 depletion.
Figures 23A-23C show flow cytometry data relating to expression of NKG2D receptor by engineered NK cells (NKX101) (as compared to non-transduced NK cells (NTNK)) in NK cells from two donors.
Figures 24A-24C depict cytotoxicity data of NKX101 cells from four different donors against the indicated CRC cell lines, as well as the associated EC50 values. Data collected at day 14-21 post-expansion with K562-mbIL15-41BBL stimulatory cells.
Figures 25A-25F show flow cytometry data relating to the characterization of NK cell from two donors and engineered to express NKX101. Figures 25A-B and D-E relate to expression by NK cells from 2 donors of CD56 before (A-B) and after (D-E) CD3 depletion. Figures 25C and 25F show flow cytometry data relating to NKX101 expression (as compared to non-transduced NK cells (NTNK)) in NK cells from these two donors.
Figures 26A-26C depict analysis of cytokines released by the indicated CRC cell line in response to either non-transduced NK cells (NTNK) or NK cells expressing NKX101.
Figures 27A-27E show standard curves for each cytokine analyzed and summarized in Figures 26A-26E.
Figures 28A-28B display flow cytometry data related to activating receptor expression by NK cells during expansion.
Figures 29A-29C displays data on the *in vivo* persistence and cytotoxicity of engineered NK cells according to embodiments disclosed herein. Figure 29A shows enhanced *in vivo* persistence of non-limiting embodiments of engineered NK cells disclosed herein. Figure 29B shows enhanced *in vivo* cytotoxicity data for non-limiting embodiments of engineered NK cells disclosed herein. Figure 29C shows bioluminescent intensity imaging data that underlie the data of Figure 29B.
Figures 30A-30B relate to data from a THP1 AML xenograft model. Figure 30A depicts in vivo data related to the cytotoxicity of an NKG2D CAR according to embodiments disclosed herein against acute myelogenous leukemia cells. Figure 30B provides a summary of the *in vivo* data.
Figure 31 depicts summary data related to the cytotoxicity of an NKG2D CAR according to embodiments disclosed herein against various acute myelogenous leukemia cells.

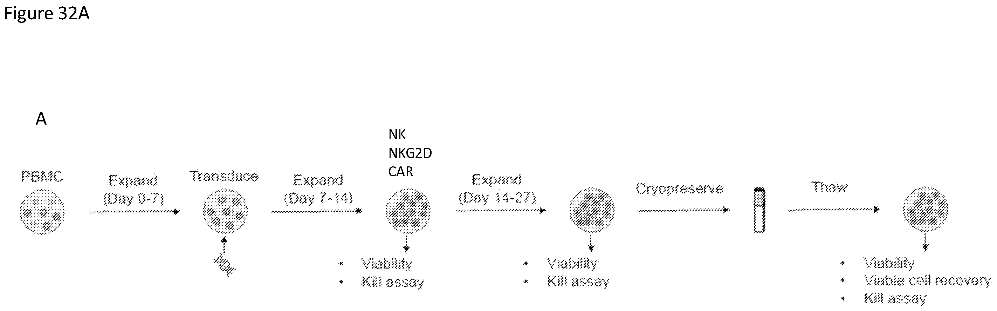

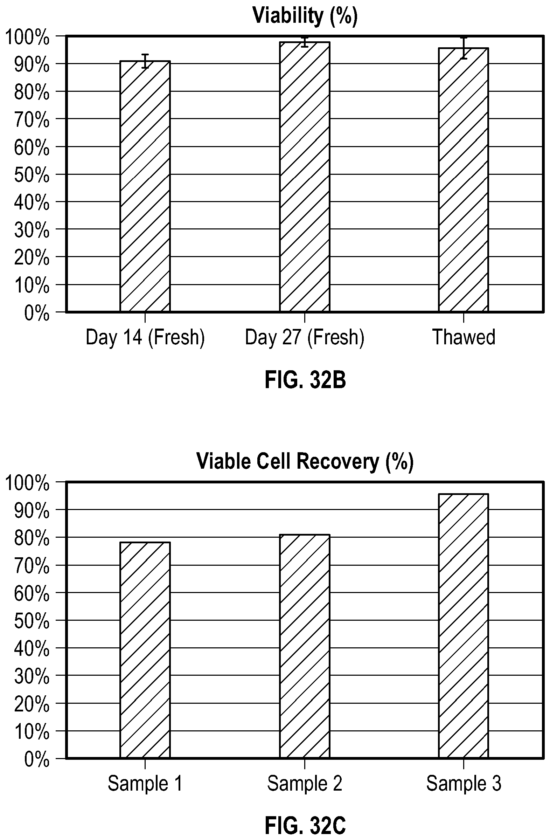

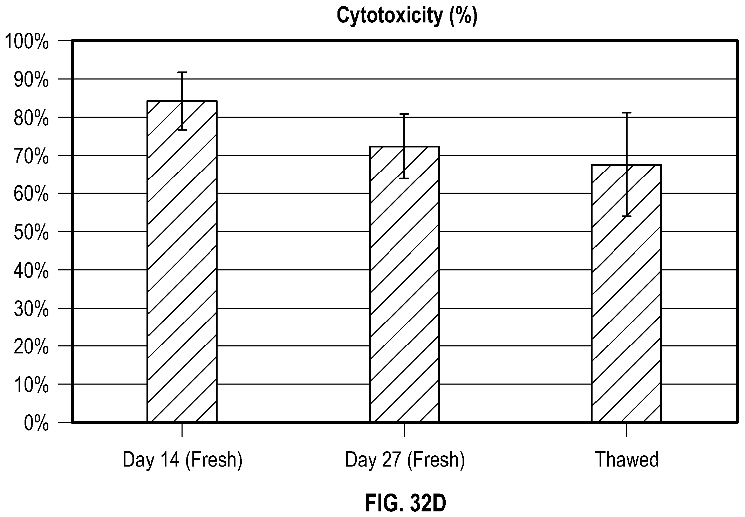

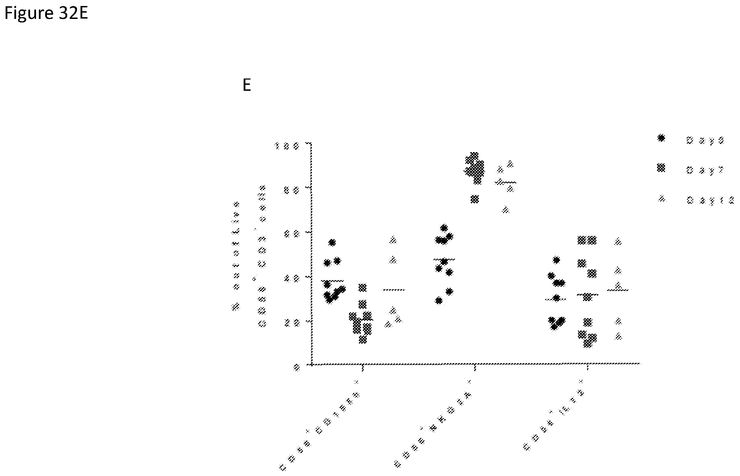

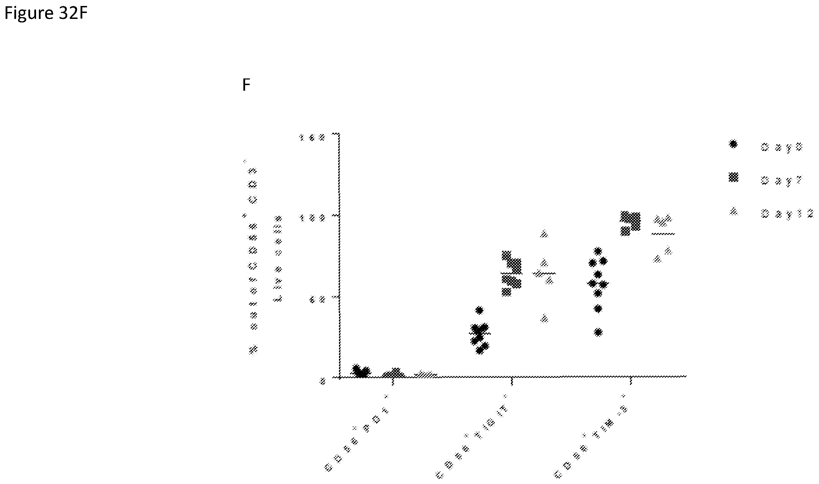

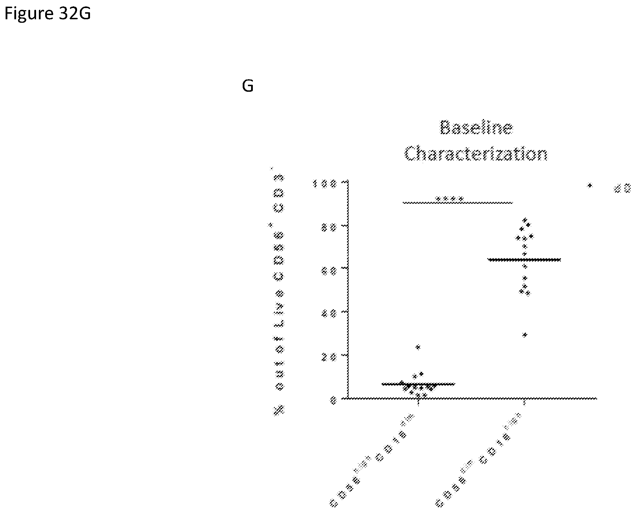

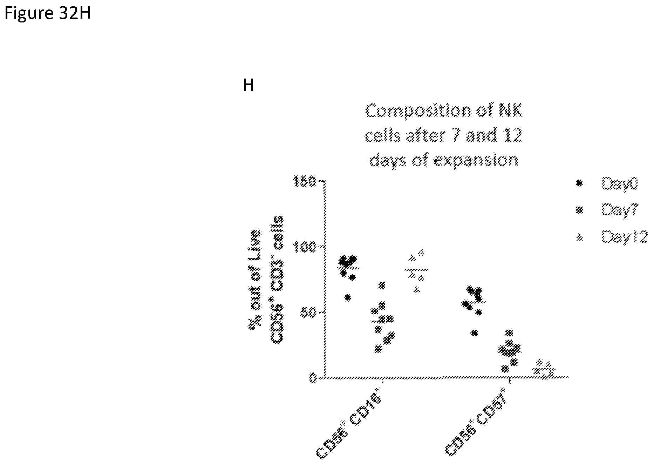

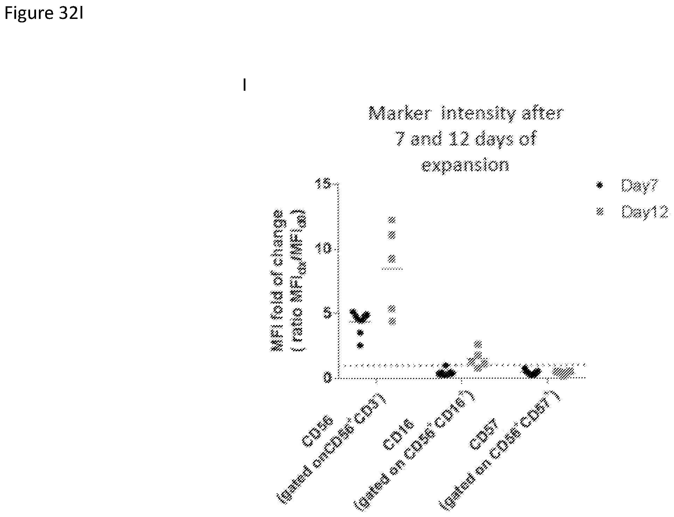

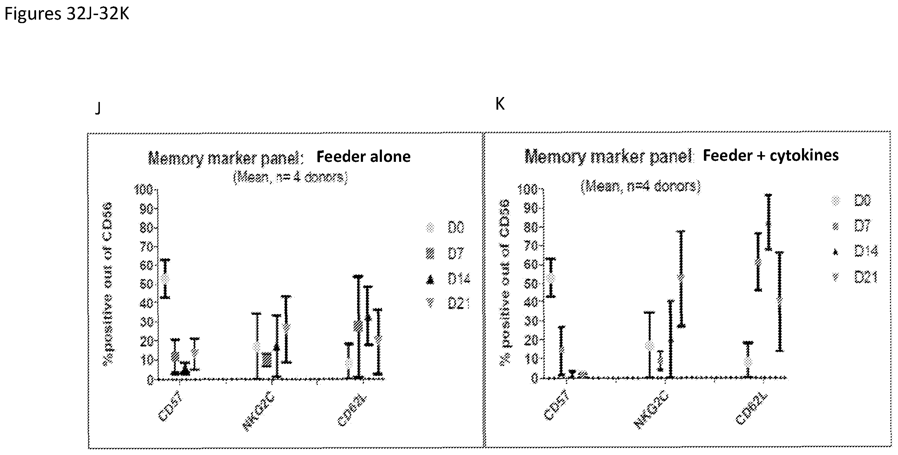

Figures 32A-32K relate to further characterization of NK cells and engineered NK cells as disclosed herein. Figure 32A shows a schematic protocol for characterizing the engineered NK cells disclosed herein and their response to cryopreservation. Figure 32B shows data related to engineered NK cell viability. Figure 32C shows data related to engineered NK cell viable cell recovery. Figure 32D shows data related to engineered NK cell retention of cytotoxicity. Figure 32E shows summary data related to expression of certain markers by engineered NK cells during expansion. Figure 32F shows additional summary data related to expression of certain markers by engineered NK cells during expansion. Figure 32G shows summary data related to expression of CD16 by engineered NK cells during expansion. Figure 32H shows summary data related to marker expression by engineered NK cells during expansion. Figure 32I shows summary data related to the intensity of marker expression by engineered NK cells during expansion. Figure 32J shows summary data related to marker expression by engineered NK cells during expansion with feeder cells alone. Figure 32K shows summary data related to marker expression by engineered NK cells during expansion with feeder cells and additional cytokines.
Figures 33A-33C related to expression of various surface markers by engineered NK cells according to embodiments disclosed herein or on target cells (here Raji and Nalm6 cells). Figure 33A shows CD16 expression on NK cells expressing an NKG2D CAR according to several embodiments disclosed herein. Figure 33B shows CD19 expression on Raji and Nalm6 tumor cells. Figure 33C shows CD20 expression on Raji and Nalm6 tumor cells.
Figure 34 shows data related to reduction in Raji cell growth when exposed to NK cells expressing a non-limiting example of an NKG2D CAR as well as an anti-CD20 antibody.
Figures 35A-35B related to the inhibition of HL60 leukemia cell growth when exposed to NK cells expressing a non-limiting example of an NKG2D CAR as well as an HDAC inhibitor. Figure 35A shows HL60 cell growth with the indicated constructs. Figure 35B shows the same constructs in conjunction with administration of an HDAC inhibitor.
Figure 36 depicts data related to reduction in cell growth in the presence of various immune cell types expressing a non-limiting embodiment of an NKG2D CAR as disclosed herein.

### DETAILED DESCRIPTION

Delivery of a drug product to the site of therapeutic activity is an important factor to the drug's performance and safety. In the case of cellular immunotherapies, issues of delivery can be an additional hurdle to effective therapy, as effector cell types are frequently excluded from the tumor microenvironment. While tumor infiltration of NK cells has been associated with good prognosis in several tumor types, NK cells have also been observed to poorly infiltrate some tumor types (e.g., renal cell carcinoma, colorectal carcinoma, and melanoma). Lack of infiltration, perhaps associated with gaps in the chemokine receptor repertoire expressed by NK cells, may account for the varied clinical results achieved using adoptively infused NK cells in connection with certain tumors.

Some therapies benefit from local delivery of therapeutic agents to the liver. For example, administration of chemotherapeutic agents via the intra-hepatic artery has been demonstrated. Similarly, regional delivery of CAR T cells to the liver for the treatment of hepatocellular carcinoma has been performed.

In accordance with several embodiments, disclosed herein, cell therapies using NK cells are an attractive option for use in hepatocellular carcinoma. Ligands of the NKG2D receptor are expressed in hepatocellular carcinoma, and overexpression of the NKG2D receptor improves NK cell cytotoxicity towards hepatocellular carcinoma-derived cell lines in vitro and in vivo. Further, while NK cells derived from hepatocellular carcinoma patients may exhibit signs of exhaustion, exposure to IL-15 can restore some functionality. In some embodiments, intrahepatic delivery of NK cells is a highly effective method for use of these cells for treatment of hepatocellular carcinoma. In several embodiments, NK-based therapy using engineered NK cells further increases the efficacy over native NK cells. For example, according to some embodiments, NK cells are engineered to express a cytotoxic receptor complex comprising an NKG2D receptor (or portion thereof). According to some embodiments, NK cells are engineered to express IL-15, in some embodiments, a secreted form, while in some embodiments, the IL-15 is membrane bound. While NK infiltration to hepatocellular carcinoma may be limited, and potentially suppressed by the tumor microenvironment, local delivery to the liver of a such engineered NK cells can both ensure delivery of an effective dose to the site of disease, and contain the cells in the anatomical compartment in which they're needed, thus limiting potential off-target toxicities. Thus, as discussed in more detail below, such compositions and methods of locally delivering such engineered NK cells enhances the sensitivity of the NKs towards NKG2D ligands and improve their ability to resist the suppressive forces of the tumor microenvironment, and provides for enhanced anti-cancer therapy.

### Engineered NK Cells for Immunotherapy

Various types of immune cells can be used in cellular immunotherapy. Some embodiments, relate to T cell-based compositions (e.g., those expressing NKG2D-based cytotoxic receptor complexes as disclosed herein) and methods. Several embodiments relate to NK cell-based compositions and methods. According to several embodiments, compositions comprising engineered NK cells have certain advantageous characteristics. NK cells enables the use of either autologous or donor-derived allogeneic cells. In accordance with several embodiments, engineered NK cells have minimal cytotoxicity against normal cells. This advantage is further enhanced when coupled with local delivery, for example via the intra-hepatic artery. Engineered NK cells also have a significant cytotoxic effect, unexpectedly elevated over that of native NK cells, which are already quite potent.

According to several embodiments, NK cells are engineered to express one or more cytotoxic receptor complexes. The complexes comprise, depending on the embodiment, a domain that binds to tumor ligands (e.g., an extracellular domain), and a cytotoxic signaling complex that comprises a transmembrane domain, a signaling domain, and/or a co-stimulatory domain. Each domain may comprise one or more subdomains.

### Domains that Bind Tumor Ligands

In several embodiments, engineered NK cells are leveraged for their ability to recognize and destroy tumor cells. NK cells express both inhibitory and activating receptors on the cell surface. Inhibitory receptors bind self-molecules expressed on the surface of healthy cells (thus preventing immune responses against "self" cells), while the activating receptors bind ligands expressed on abnormal cells, such as tumor cells. A "tip" in the balance between inhibitory and activating receptor activation in favor of activating receptors leads to NK cell activation and target (e.g., tumor) cell lysis. Natural killer Group 2 member D (NKG2D) is an NK cell activating receptor that recognizes a variety of ligands expressed on cells. The surface expression of various NKG2D ligands is generally low in healthy cells but is upregulated upon, for example, malignant transformation. Non-limiting examples of ligands recognized by NKG2D include, but are not limited to, MICA, MICB, ULBP1, ULBP2, ULBP3, ULBP4, ULBP5, and ULBP6, as well as other molecules expressed on target cells that control the cytolytic or cytotoxic function of NK cells.

In several embodiments, cells are engineered to express a cytotoxic receptor complex comprising a full length NKG2D as an extracellular component to recognize ligands on the surface of tumor cells (e.g., liver cells). In one embodiment, full length NKG2D has the nucleic acid sequence of SEQ ID NO. 24.

In several embodiments, cells are engineered to express a cytotoxic receptor complex comprising a functional fragment of NKG2D as an extracellular component to recognize ligands on the surface of tumor cells (e.g., liver cells). In one embodiment, the functional fragment of NKG2D has the nucleic acid sequence of SEQ ID NO. 24. In several embodiments, the fragment of NKG2D is at least 70%, at least 75%, at least 80%, at least 85%, at least 90%, or at least 95% homologous with full-length wild-type NKG2D. In several embodiments, the fragment may have one or more additional mutations from SEQ ID NO. 24, but retains, or in some embodiments, has enhanced, ligand-binding function. In several embodiments, the NKG2D fragment is provided as a dimer, trimer, or other concatameric format, such embodiments providing enhanced ligand-binding activity. In several embodiments, the sequence encoding the NKG2D fragment is optionally fully or partially codon optimized. In one embodiment, a sequence encoding a codon optimized NKG2D fragment comprises the sequence of SEQ ID NO. 25. Advantageously, according to several embodiments, the functional fragment lacks its native transmembrane or intracellular domains but retains its ability to bind ligands of NKG2D as well as transduce activation signals upon ligand binding. A further advantage of such fragments is that expression of DAP10 to localize NKG2D to the cell membrane is not required. Thus, in several embodiments, the cytotoxic receptor complex encoded by the polypeptides disclosed herein does not comprise DAP10.

In several embodiments, the cytotoxic receptor complexes are configured to dimerize. Dimerization may comprise homodimers or heterodimers, depending on the embodiment. In several embodiments, dimerization results in improved ligand recognition by the cytotoxic receptor complexes (and hence the NK cells expressing the receptor), resulting in a reduction in (or lack) of adverse toxic effects. In several embodiments, the cytotoxic receptor complexes employ internal dimers, or repeats of one or more component subunits. For example, in several embodiments, the cytotoxic receptor complexes may optionally comprise a first NKG2D extracellular domain coupled to a second NKG2D extracellular domain, and a transmembrane/signaling region (or a separate transmembrane region along with a separate signaling region).

In several embodiments, the various domains/subdomains are separated by a linker such as, a GS3 linker (SEQ ID NO: 41) is used (or a GSn linker). This provides the potential to separate the various component parts of the receptor complex along the polynucleotide, which can enhance expression of the receptor complex.

### Cytotoxic Signaling Complex

As disclosed herein, according to several embodiments the provided cytotoxic receptor complexes comprise a domain to bind to ligands on the surface of target cells and also comprise a cytotoxic signaling complex. In several embodiments, the cytotoxic signaling complex comprises at least one transmembrane domain, at least one co-stimulatory domain, and/or at least one signaling domain. In some embodiments, more than one component part makes up a given domain - e.g., a co-stimulatory domain may comprise two subdomains. Moreover, in some embodiments, a domain may serve multiple functions, for example, a transmembrane domain may also serve to provide signaling function.

### Transmembrane Domains

In several embodiments, the NKG2D domain employed retains at least a portion of its normal transmembrane domain. In several embodiments, however, the transmembrane domain comprises at least a portion of CD8, a transmembrane glycoprotein normally expressed on both T cells and NK cells. In several embodiments, the transmembrane domain comprises CD8α. In several embodiments, the transmembrane domain is referred to as a "hinge". In several embodiments, the "hinge" of CD8α has the sequence of SEQ ID NO. 26. In several embodiments, the CD8α hinge is truncated or modified and is at least 70%, at least 75%, at least 80%, at least 85%, at least 90%, at least 95% homologous with the CD8α having the sequence of SEQ ID NO. 26.

In several embodiments, the transmembrane domain comprises a CD8α transmembrane region. In several embodiments, the CD8α transmembrane domain has the sequence of SEQ ID NO. 27. In several embodiments, the CD8α hinge is truncated or modified and is at least 70%, at least 75%, at least 80%, at least 85%, at least 90%, at least 95% homologous with the CD8α having the sequence of SEQ ID NO. 27.

In several embodiments, the transmembrane domain comprises an IgG4 short hinge. In several embodiments, the IgG4 short hinge transmembrane domain has the sequence of SEQ ID NO. 28. In several embodiments, the CD8α hinge is truncated or modified and is at least 70%, at least 75%, at least 80%, at least 85%, at least 90%, at least 95% homologous with the CD8α having the sequence of SEQ ID NO. 28.

In several embodiments, the cytotoxic signaling complex comprises a CD28 domain. In several embodiments, the transmembrane domain comprises CD28, while in several embodiments the CD28 domain is an intracellular signaling domain, while in several embodiments the CD28 domain is a transmembrane/intracellular signaling domain. In several embodiments, the CD28 transmembrane domain has the sequence of SEQ ID NO. 29. In several embodiments, the CD28 transmembrane domain can be truncated or modified, such that it is at least 70%, at least 75%, at least 80%, at least 85%, at least 90%, at least 95% homologous with the CD28 having the sequence of SEQ ID NO. 29. In several embodiments, the CD28 signaling domain has the sequence of SEQ ID NO. 40. In several embodiments, the CD28 signaling domain can be truncated or modified, such that it is at least 70%, at least 75%, at least 80%, at least 85%, at least 90%, at least 95% homologous with the CD28 having the sequence of SEQ ID NO. 40. In several embodiments, CD28 is used as the sole transmembrane/signaling domain in the construct, however, in several embodiments, CD28 can be used with one or more other domains.

In several embodiments, CD28 is used as the sole transmembrane/signaling domain in the construct, however, in several embodiments, CD28 can be used with one or more other domains. For example, combinations of CD28, OX40, 4-1BB, and/or CD3zeta are used in some embodiments.

In several embodiments, the transmembrane domain comprises the transmembrane domain of the T cell CD3 T cell receptor complex (made up of the zeta, alpha, beta, gamma, delta, and epsilon subunits). In several embodiments, the entire transmembrane domain is used, while in some embodiments a fragment thereof is used. In some embodiments, 1 to 20 (e.g., 1, 2, 3, 4, 5, 6, 8, 10 , 15 or more) extracellular CD3zeta residues are directly adjacent to the CD3zeta transmembrane domain. In some embodiments, CD3zeta transmembrane domain has the sequence of SEQ ID NO. 30. In several embodiments, the CD3zeta transmembrane domain can be truncated or modified, such that it is at least 70%, at least 75%, at least 80%, at least 85%, at least 90%, at least 95% homologous with the CD3zeta transmembrane domain having the sequence of SEQ ID NO. 30. In several embodiments the modifications to the CD3zeta transmembrane domain comprise additional nucleic acid residues to increase the length of the domain.

### Co-stimulatory Domains

In addition the various the transmembrane domains and signaling domain (and the combination transmembrane/signaling domains), additional co-activating molecules can be provided, in several embodiments. These can be certain molecules that, for example, further enhance activity of the immune cells. Cytokines may be used in some embodiments. For example, certain interleukins, such as IL-2 and/or IL-15 as non-limiting examples, are used. In some embodiments, the immune cells for therapy are engineered to express such molecules as a secreted form. In additional embodiments, such co-stimulatory domains are engineered to be membrane bound, acting as autocrine stimulatory molecules (or even as paracrine stimulators to neighboring cells delivered). In several embodiments, NK cells are engineered to express membrane-bound interleukin 15 (mbIL15). In such embodiments, mbIL15 expression on the NK enhances the cytotoxic effects of the engineered NK cell by enhancing the proliferation and/or longevity of the NK cells. In several embodiments, mbIL15 has the nucleic acid sequence of SEQ ID NO. 39. In several embodiments, mbIL15 can be truncated or modified, such that it is at least 70%, at least 75%, at least 80%, at least 85%, at least 90%, at least 95% homologous with the sequence of SEQ ID NO. 39.

In some embodiments, the engineered cytotoxic receptor complex is encoded by a polynucleotide that includes one or more cytosolic protease cleavage sites, for example a T2A cleavage site, a P2A cleavage site, an E2A cleavage site, and/or a F2A cleavage site. Such sites are recognized and cleaved by a cytosolic protease, which can result in separation (and separate expression) of the various component parts of the receptor encoded by the polynucleotide. As a result, depending on the embodiment, the various constituent parts of a engineered cytotoxic receptor complex can be delivered to an NK cell in a single vector or by multiple vectors. Thus, as shown schematically, in the Figures, a construct can be encoded by a single polynucleotide, but also include a cleavage site, such that downstream elements of the constructs are expressed by the cells as a separate protein (as is the case in some embodiments with IL-15). In several embodiments, a T2A cleavage site is used. In several embodiments, a T2A cleavage site has the nucleic acid sequence of SEQ ID NO. 38. In several embodiments, mbIL15 can be truncated or modified, such that it is at least 70%, at least 75%, at least 80%, at least 85%, at least 90%, at least 95% homologous with the sequence of SEQ ID NO. 38.

### Signaling Domains

In several embodiments, signaling is provided through 4-1BB (also known as CD137 and tumor necrosis factor receptor superfamily member 9 (TNFRSF 9)). While 4-1BB has a natural role as a stimulatory molecule for activated T cells (e.g., crosslinking of 4-1BB enhances T cell proliferation and cytolytic activity), as disclosed herein, several embodiments leverage the function of 4-1BB activity with NK cells. In several embodiments, 4-1BB has the sequence of SEQ ID NO. 31. In several embodiments, 4-1BB can be truncated or modified, such that it is at least 70%, at least 75%, at least 80%, at least 85%, at least 90%, at least 95% homologous with the 4-1BB having the sequence of SEQ ID NO. 31. In several embodiments, 4-1BB is the sole signaling domain. In several embodiments, however, enhanced signaling is achieved through the use of multiple signaling domains whose activities act synergistically.

In several embodiments, the NK cells engineered according to several embodiments disclosed herein comprise at least one subunit of the CD3 T cell receptor complex (or a fragment thereof). In several embodiments, the signaling domain comprises the CD3 zeta subunit. In several embodiments, the CD3 zeta has the sequence of SEQ ID NO. 32. In several embodiments, the CD3 zeta can be truncated or modified, such that it is at least 70%, at least 75%, at least 80%, at least 85%, at least 90%, at least 95% homologous with the CD3 zeta having the sequence of SEQ ID NO. 32.

In several embodiments, the CD3 zeta is mutated (e.g., amino acid mutations, insertions, or deletions) such that the domain no longer includes a canonical immunoreceptor tyrosine-based activation motif (ITAM) motif. In some embodiments, the resultant engineered NK cells exhibit particularly enhanced cytotoxicity against target cells, with limited or reduced adverse side effects. In several embodiments, the signaling domain comprises a non-ITAM CD3 zeta subunit. In several embodiments, the non-ITAM CD3 zeta has the sequence of SEQ ID NO. 33. In several embodiments, the non-ITAM CD3 zeta can be truncated or modified, such that it is at least 70%, at least 75%, at least 80%, at least 85%, at least 90%, at least 95% homologous with the non-ITAM CD3 zeta having the sequence of SEQ ID NO. 33.

This, in several embodiments, results from the synergistic interactions of the various portions of the cytotoxic receptor complex that are used in that given embodiment. In several embodiments, CD3zeta in conjunction with 4-1BB provides synergistic stimulation effects, resulting in particularly effective (e.g., cytotoxic) NK cells.

In several embodiments, the signaling domain comprises an OX40 domain. In several embodiments the OX40 domain is an intracellular signaling domain. In several embodiments, the OX40 intracellular signaling domain has the sequence of SEQ ID NO. 35. In several embodiments, the OX40 intracellular signaling domain can be truncated or modified, such that it is at least 70%, at least 75%, at least 80%, at least 85%, at least 90%, at least 95% homologous with the OX40 having the sequence of SEQ ID NO. 35. In several embodiments, OX40 is used as the sole transmembrane/signaling domain in the construct, however, in several embodiments, OX40 can be used with one or more other domains. For example, combinations of CD28, OX40, 4-1BB, and/or CD3zeta are used in some embodiments.

In several embodiments, the signaling domain comprises an NKp80 domain. In several embodiments the NKp80 domain is an intracellular signaling domain. In several embodiments, the NKp80 intracellular signaling domain has the sequence of SEQ ID NO. 36. In several embodiments, the NKp80 intracellular signaling domain can be truncated or modified, such that it is at least 70%, at least 75%, at least 80%, at least 85%, at least 90%, at least 95% homologous with the OX40 having the sequence of SEQ ID NO. 36. In several embodiments, NKp80 is used as the sole transmembrane/signaling domain in the construct, however, in several embodiments, NKp80 can be used with one or more other domains. For example, combinations of CD28, OX40, 4-1BB, and/or CD3zeta are used in some embodiments.

In several embodiments, the signaling domain comprises a fragment of CD16. In several embodiments, the fragment of CD16 is an intracellular region of CD16 (CD16 IC). In several embodiments the CD16 IC domain is an intracellular signaling domain. In several embodiments, the CD16 IC intracellular signaling domain has the sequence of SEQ ID NO. 37. In several embodiments, the CD16 IC intracellular signaling domain can be truncated or modified, such that it is at least 70%, at least 75%, at least 80%, at least 85%, at least 90%, at least 95% homologous with the OX40 having the sequence of SEQ ID NO. 37. In several embodiments, CD16 IC is used as the sole transmembrane/signaling domain in the construct, however, in several embodiments, CD16 IC can be used with one or more other domains. For example, combinations of CD28, OX40, 4-1BB, and/or CD3zeta are used in some embodiments.

### Cytotoxic Receptor Complex Constructs

In line with the above, a variety of cytotoxic receptor complexes (also referred to as cytotoxic receptors) are provided for herein. The expression of these complexes in NK cells allows the targeting and destruction of particular target cells, such cancerous cells, in particular, cancerous liver cells. Non-limiting examples of such cytotoxic receptor complexes are discussed in more detail below.

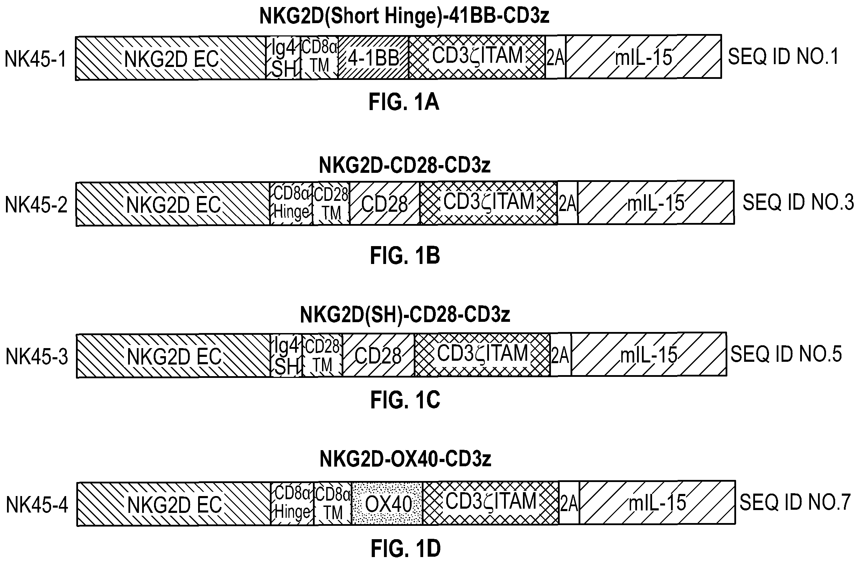

In several embodiments, there are provided polynucleotides encoding a NKG2D/4-1BB/CD3z cytotoxic receptor complex (see Figure 1A, NK45-1). Such receptor complexes comprise an extracellular domain comprising an NKG2D fragment that binds native ligands of NKG2D. The construct further comprises an IgG4 hinge and CD8α transmembrane domain, a 4-1BB subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 1. In several embodiments, the cytotoxic receptor complex comprises the amino acid sequence of SEQ ID NO: 2. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 1, but remains, depending on the embodiment, at least 70%, at least 75%, at least 80%, at least 85%, at least 90%, or at least 95% homologous with SEQ ID NO. 1. Additionally, as indicated above, different extracellular domains may also be used, such as for example, that of SEQ ID NO. 23 or 25, as non-limiting examples.

In several embodiments, there are provided polynucleotides encoding a NKG2D/CD28/CD3z cytotoxic receptor complex (see Figure 1B, NK45-2). Such receptor complexes comprise an extracellular domain comprising an NKG2D fragment that binds native ligands of NKG2D. The construct further comprises a CD8α hinge and a CD28 transmembrane domain, a CD28 signaling subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 3. In several embodiments, the cytotoxic receptor complex comprises the amino acid sequence of SEQ ID NO: 4. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 3, but remains, depending on the embodiment, at least 70%, at least 75%, at least 80%, at least 85%, at least 90%, or at least 95% homologous with SEQ ID NO. 3. Additionally, as indicated above, different extracellular domains may also be used, such as for example, that of SEQ ID NO. 23 or 25, as non-limiting examples.

In several embodiments, there are provided polynucleotides encoding a NKG2D(SH)/CD28/CD3z cytotoxic receptor complex (see Figure 1C, NK45-3). Such receptor complexes comprise an extracellular domain comprising an NKG2D fragment that binds native ligands of NKG2D. The construct further comprises an IgG4 short hinge and a CD28 transmembrane domain, a CD28 signaling subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 5. In several embodiments, the cytotoxic receptor complex comprises the amino acid sequence of SEQ ID NO: 6. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 5, but remains, depending on the embodiment, at least 70%, at least 75%, at least 80%, at least 85%, at least 90%, or at least 95% homologous with SEQ ID NO. 5. Additionally, as indicated above, different extracellular domains may also be used, such as for example, that of SEQ ID NO. 23 or 25, as non-limiting examples.

In several embodiments, there are provided polynucleotides encoding a NKG2D/OX40/CD3z cytotoxic receptor complex (see Figure 1D, NK45-4). Such receptor complexes comprise an extracellular domain comprising an NKG2D fragment that binds native ligands of NKG2D. The construct further comprises a CD8α hinge and a CD8α transmembrane domain, an OX40 signaling subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 7. In several embodiments, the cytotoxic receptor complex comprises the amino acid sequence of SEQ ID NO: 8. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 7, but remains, depending on the embodiment, at least 70%, at least 75%, at least 80%, at least 85%, at least 90%, or at least 95% homologous with SEQ ID NO. 7. Additionally, as indicated above, different extracellular domains may also be used, such as for example, that of SEQ ID NO. 23 or 25, as non-limiting examples.

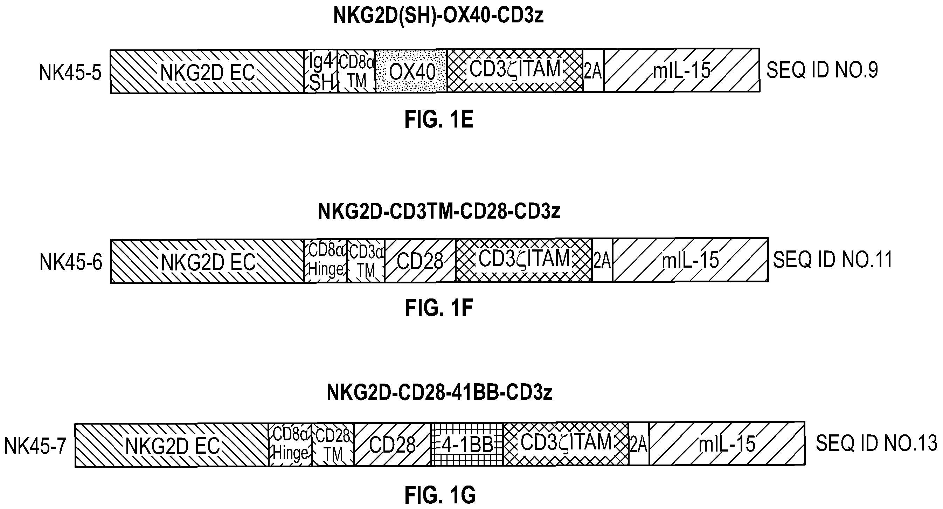

In several embodiments, there are provided polynucleotides encoding a NKG2D(SH)/OX40/CD3z cytotoxic receptor complex (see Figure 1E, NK45-5). Such receptor complexes comprise an extracellular domain comprising an NKG2D fragment that binds native ligands of NKG2D. The construct further comprises an IgG4 short hinge and a CD8α transmembrane domain, an OX40 signaling subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 9. In several embodiments, the cytotoxic receptor complex comprises the amino acid sequence of SEQ ID NO: 10. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 9, but remains, depending on the embodiment, at least 70%, at least 75%, at least 80%, at least 85%, at least 90%, or at least 95% homologous with SEQ ID NO. 9. Additionally, as indicated above, different extracellular domains may also be used, such as for example, that of SEQ ID NO. 23 or 25, as non-limiting examples.

In several embodiments, there are provided polynucleotides encoding a NKG2D/CD3TM/CD28/CD3z cytotoxic receptor complex (see Figure 1F, NK45-6). Such receptor complexes comprise an extracellular domain comprising an NKG2D fragment that binds native ligands of NKG2D. The construct further comprises a CD8α hinge and a CD3 transmembrane domain, a CD28 signaling subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 11. In several embodiments, the cytotoxic receptor complex comprises the amino acid sequence of SEQ ID NO: 12. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 11, but remains, depending on the embodiment, at least 70%, at least 75%, at least 80%, at least 85%, at least 90%, or at least 95% homologous with SEQ ID NO. 11. Additionally, as indicated above, different extracellular domains may also be used, such as for example, that of SEQ ID NO. 23 or 25, as non-limiting examples.

In several embodiments, there are provided polynucleotides encoding a NKG2D/CD28/4-1BB/CD3z cytotoxic receptor complex (see Figure 1G, NK45-7). Such receptor complexes comprise an extracellular domain comprising an NKG2D fragment that binds native ligands of NKG2D. The construct further comprises a CD8α hinge and a CD28 transmembrane domain, a CD28 signaling subdomain, a 4-1BB signaling subdomain and a CD3zeta ITAM subdomain (acting in concert as a signaling domain) as well as an IL-15 domain (acting as a costimulatory domain). In several embodiments, this cytotoxic receptor complex is encoded by the nucleic acid sequence of SEQ ID NO: 13. In several embodiments, the cytotoxic receptor complex comprises the amino acid sequence of SEQ ID NO: 14. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 13, but remains, depending on the embodiment, at least 70%, at least 75%, at least 80%, at least 85%, at least 90%, or at least 95% homologous with SEQ ID NO. 13. Additionally, as indicated above, different extracellular domains may also be used, such as for example, that of SEQ ID NO. 23 or 25, as non-limiting examples.

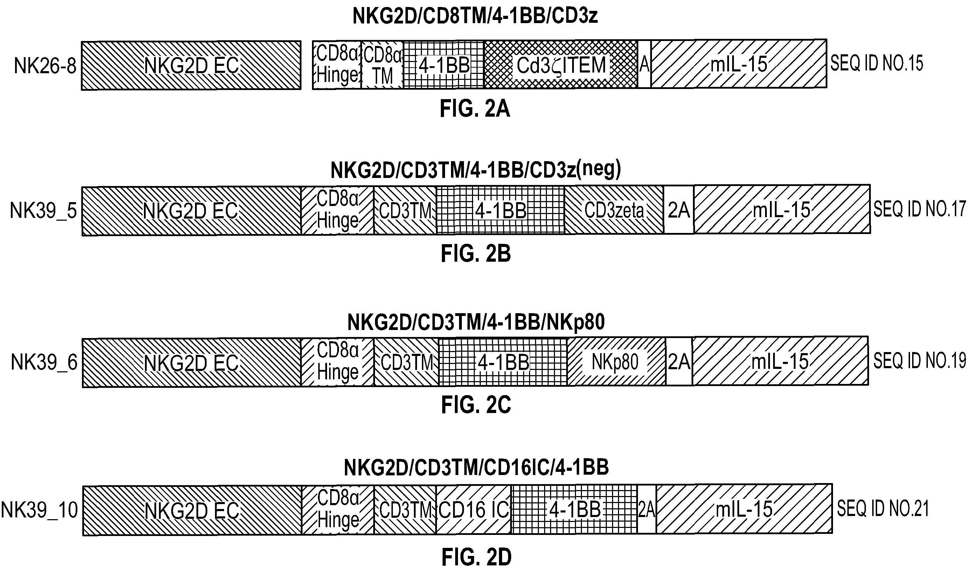

In several embodiments, there are provided polynucleotides encoding a NKG2D/CD8TM/4-1BB/CD3z cytotoxic receptor complex (see Figure 2A, NK26-8). Such receptor complexes comprise an extracellular domain comprising an NKG2D fragment that binds native ligands of NKG2D. The construct further comprises a CD8α hinge and a CD8α transmembrane domain, a 4-1BB signaling subdomain and CD3zeta ITAM subdomain (acting in concert as a signaling domain) as well as an IL-15 domain (acting as a co-stimulatory domain). In several embodiments, this cytotoxic receptor complex is encoded by the nucleic acid sequence of SEQ ID NO: 15. In several embodiments, the cytotoxic receptor complex comprises the amino acid sequence of SEQ ID NO: 16. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 15, but remains, depending on the embodiment, at least 70%, at least 75%, at least 80%, at least 85%, at least 90%, or at least 95% homologous with SEQ ID NO. 15. Additionally, as indicated above, different extracellular domains may also be used, such as for example, that of SEQ ID NO. 23 or 25, as non-limiting examples.

In several embodiments, there are provided polynucleotides encoding a NKG2D/CD3TM/4-1BB/CD3z(neg) cytotoxic receptor complex (see Figure 2B, NK39-5). Such receptor complexes comprise an extracellular domain comprising an NKG2D fragment that binds native ligands of NKG2D. The construct further comprises a CD8α hinge and a CD3 transmembrane domain, a 4-1BB signaling subdomain and non-ITAM CD3zeta subdomain (acting in concert as a signaling domain) as well as an IL-15 domain (acting as a co-stimulatory domain). In several embodiments, this cytotoxic receptor complex is encoded by the nucleic acid sequence of SEQ ID NO: 17. In several embodiments, the cytotoxic receptor complex comprises the amino acid sequence of SEQ ID NO: 18. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 18, but remains, depending on the embodiment, at least 70%, at least 75%, at least 80%, at least 85%, at least 90%, or at least 95% homologous with SEQ ID NO. 18. Additionally, as indicated above, different extracellular domains may also be used, such as for example, that of SEQ ID NO. 23 or 25, as non-limiting examples.

In several embodiments, there are provided polynucleotides encoding a NKG2D/CD3TM/4-1BB/NKp80 cytotoxic receptor complex (see Figure 2C, NK39-6). Such receptor complexes comprise an extracellular domain comprising an NKG2D fragment that binds native ligands of NKG2D. The construct further comprises a CD8α hinge and a CD3 transmembrane domain, a 4-1BB signaling subdomain and an NKp80 signaling subdomain (acting in concert as a signaling domain) as well as an IL-15 domain (acting as a co-stimulatory domain). In several embodiments, this cytotoxic receptor complex is encoded by the nucleic acid sequence of SEQ ID NO: 19. In several embodiments, the cytotoxic receptor complex comprises the amino acid sequence of SEQ ID NO: 20. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 19, but remains, depending on the embodiment, at least 70%, at least 75%, at least 80%, at least 85%, at least 90%, or at least 95% homologous with SEQ ID NO. 19. Additionally, as indicated above, different extracellular domains may also be used, such as for example, that of SEQ ID NO. 23 or 25, as non-limiting examples.

In several embodiments, there are provided polynucleotides encoding a NKG2D/CD3TM/CD16IC/4-1BB cytotoxic receptor complex (see Figure 2D, NK39-10). Such receptor complexes comprise an extracellular domain comprising an NKG2D fragment that binds native ligands of NKG2D. The construct further comprises a CD8α hinge and a CD3 transmembrane domain, CD16 intracellular signaling subdomain and a 4-1BB signaling subdomain and an NKp80 signaling subdomain (acting in concert as a signaling domain) as well as an IL-15 domain (acting as a co-stimulatory domain). In several embodiments, this cytotoxic receptor complex is encoded by the nucleic acid sequence of SEQ ID NO: 21. In several embodiments, the cytotoxic receptor complex comprises the amino acid sequence of SEQ ID NO: 22. As schematically depicted the construct includes IL-15, which in several embodiments, renders the NK cells expressing this construct particularly efficacious against target tumor cells. It shall be appreciated that the IL-15 can, in accordance with several embodiments, be encoded on a separate construct. In some embodiments, the sequence of the cytotoxic receptor complex may vary from SEQ ID NO. 21, but remains, depending on the embodiment, at least 70%, at least 75%, at least 80%, at least 85%, at least 90%, or at least 95% homologous with SEQ ID NO. 21. Additionally, as indicated above, different extracellular domains may also be used, such as for example, that of SEQ ID NO. 23 or 25, as non-limiting examples.

Optionally, depending on the embodiment, any of the polynucleotides disclosed herein may also encode truncations and/or variants of one or more of the constituent subunits of a cytotoxic receptor complex, while maintaining the ability to impart cytotoxic effects against a target cell. In addition, any of the polynucleotides disclosed herein may also optionally include codon-optimized nucleotide sequences for all or a portion of the various constituent domains of a cytotoxic receptor complex. As used herein, the terms "fragment" and "truncated" shall be given their ordinary meaning and shall also include N- and C-terminal deletion variants of proteins.

The polynucleotides encoding the cytotoxic receptor complexes described herein may be inserted into vectors to achieve expression in NK cells. In one embodiment, the polynucleotide is operably linked to at least one regulatory element for the expression of the cytotoxic receptor complex (e.g., a promoter). In specific embodiments, transcriptional regulatory elements are used, for example an internal ribosome entry site (IRES) or enhancer element, to direct the transcription of the cytotoxic receptor complex. In some embodiments, the polynucleotide comprises one or more cytosolic protease cleavage sites, as discussed herein. Depending on the embodiment, the various constituent parts of a cytotoxic receptor complex can be delivered to an NK cell in a single, or multiple, vectors. Regardless of the number of vectors used, any polynucleotide may optionally include a tag sequence, allowing identification of the presence of NK cells expressing the construct. For example, in several embodiments a FLAG tag (DYKDDDDK, SEQ ID NO. 42) is used. Also available are other tag sequences, such as a polyhistidine tag (His-tag) (HHHHHH, SEQ ID NO. 43), HA-tag or myc-tag (EQKLISEEDL; SEQ ID NO: 44). Alternatively, green fluorescent protein, or other fluorescent moiety, is used. Combinations of tag types can also be used, to individually recognize sub-components of a cytotoxic receptor complex.

In several embodiments, the polynucleotide encoding the cytotoxic receptor complex is an mRNA that may be introduced into NK cells, for example, by electroporation. In another embodiment, the vector is a virus, preferably a retrovirus, which may be introduced into NK cells by transduction. In several embodiments, the vector is a Murine Stem Cell Virus (MSCV). In additional embodiments, other vectors may be used, for example lentivirus, adenovirus, adeno-associated virus, and the like. In several embodiments, non-HIV-derived retroviruses are used. Alternatively, plasmids, phagemids, cosmids, viral vectors, phage, artificial chromosomes could be used.

In one embodiment, the cytotoxic receptor complexes are transiently expressed in the NK cells. In another embodiment, the cytotoxic receptor complex are stably expressed in NK cells. In an additional embodiment, the NK cells are autologous cells. In yet another embodiment, the NK cells are donor-derived (allogeneic) cells.

### Administration and Dosing

Further provided herein are populations of natural killer (NK) cells engineered to express a cytotoxic receptor complex for use in methods of treating a subject having cancer, and in particular hepatocellular carcinoma, comprising administering to the subject a composition comprising NK cells engineered to express a cytotoxic receptor complex as disclosed herein. In certain embodiments, such use in treatment of a subject with a genetically engineered cell(s) described herein achieves one, two, three, four, or more of the following effects, including, for example: (i) reduction or amelioration the severity of disease or symptom associated therewith; (ii) reduction in the duration of a symptom associated with a disease; (iii) protection against the progression of a disease or symptom associated therewith; (iv) regression of a disease or symptom associated therewith; (v) protection against the development or onset of a symptom associated with a disease; (vi) protection against the recurrence of a symptom associated with a disease; (vii) reduction in the hospitalization of a subject; (viii) reduction in the hospitalization length; (ix) an increase in the survival of a subject with a disease; (x) a reduction in the number of symptoms associated with a disease; (xi) an enhancement, improvement, supplementation, complementation, or augmentation of the prophylactic or therapeutic effect(s) of another therapy.

Administration can be by a variety of routes, including, without limitation, intravenous, intra-arterial, subcutaneous, intramuscular, intrahepatic, intraperitoneal and/or local delivery to an affected tissue. In several embodiments, administration is locally achieved to the liver through injection to, for example, the intra-hepatic artery. In additional embodiments, administration is local to accomplish treatment to other organs. For example, in several embodiments, compositions disclosed herein are administered to treat ovarian cancer. In some embodiments, such treatment is done via intraperitoneal administration. Doses of NK cells can be readily determined for a given subject based on their body mass, disease type and state, and desired aggressiveness of treatment, but range, depending on the embodiments, from about 10⁵ cells per kg to about 10¹² cells per kg (e.g., 10⁵-10⁷, 10⁷-10¹⁰, 10¹⁰-10¹² and overlapping ranges therein). In one embodiment, a dose escalation regimen is used. In several embodiments, a range of NK cells is administered, for example between about 1 x 10⁶ cells/kg to about 1 x 10⁸ cells/kg. Doses may also be determined, at least in part, by a desired effector:target ratio. In several embodiments, the dose can yield an effector:target ratio of about 100:1, about 50:1, about 25:1 10:1, about 5:1, about 3:1, about 2:1, about 1:1, about 1:2, about 1:5, about 1:10, about 1:25, about 1:50, about 1:100, or other ratios therebetween (including endpoints). Depending on the embodiment, various types of cancer can be treated. In several embodiments, hepatocellular carcinoma is treated by the use. Additional embodiments provided for herein include a use in the treatment or prevention of the following non-limiting examples of cancers including, but not limited to, acute lymphoblastic leukemia (ALL), acute myeloid leukemia (AML), adrenocortical carcinoma, Kaposi sarcoma, lymphoma, gastrointestinal cancer, appendix cancer, central nervous system cancer, basal cell carcinoma, bile duct cancer, bladder cancer, bone cancer, brain tumors (including but not limited to astrocytomas, spinal cord tumors, brain stem glioma, glioblastoma, craniopharyngioma, ependymoblastoma, ependymoma, medulloblastoma, medulloepithelioma), breast cancer, bronchial tumors, Burkitt lymphoma, cervical cancer, colon cancer, chronic lymphocytic leukemia (CLL), chronic myelogenous leukemia (CML), chronic myeloproliferative disorders, ductal carcinoma, endometrial cancer, esophageal cancer, gastric cancer, Hodgkin lymphoma, non-Hodgkin lymphoma, hairy cell leukemia, renal cell cancer, leukemia, oral cancer, nasopharyngeal cancer, liver cancer, liver dominant metastatic colorectal cancer, cholangiocarcinoma, lung cancer (including but not limited to, non-small cell lung cancer, (NSCLC) and small cell lung cancer), pancreatic cancer, bowel cancer, lymphoma, melanoma, ocular cancer, ovarian cancer, pancreatic cancer, prostate cancer, pituitary cancer, uterine cancer, and vaginal cancer.

In some embodiments, also provided herein are nucleic acid and amino acid sequences that have homology of at least 80%, 85%, 90%, 95%, 96%, 97%, 98%, 99% (and ranges therein) as compared with the respective nucleic acid or amino acid sequences of SEQ ID NOS. 1-44 and that also exhibit one or more of the functions as compared with the respective SEQ ID NOS. 1-44: including but not limited to, (i) enhanced proliferation, (ii) enhanced activation, (iii) enhanced cytotoxic activity against cells presenting ligands to which NK cells harboring receptors encoded by the nucleic acid and amino acid sequences bind, (iv) enhanced homing to tumor or infected sites, (v) reduced off target cytotoxic effects, (vi) enhanced secretion of immunostimulatory cytokines and chemokines (including, but not limited to IFNg, TNFa, IL-22, CCL3, CCL4, and CCL5), (vii) enhanced ability to stimulate further innate and adaptive immune responses, and (viii) combinations thereof.

Additionally, in several embodiments, there are provided amino acid sequences that correspond to any of the nucleic acids disclosed herein, while accounting for degeneracy of the nucleic acid code. Furthermore, those sequences (whether nucleic acid or amino acid) that vary from those expressly disclosed herein, but have functional similarity or equivalency are also contemplated within the scope of the present disclosure. The foregoing includes mutants, truncations, substitutions, or other types of modifications.

In several embodiments, polynucleotides encoding the disclosed cytotoxic receptor complexes are mRNA. In some embodiments, the polynucleotide is DNA. In some embodiments, the polynucleotide is operably linked to at least one regulatory element for the expression of the cytotoxic receptor complex.

Additionally provided, according to several embodiments, is a vector comprising the polynucleotide encoding any of the polynucleotides provided for herein, wherein the polynucleotides are optionally operatively linked to at least one regulatory element for expression of a cytotoxic receptor complex. In several embodiments, the vector is a retrovirus.

Further provided herein are engineered natural killer cells comprising the polynucleotide, vector, or cytotoxic receptor complexes as disclosed herein. In several embodiments, these NK cells are suitable for local delivery to a tumor site, such as the liver, for the treatment of prevention of disease, such as, hepatocellular carcinoma.

Additionally provided is a population of natural killer (NK) cells engineered to express a cytotoxic receptor complex for use in a method for treating a liver tumor, the method comprising administering to a patient with a liver tumor a composition comprising a population of natural killer (NK) cells engineered to express a cytotoxic receptor complex, wherein the cytotoxic receptor complex comprises an extracellular domain that binds a ligand of a Natural Killer Group 2D (NKG2D) receptor, wherein the ligand of NKG2D is selected from MICA, MICB, ULBP1, ULBP2, ULBP3, ULBP4, ULBP5, ULBP6, and combinations thereof, wherein the extracellular domain is encoded by at least a portion of the polynucleotide of SEQ ID NO. 23, and a cytotoxic signaling complex comprising a transmembrane domain, a co-stimulatory domain, and a signaling domain, and wherein the administration comprises an injection into the intra-hepatic artery of the patient. In several embodiments, the composition comprises between about 10⁷ and about 10¹⁰ NK cells. In several embodiments, the method further comprises administering to the patient IL-2. In several embodiments, the signaling domain of the cytotoxic signaling complex comprises a CD3zeta domain and wherein the extracellular domain is encoded by the polynucleotide of SEQ ID NO. 24. In several embodiments, the CD3zeta domain comprises at least one immunoreceptor tyrosine-based activation motif (ITAM) motif. In several embodiments, the signaling domain of the cytotoxic signaling complex further comprises an OX40 domain. In several embodiments, the signaling domain of the cytotoxic signaling complex further comprises a 4-1BB domain. In several embodiments, the signaling domain of the cytotoxic signaling complex further comprises a CD28 domain. In several embodiments, the signaling domain of the cytotoxic signaling complex further comprises an NKp80 domain.

Additionally provided is a population of natural killer (NK) cells engineered to express a cytotoxic receptor complex for use in a method for treating hepatocellular carcinoma, the method comprising administering to a patient with a hepatocellular carcinoma a composition comprising a population of natural killer (NK) cells engineered to express a cytotoxic receptor complex, wherein the cytotoxic receptor complex comprises an extracellular domain coupled to a transmembrane domain, the transmembrane domain further coupled to a signaling domain, wherein the extracellular domain comprises a fragment of a Natural Killer Group 2D (NKG2D) receptor, wherein the fragment the NKG2D receptor has at least 95% sequence identity to the NKG2D receptor fragment encoded by the polynucleotide of SEQ ID NO. 24, and wherein the administration comprises an injection into the intra-hepatic artery of the patient. In several embodiments, the cytotoxic receptor complex is encoded by the nucleic acid sequence of SEQ ID NO. 1. In several embodiments, the cytotoxic receptor complex has the amino acid sequence of SEQ ID NO. 2. In several embodiments, the cytotoxic receptor complex is encoded by the nucleic acid sequence of SEQ ID NO. 3. In several embodiments, the cytotoxic receptor complex has the amino acid sequence of SEQ ID NO. 4. In several embodiments, the cytotoxic receptor complex is encoded by the nucleic acid sequence of SEQ ID NO. 5. In several embodiments, the cytotoxic receptor complex has the amino acid sequence of SEQ ID NO. 6. In several embodiments, the cytotoxic receptor complex is encoded by the nucleic acid sequence of SEQ ID NO. 7. In several embodiments, the cytotoxic receptor complex has the amino acid sequence of SEQ ID NO. 8. In several embodiments, the cytotoxic receptor complex is encoded by the nucleic acid sequence of SEQ ID NO. 9. In several embodiments, the cytotoxic receptor complex has the amino acid sequence of SEQ ID NO. 10. In several embodiments, the cytotoxic receptor complex is encoded by the nucleic acid sequence of SEQ ID NO. 11. In several embodiments, the cytotoxic receptor complex has the amino acid sequence of SEQ ID NO. 12. In several embodiments, the cytotoxic receptor complex is encoded by the nucleic acid sequence of SEQ ID NO. 13. In several embodiments, the cytotoxic receptor complex has the amino acid sequence of SEQ ID NO. 14. In several embodiments, cytotoxic receptor complex is encoded by the nucleic acid sequence of SEQ ID NO. 15. In several embodiments, the cytotoxic receptor complex has the amino acid sequence of SEQ ID NO. 16. In several embodiments, the cytotoxic receptor complex is encoded by the nucleic acid sequence of SEQ ID NO. 17. In several embodiments, the cytotoxic receptor complex has the amino acid sequence of SEQ ID NO. 18. In several embodiments, the cytotoxic receptor complex is encoded by the nucleic acid sequence of SEQ ID NO. 19. In several embodiments, the cytotoxic receptor complex has the amino acid sequence of SEQ ID NO. 20. In several embodiments, the cytotoxic receptor complex is encoded by the nucleic acid sequence of SEQ ID NO. 21. In several embodiments, the cytotoxic receptor complex has the amino acid sequence of SEQ ID NO. 22. In several embodiments, the injection into the intra-hepatic artery of the patient is a percutaneous injection. In several embodiments, the NK cells are autologous to the patient. In several embodiments, the method further comprises administering IL-2. In several embodiments, the method further comprises administering an additional anti-cancer agent. In several embodiments, the additional anti-cancer agent is a tyrosine kinase inhibitor. In several embodiments, the tyrosine kinase inhibitor is selected from the group consisting of sorafenib, nilotinib, imantinib, gefitinib, erlotinib, sunitinib, adavosertib, lapatinib, and combinations thereof.

Additionally provided is a population of natural killer (NK) cells engineered to express a cytotoxic receptor complex for use in a method for treating a primary liver tumor, the method comprising administering to a patient with a hepatocellular carcinoma a composition comprising a population of natural killer (NK) cells engineered to express a cytotoxic receptor complex, wherein the cytotoxic receptor complex comprises an extracellular domain coupled to a transmembrane domain, the transmembrane domain further coupled to a signaling domain, wherein the extracellular domain comprises a fragment of a Natural Killer Group 2D (NKG2D) receptor, wherein the cytotoxic receptor complex comprises an amino acid sequence at least 90% identical in sequence to SEQ ID NO: 8; and administering at least one additional anti-cancer agent prior to, concurrent with, or after the population of NK cells is administered, wherein the additional anti-cancer agent is sorafenib, nilotinib, imantinib, gefitinib, erlotinib, sunitinib, adavosertib, lapatinib, and combinations thereof.

Additionally provided is a system for combination therapy for treating a primary liver tumor, comprising a composition comprising a population of natural killer (NK) cells engineered to express a cytotoxic receptor complex, wherein the cytotoxic receptor complex comprises an extracellular domain coupled to a transmembrane domain, the transmembrane domain further coupled to a signaling domain, wherein the extracellular domain comprises a fragment of a Natural Killer Group 2D (NKG2D) receptor, wherein the cytotoxic receptor complex comprises an amino acid sequence at least 90% identical in sequence to SEQ ID NO: 8; and at least one additional anti-cancer agent, wherein the additional anti-cancer agent is sorafenib, nilotinib, imantinib, gefitinib, erlotinib, sunitinib, adavosertib, lapatinib, and combinations thereof, and wherein the additional anti-cancer agent is configured for administration to a subject with a primary liver tumor prior to, concurrent with, or after the population of NK cells is administered.

Additionally provided is a composition according to any one of the embodiments described herein.

Additionally provided is a composition or use thereof in treating tumors in the liver or other organs according to any one of the embodiments described herein.

Additionally provided is a composition according to any one of the embodiments described herein wherein engineered NK cells exhibit at least a 3-fold increase in cytotoxicity as compared to native NK cells.

Additionally provided is a composition or use thereof in treating tumors in the liver or other organs according to any one of the embodiments described herein, wherein engineered NK cells exhibit at least a 3-fold increase in cytotoxicity as compared to native NK cells.

Additionally provided is a population of natural killer (NK) cells engineered to express a cytotoxic receptor complex for use in a method for treating a tumor, the method comprising accessing a blood supply vessel of a patient with a tumor, wherein the tumor receives at least a portion of its blood supply via the blood supply vessel, and administering a population of natural killer (NK) cells engineered to express a cytotoxic receptor complex, wherein the cytotoxic receptor complex comprises a functional fragment of a C-type lectin-like receptor, and a cytotoxic signaling complex comprising a transmembrane domain, a co-stimulatory domain, and a signaling domain, and wherein the C-type lectin-like receptor comprises a Natural Killer Group 2D (NKG2D) receptor, wherein the functional fragment of the C-type lectin-like receptor comprises SEQ ID NO. 24, wherein the engineered NK cells exhibit at least a 3-fold increase in potency against a tumor cell as compared to non-engineered NK cells; and administering at least one additional anti-cancer agent prior to, concurrent with, or after the population of NK cells is administered. In several embodiments, the additional anti-cancer agent is a tyrosine kinase inhibitor. In several embodiments, the additional anti-cancer agent is Sorafenib, nilotinib, imantinib, gefitinib, erlotinib, sunitinib, adavosertib, lapatinib, and combinations thereof. In several embodiments, the signaling domain of the cytotoxic signaling complex comprises an OX40 domain.

### EXAMPLES

The methods and materials described below are non-limiting examples of methods and materials that can be used in accordance with the embodiments disclosed herein.

### Hepatocellular Carcinoma

### Background:

Natural killer (NK) cells offer an alternative, or a supplement in several embodiments, to T cells for cellular immunotherapy, as they are not HLA-restricted, and are therefore suitable for allogeneic off-the-shelf use. Expanded NK cells can be engineered to express chimeric receptors to further improve their cytotoxicity against both hematopoietic and solid tumors. In healthy humans, a high proportion of intrahepatic lymphocytes are NK cells, and for cases of hepatocellular carcinoma (HCC), infiltration of CD56+ NK cells positively correlates with cell apoptosis and patient survival. Multiple ligands of the natural killer group 2D (NKG2D) receptor are highly expressed in HCC tissues, but at low levels on healthy tissues, making NKG2D an attractive candidate for NK cell engineering to target HCC. Engineered NK cells according to several embodiments disclosed herein, collectively referred to as NKX101, are engineered to express an NKG2D chimeric receptor can improve their cytotoxicity against HCC cells, enhance pro-inflammatory cytokine response, and control tumor burden. Additional experiments were performed related to primary liver metastases pursuant to colorectal cancer.

### Materials and Methods:

NK cells from peripheral blood mononuclear cells were expanded using co-culture with irradiated K562-mbIL15-41BBL stimulatory cells. Non-limiting examples of such cells are provided in US Patent No. 7435596 or 8026097. In several embodiments, further modifications, such as those disclosed in PCT Application No. PCT/SG2018/050138, are used. NKX101 cells were generated by transduction with a bicistronic virus encoding NKG2D, an intracellular OX40 costimulatory domain, CD3ζ signaling domain, and membrane-bound IL-15, which supports prolonged cell survival and proliferation. As discussed herein, NKX101 refers not only to the specific construct utilized in this non-limiting example, but also to other cytotoxic receptor constructs disclosed herein. NKX101 NK cell pro-inflammatory cytokine release in response to 24 hr co-culture with HCC cell lines at a 1:1 ratio was analyzed by Luminex. The *in vitro* cytotoxicity of NKX101 NK cells at 4, 24 and 48 hr endpoints was measured using a panel of HCC cell lines engineered to express luciferase. *In vivo* activity of NKX101 NK cells was evaluated using an NSG mouse xenograft tumor model in which intraperitoneal (IP) injection of 4 x 10⁶ luciferase expressing SNU449 HCC cells was followed one week later with IP administration of 3 x 10⁶ NKX101 NK cells or unmodified NK cells (NT NK), and tumor growth was measured using IVIS bioluminescence imaging. Combination with a sub-EC50 concentration of the kinase inhibitor Sorafenib (used here as a non-limiting example of an additional anti-cancer agent), an approved agent for HCC, was tested for additive cytotoxic effect with at low E:T ratios of NKX101 NK cells *in vitro* at 48 hrs. Expression of NKG2D ligands was assessed by flow cytometry and analyzed for linear correlation with NKX101 NK cell cytotoxicity across a panel of HCC cell lines.

### Results:

In brief, the results demonstrate that NKX101 NK cells have dramatically improved cytotoxicity versus NT NK cells (NT NK) against a panel of HCC cell lines in vitro. Co-culture of NKX101 NK cells with HCC cell lines was shown to enhance pro-inflammatory cytokine release. In an *in vivo* NSG mouse xenograft HCC model, NKX101 NK cells provided complete tumor clearance in 4/5 mice, in contrast to stable control by NT NKs. Combination with the kinase inhibitor Sorafenib provided additive effect with NKX101 NK cells *in vitro.* High expression of NKG2D ligands was observed by flow cytometry, and while no individual ligand demonstrated a significant correlation with NKX101 NK cell cytotoxicity, in several embodiments an agent is developed that can identify NKG2D ligand expression and correlate that expression with susceptibility to the cytotoxic effects from NKX101 NK cells.

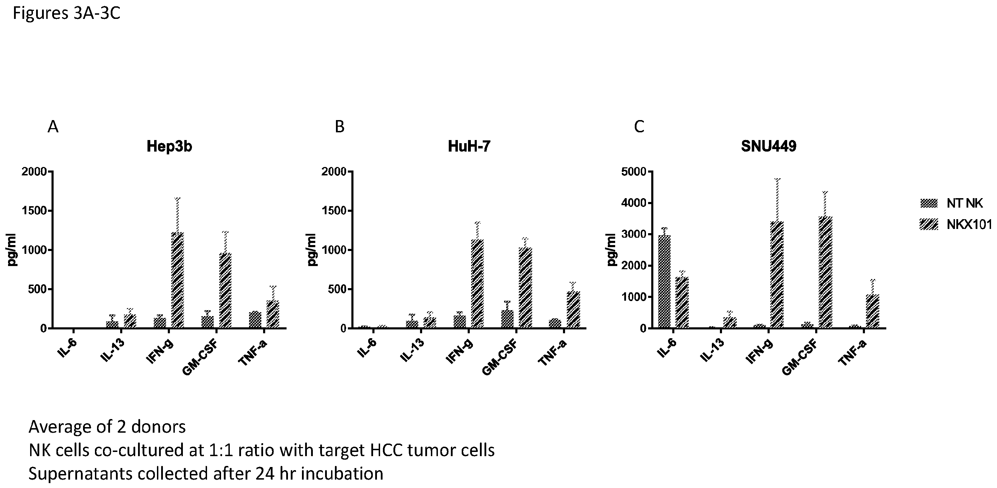

In greater detail, in order to assess the ability of various engineered NK cells as disclosed herein, several experiments were undertaken to assess the efficacy of the cells in an in vitro setting against hepatocellular carcinoma cell lines, in an in vivo animal model. Additional experiments relate to assessing the efficacy of engineered NK cells as disclosed herein when used in combination with another anti-cancer agent, with the TKI inhibitor Sorafenib as a non-limiting example of such an agent. Figures 3A-3B depict data related to the expression of various indicators of cytotoxicity after exposing HCC cells to native NK cells or engineered NK cells according to embodiments disclosed herein. In the non-limiting examples discussed hereafter, the designation of NKX101 refers to an engineered NK cell that expresses a truncated NKG2D extracellular domain capable of binding ligands of the NKG2D receptor. In several embodiments the truncated NKG2D domain is coupled to a CD8alpha hinge and CD8alpha TM domain. In several embodiments, the truncated NKG2D domain is coupled to an OX40 co-stimulatory domain and a CD3zeta signaling domain. In several embodiments, the construct further comprises membrane bound IL15. In several embodiments, the NKX101 has the nucleotide sequence of SEQ ID NO: 7 or the amino acid sequence set forth in SEQ ID NO: 8. However, in several embodiments other NKG2D constructs as disclosed herein (all NKG2D constructs are collectively referred to as NKX101 NK cells) operate in similar fashion and produce similar results to those discussed below. As disclosed herein in several embodiments, nucleotides or amino acid sequences having ~85%, 90%, 95% or more sequence homology with SEQ ID 7 or 8 (or any other construct disclosed herein) are cytotoxic against HCC cells.

Returning to Figure 3, Panels 3A-3C depict the concentration of various cytokines from the supernatant of a co-culture of the indicated HCC cell lines with either non-transduced NK cells (NT NK) or NKX101, at a 1:1 effector:target ratio. As shown, the co-culture of HCC cells with NKX101 induces greater expression of various cytokines that are indicative of cytotoxic signaling from the NKX101 NK cells. For example, in co-culture of Hep3b cells with NKX101 NK cells, there is a much greater concentration of IFNg in the supernatant as compared to with NT NK cells. This indicates that the NKX101 NK cells have enhanced secretion of immunostimulatory cytokines and chemokines. In several embodiments, cytokines and chemokines other than those measured here are secreted at greater levels (e.g., IL-22, CCL3, CCL4, and/or CCL5).

Figured 4A-4J depict data related to the degree of cytotoxicity induced by engineered NK cells as disclosed herein (NKX101 NK cells) against nine different HCC cell lines at the indicated effector:target ratios. Figures 4A-4I depict the data for each HCC line, as % cytotoxicity. By way of example, Figure 4A indicates that at a 2:1 effector:target ratio, NKX101 NK cells induce cytotoxicity in nearly 100% of the Hep3b cells in co-culture. As the E:T ratio is dropped to 1:1, the effects are reduced to about 90% cytotoxicity, and at 1:2 E:T, NKX101 is still able to kill ~75% of the target cells. At each of the E:T ratios, the NKX101 NK cells kill much more than the NT NK cells, which induce cytotoxicity in about 20% of the Hep3b cells in co-culture. Each of the other HCC cell lines exhibited a greater sensitivity to NKX101 cells as compared to NT NK cells at almost all E:T ratios. At 1:2 E:T, HuH-7 cells react approximately equivalently to NKX101 NK cells and NT NK cells. However, in several embodiments, an adjunct agent can be used to enhance the effects of NKX101 (described below). Figure 4J depicts a summary of the resistance (lack of sensitivity) of the HCC cells to NKX101. These data demonstrated that at the lowed E:T tested, the majority of the HCC cells were still subject to NKX101-induced cytotoxicity to some degree.

Figures 4K-4S depict additional data related to the degree of cytotoxicity of engineered NK cells from two additional donors against nine different hepatocellular carcinoma cell lines. Data are shown for both engineered NK cells that express an NKG2D chimeric receptor as disclosed herein (labeled NKX101 plus the donor number) and for non-transduced NK cells from the corresponding donor. As shown in Figure 4K, NK cells engineered to express NKX101 exhibited substantial cytotoxic effects against Hep3b cells, even at an effector:target ratio of 1:2. At that ratio, both sets of cells achieved approximately 70-75% cytotoxicity. As the ratio of the effector cells to target cells with increased, corresponding increases in cytotoxicity were observed, with approximately 80%-90% at a 1:1 ratio, and nearly 100% cytotoxicity at a 2:1 ratio. While the percent cytotoxicity at each effector:target ratio varied across the other eight hepatocellular carcinoma cell lines, the overall pattern to the data indicates that engineered NK cells exhibit progressively higher degrees of cytotoxicity as the effector:target ratio is increased. Moreover, these data indicate that, according to several embodiments 100% cytotoxicity against the target cell line can be achieved using engineered NK cells as disclosed herein. Shown in a different way in Figures 4T-4BB, the cytotoxic effects of engineered NK cells (and the corresponding on transduced NK cells from the same donors) are expressed as the percent of control luciferase signal detected, with a decrease in luciferase detection corresponding to an increase in the cytotoxic effects on the indicated cell line. Data in these figures is the mean of the two donors shown in Figures 4K-4S. These summary data further support the conclusion that engineered NK cells according to embodiments disclosed herein can yield a substantial increase in cytotoxic effects against a variety of cancer cell lines. Thus, according to several embodiments engineered NK cells as disclosed herein provide for an effective anticancer therapy. In some embodiments, as disclosed in more detail herein, use of engineered NK cells in conjunction with an additional anticancer agent leads to a synergistic cytotoxic impact on target cells and unexpectedly further enhances the ability to treat tumors, in particular solid tumors such as those in the liver due to hepatocellular carcinoma (or another liver cancer) or even for distant tumor types that have primary metastases in the liver (such as, for example colorectal cancer).

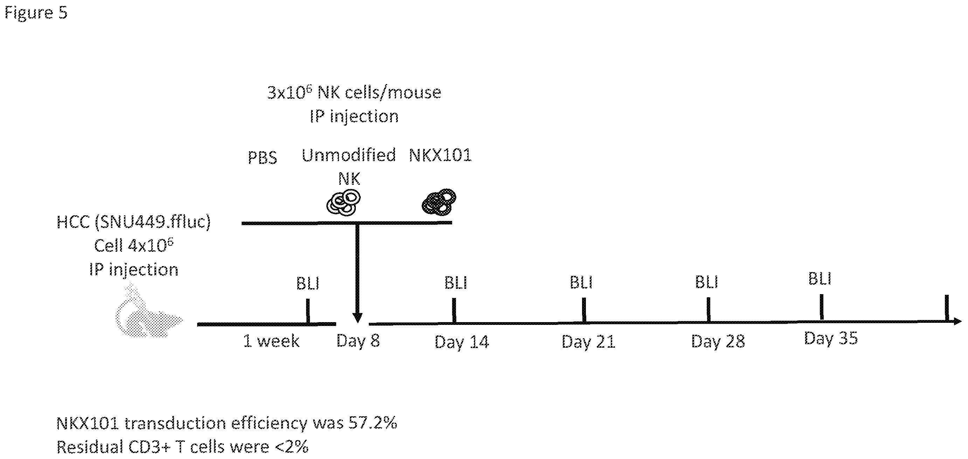

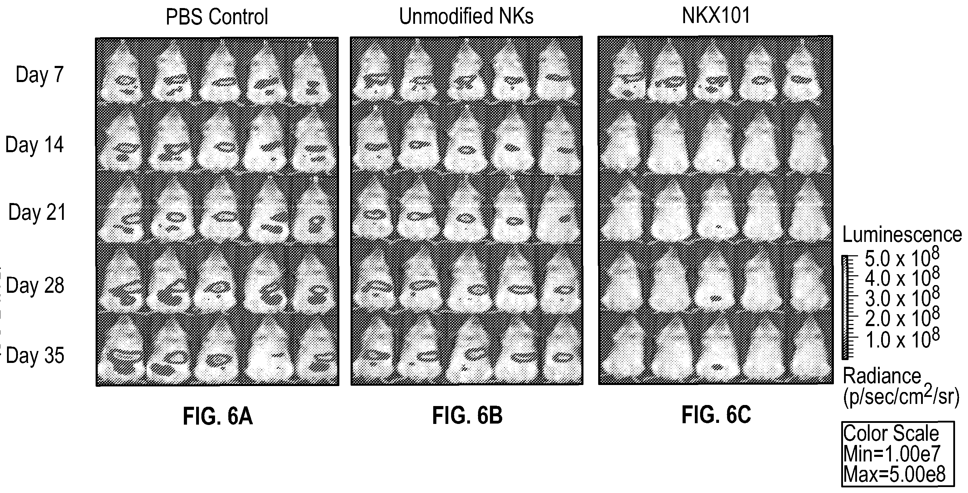

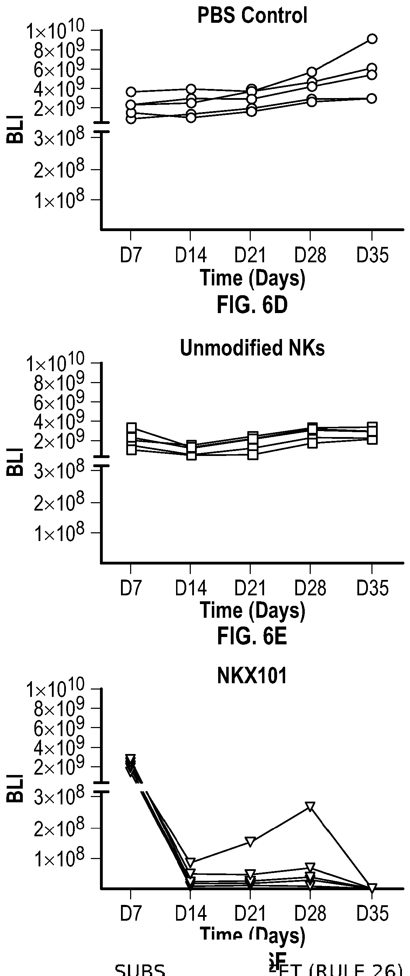

Figure 5 depicts a schematic timeline for an in vivo tumor modeling experiment. Mice received an injection of 4x10⁶ HCC cells (specifically SNU449 cells expressing firefly luciferase). Eight days later, 3x10⁶ NK cells (either NT NK cells or NKX101 NK cells) were delivered by IP injection. Bioluminescent imaging was performed every 7 days thereafter to assess tumor burden. Figures 6A-6F show the results of the in vivo tumor burden experiment. Figure 6A shows the bioluminescent imaging for mice receiving a PBS control. As depicted, significant tumor burden was present after 7 days, with nearly consistent tumor growth in all animals. Figure 6D depicts the intensity of the bioluminescent imaging signal over time. Figure 6B shows the same data for animals receiving unmodified NKs. In contrast to PBS, unmodified NKs appeared to hold tumor burden relatively steady over time - avoiding the increase seen in PBS-treated animals, but without significant reduction (see also 6E). Figure 6F shows the same data for animals treated with NKX101 NK cells. In stark contrast to even native NK cells, tumor burden was sharply reduce as early as 14 days post-NKX101 delivery. Tumor burden approached zero in 4 of the 5 mice shown in 6C. These data are reflected in Figure 6F as well. These results demonstrate a significant anti-tumor effect induced by NKX101 NK cells.

As discussed herein, according to several embodiments, one or more additional anti-cancer agents can be administered in conjunction with engineered NK cells disclosed herein. In several embodiments, the multiple mechanisms of action at play result in a synergistic effect (e.g., reduction in tumor burden, tumor growth rate, tumor cell persistence etc.). In other words, in several embodiments the use of engineered NK cells with an additional anti-cancer agent yield unexpectedly enhanced anti-cancer effects. By way of non-limiting example, one such additional anti-cancer agent that can be used in conjunction with engineered NK cells are tyrosine kinase inhibitors. Sorafenib is a small molecule which inhibits Raf-1, B-Raf and VEGFR-2 signaling pathways, and is presently widely used as a front-line therapy for hepatocellular carcinoma. Figure 7A, shows data related to a dose-response curve to calculate the EC50 of sorafenib against the indicated hepatocellular carcinoma cell lines. EC50 values are shown in Figure 7B. Sensitivity of the various hepatocellular carcinoma cell lines 2 sorafenib varied, with Huh-7 being the most sensitive and requiring a sorafenib concentration of 7.955 micromolar to achieve 50% cytotoxicity. On the other end of the spectrum, SNU-387 cells required 29.45 micromolar sorafenib in order to achieve 50% cytotoxicity. Figure 7C-7E depict data related to the use of sorafenib with engineered NK cells. Shown are data for the indicated HCC cells where they were treated without NK cells (Sorafenib control) and either NK cells alone, NK cells with the TKI Sorafenib, NKX101 NK cells, or NKX101 NK cells with Sorafenib. These data indicate that, despite the significant cytotoxic effects of NKX101 NK cells discussed above, administration with an additional anticancer agent such as a TK inhibitor like Sorafenib can still increase cytotoxic effects. As indicated the NKX101 with Sorafenib allowed for greater cytotoxicity as compared to NKX101 NK cells, notably even at the lower E:T ratios. The cytotoxic effects were greater than NK cells alone (lowest trace in each graph), and even outpaced those seen with NK cells plus Sorafenib. In several embodiments, such combination therapy allows for synergistic anti-cancer effects and an improved outcome for patients.

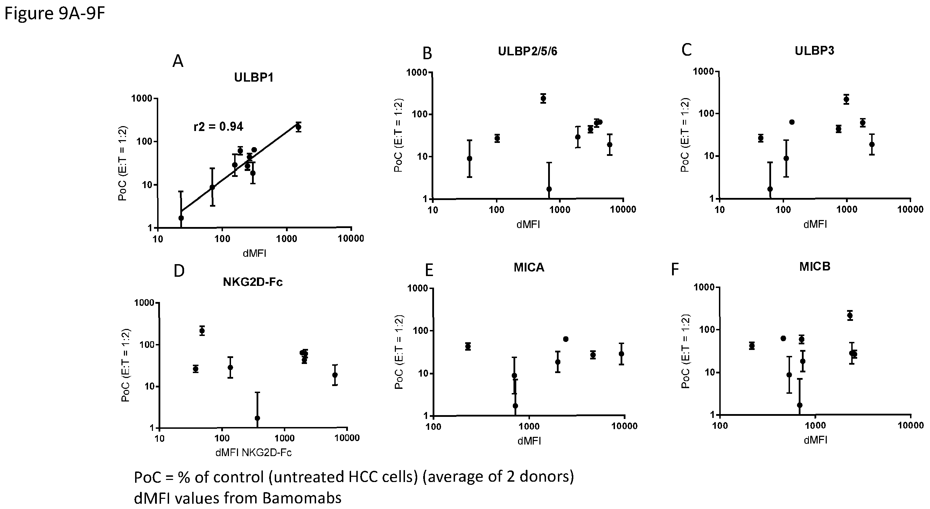

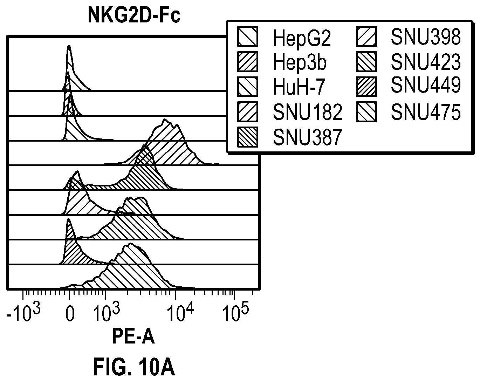

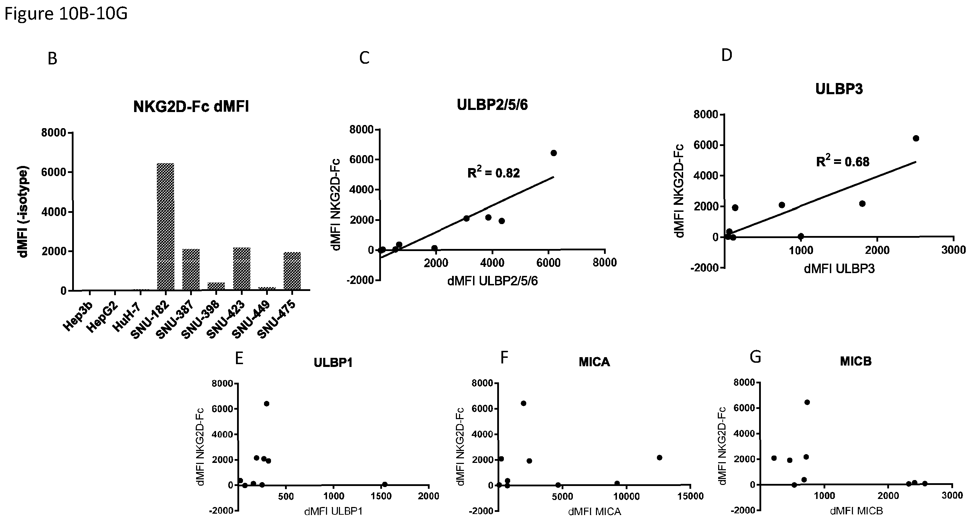

Figure 8A-8a depicts the expression of various NKG2D ligands on the indicated HCC cells. Figure 9 relates to the potential correlation between the degree of expression of the indicated ligand of NKG2D with the corresponding Percent of Control (untreated HCC cells) for each of the 9 HCC lines tested. Figure 10A-10G relate to experiments to develop reagent that functions to identify when an NKG2D ligand is overexpressed. The NKG2D receptor was fused with the Fc region of IgG. The binding of this fusion to the various HCC cells was measured by flow cytometry (Figure 10A) and also by way of an antibody against the Fc region (10B). This approach would allow a tagging of cells with elevated expression of one of the NKG2D ligands to be identified (the NKG2D portion of the fusion binds the ligand, and the Fc portion serves as a tag for detecting the cell to which the fusion is bound). Figures 10C-G show the correlation between binding/detection of NKG2D-FC and the indicated NKG2D ligand.

Data were also generated to demonstrate the enhanced potency (e.g., cytotoxicity) of engineered NK cells according to embodiments disclosed herein as compared to native NK cells. Hep3b cells and SNU449 cells (both non-limiting examples of tumor cells) were engineered to express luciferase. Two separate donors were used, and NK cells were collected. Native NK cells were not genetically modified, while NKX101 cells were generated through modification of NK cells such that they express a CAR as disclosed herein. As is disclosed in several embodiments, in this experiment, NKX101 cells were generated by transduction with a bicistronic virus encoding NKG2D, an intracellular OX40 costimulatory domain, CD3ζ signaling domain, and membrane-bound IL-15, which supports prolonged cell survival and proliferation. It shall be appreciated that NK cells can be modified with other constructs disclosed herein. Both sets of cells (NKX101 and unmodified (NT NK) NK cells) were expanded with K562-mbIL15-41BBL stimulatory cells. Cytotoxicity was evaluated for each NK cell type against Hep3b or SNU449 cells at various effector:target ratios. EC50 values were calculated for each of the NT NK cells and NKX101 (EC50 values were calculated using Prism 4-parameter non-linear regression analysis.)

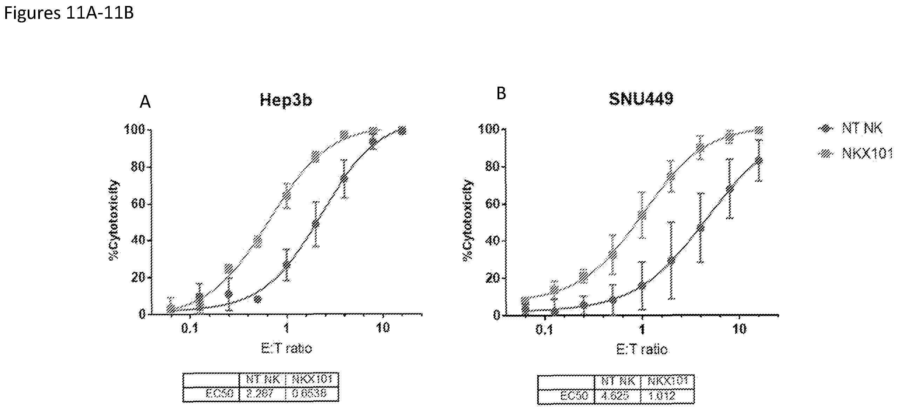

Figure 11A depicts the EC50 data of NT NK cells and NKX101 against Hep3b cells. These data confirm that NKX101 cells have enhanced cytotoxicity as compared to unmodified NK cells, as evidenced from this assay by the smaller number of NKX101 cells required to achieve 50% cytotoxicity against the Hep3b target cells. In fact, in this assay, NKX101 cells were over 3.5-fold more cytotoxic than NT NK cells. Figure 11B shows similar data depicting the significantly enhanced cytotoxicity of NKX101 as compared to NT NK cells. Acting against SNU449 cells, NKX101 was over 4.5-fold more potent. These data demonstrate that, as disclosed herein according to several embodiments, engineered NK cells have enhanced cytotoxicity as compared to non-engineered NK cells. Depending on the embodiment, engineered NK cells are enhanced (e.g., show increased cytotoxicity) relative to native NK cells by about 2-fold to about 20-fold, including about 2-fold, about 3-fold, about 5 fold, about 8-fold, about 10-fold, about 15-fold, about 20-fold, or any amount therebetween, including endpoints of the listed ranges.

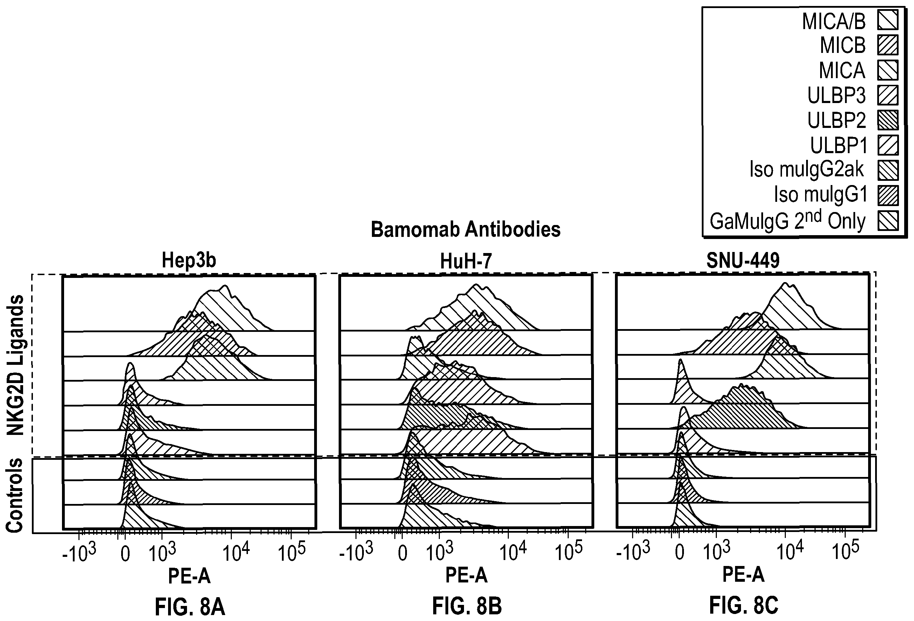

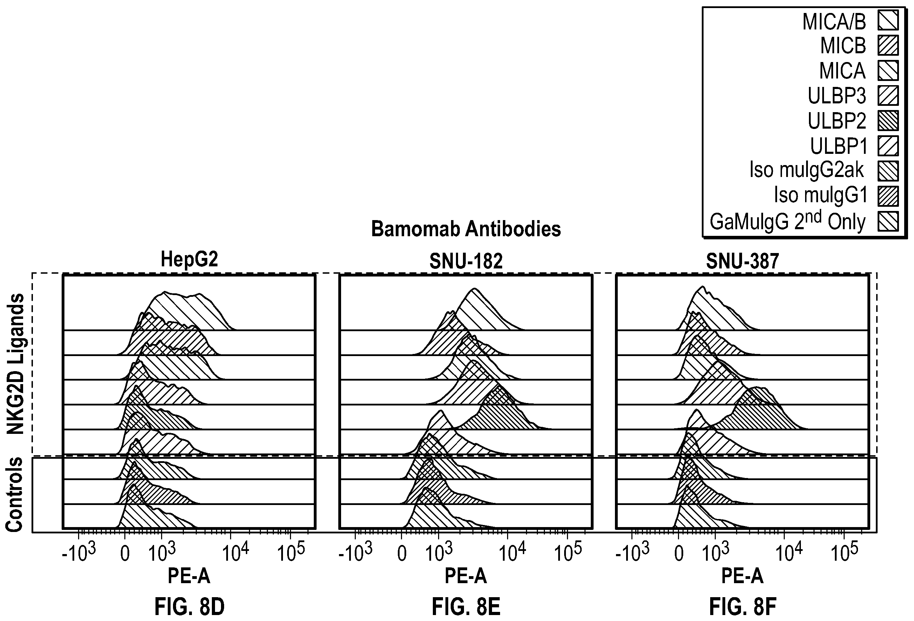

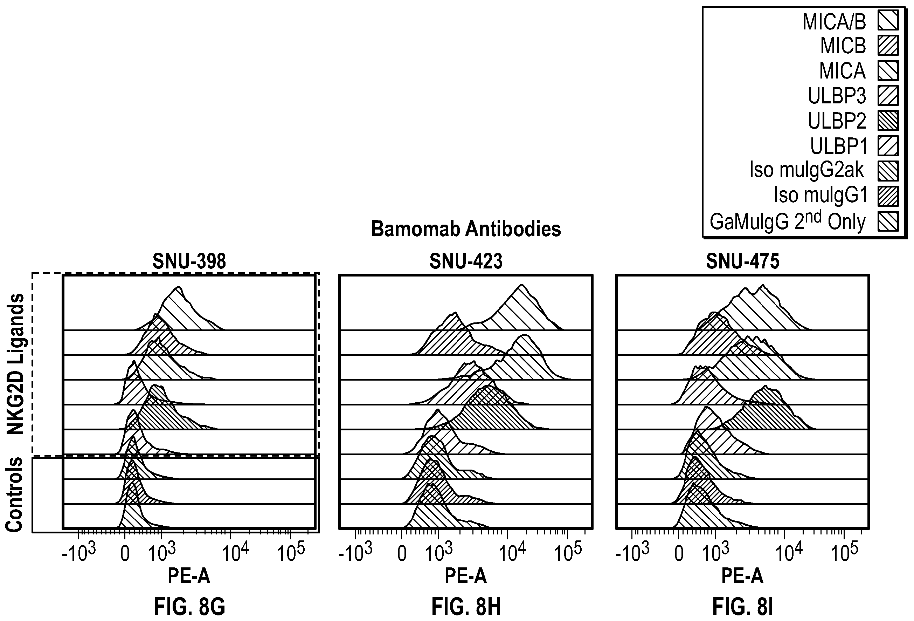

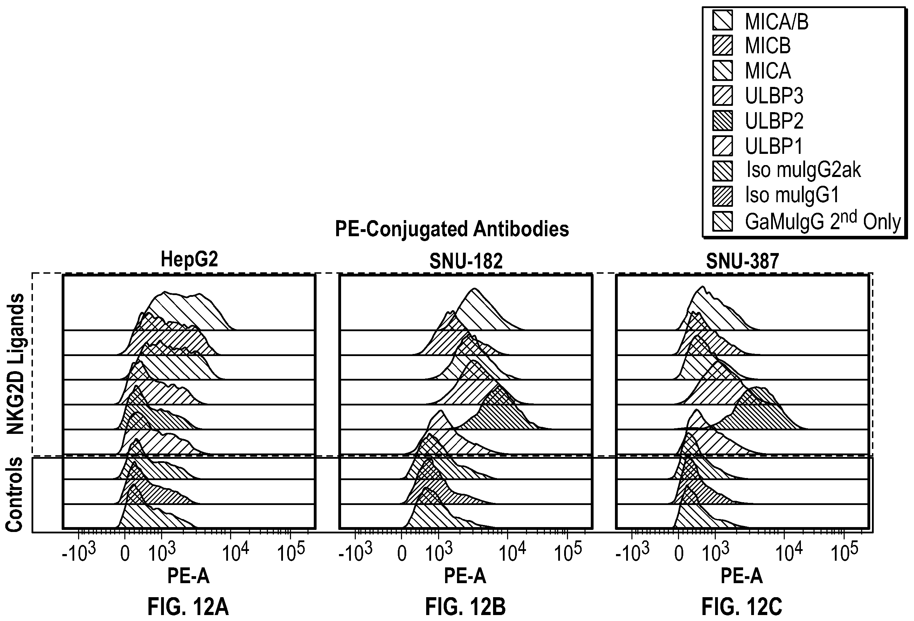

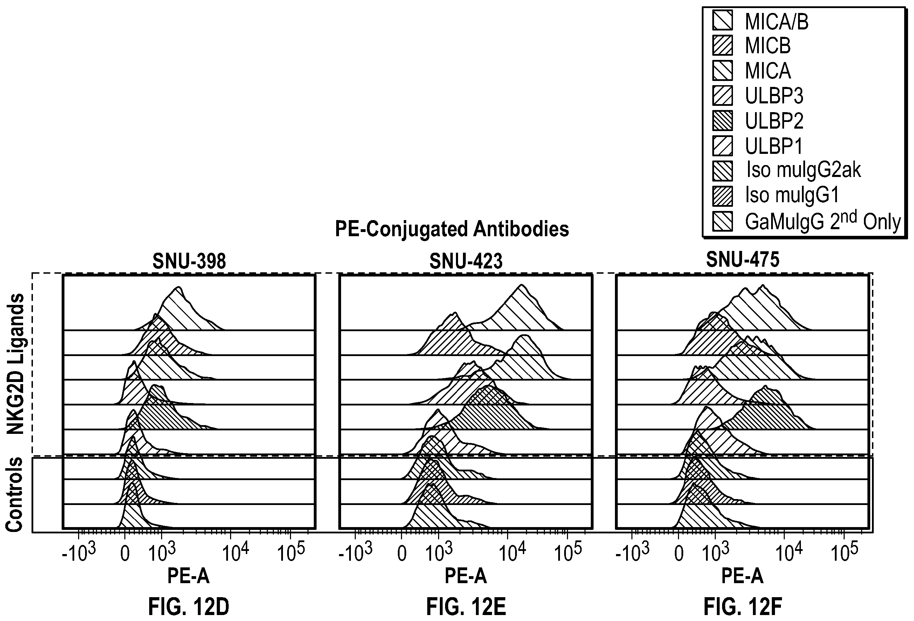

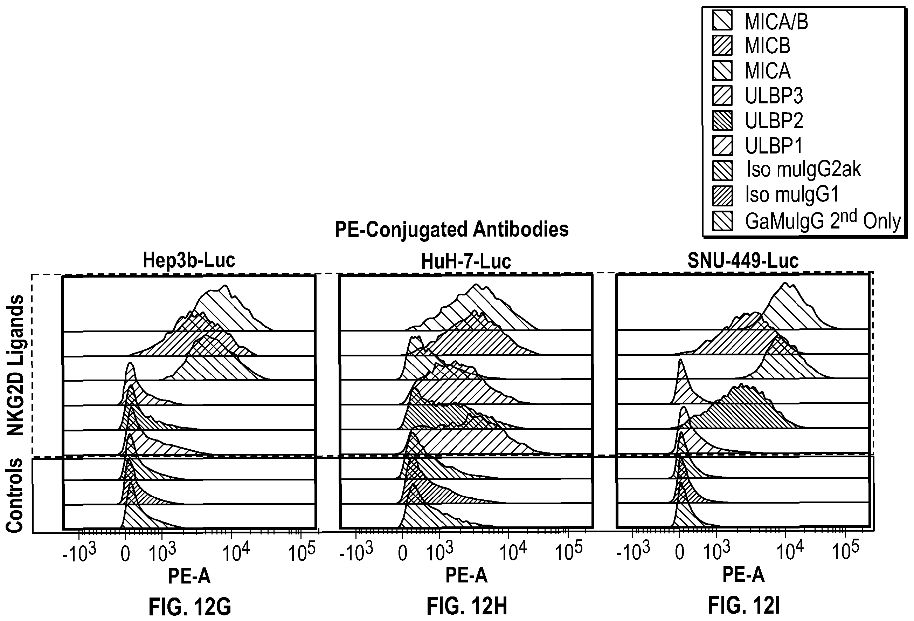

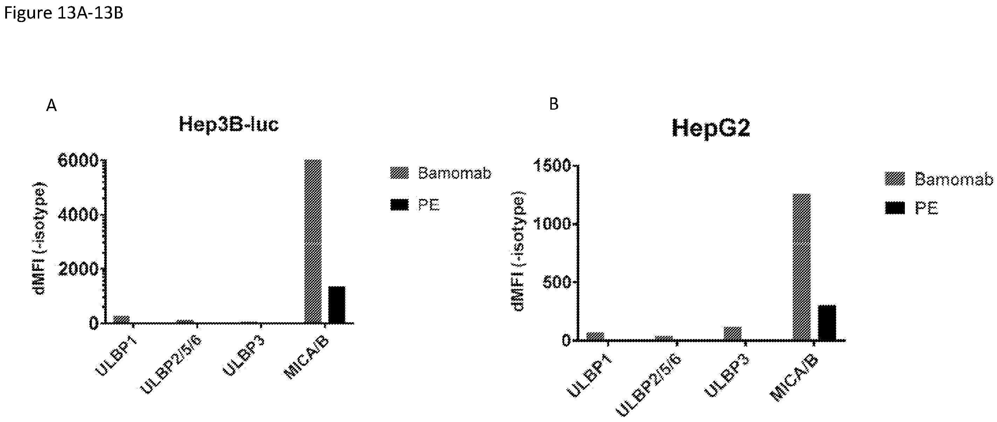

Figures 12A-12I depict flow cytometry data related to the expression of various NKG2D ligands on each of nine hepatocellular carcinoma cell lines, as measured by PE conjugated antibodies (R&D Systems, Minneapolis Minnesota, USA). NKG2D ligand expression as shown surrounded by a dashed box, of the corresponding isotype controls are shown surrounded by a solid box. Figures 13A-13I show the comparative data between the antibodies used to generate the data shown in Figure 12 as compared to those antibodies used to generate the data shown in Figure 8. Data are shown as the delta in Mean Fluorescence Intensity (dMFI, calculated as the fluorescent signal for the indicated target minus the background fluorescence from the corresponding isotype control antibody). While the absolute values for the change in intensity differed modestly depending on which antibody type was used, across the nine hepatocellular carcinoma lines, the general pattern of expression of NKG2D ligands was consistent, regardless of which antibody was used. While certain cell lines, such as SNU-182 and HuH-7 showed significant expression of several NKG2D ligands, other cell lines, such as He3B and HepG2, showed limited expression of NKG2D ligands other than MICA/MICB. Across all cell lines the greatest expression appeared to be in connection with ULBP2/5/6 and MICA/MICB.

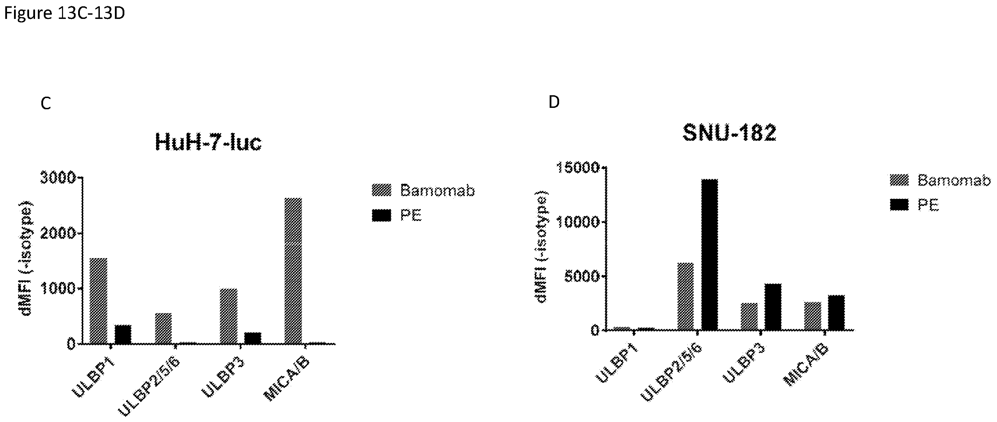

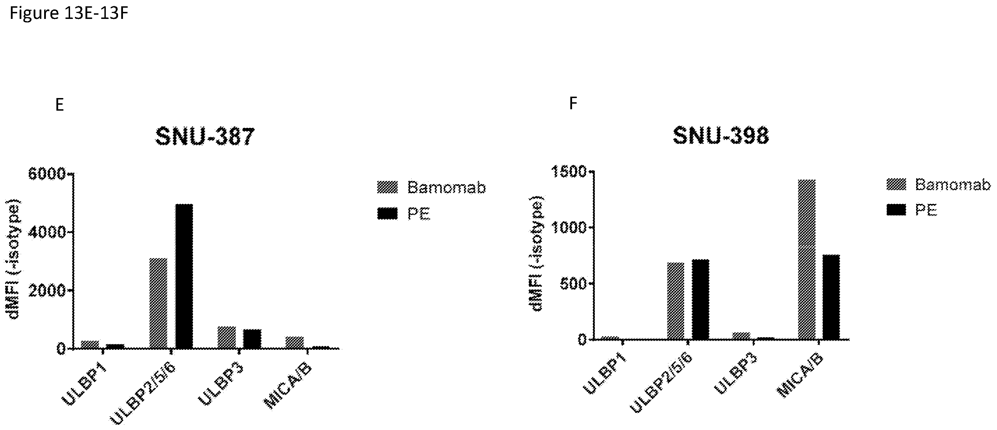

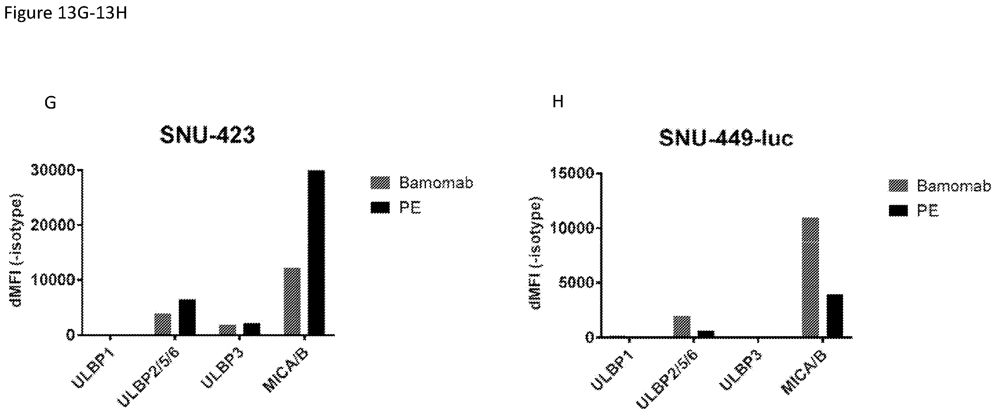

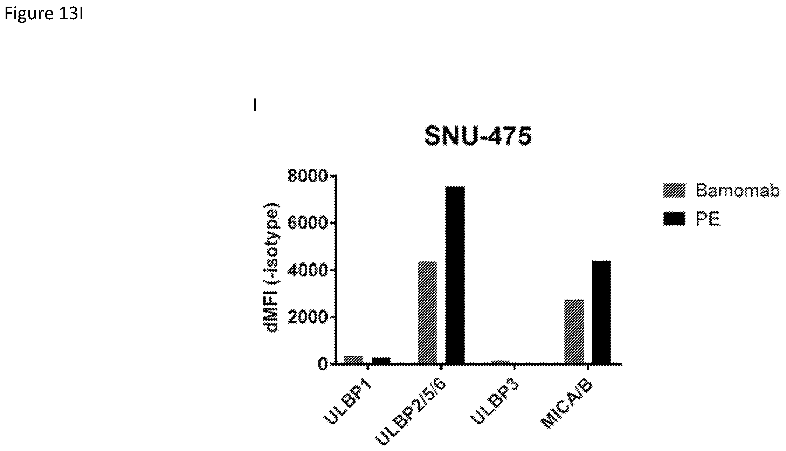

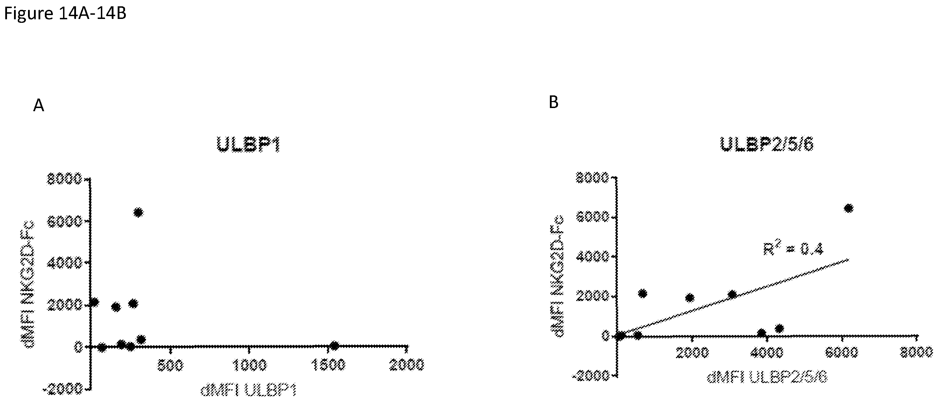

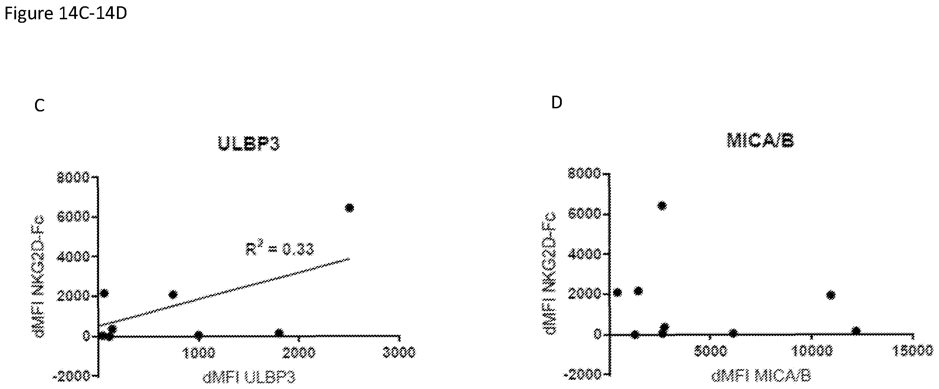

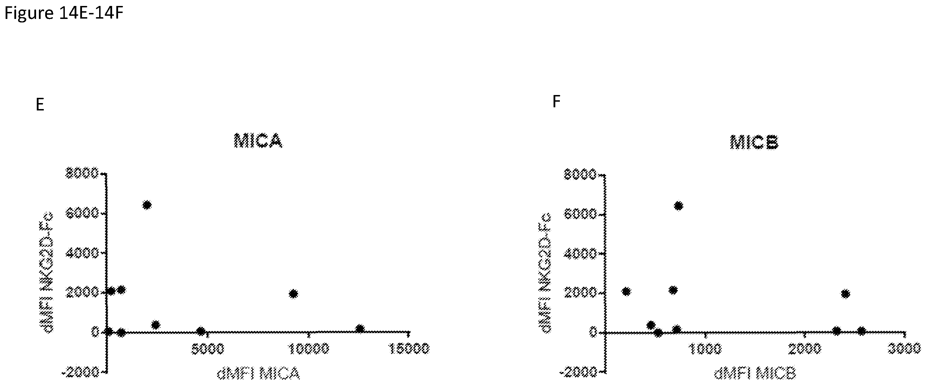

In order to determine whether the expression of any specific NKG2D ligand was dominant, an experiment was undertaken to identify a correlation between expression of each individual NKG2D ligand as compared to all NKG2D ligands as a whole. This was accomplished by evaluating the binding of an NKG2D-Fc fusion antibody that is capable of binding any NKG2D ligand to hepatocellular carcinoma cells and comparing that to the binding of NKG2D-ligandspecific antibodies to each of the nine types of hepatocellular carcinoma cells. These data were then plotted against each other and a regression analysis was performed to identify correlations and expression. These data are shown in Figures 14A-14F, with expression of total NKG2D ligands on the Y-axis and the specific indicated NKG2D ligand on the X-axis. Across each of the six specific NKG2D ligands, as compared to total NKG2D ligand expression, no significant correlation was identified. This seems consistent with the dMFI values from Figures 12 and 13, and seems to indicate a relatively high degree of heterogeneity amongst various tumor cell types. Advantageously however the engineered NK cells as disclosed herein utilize an NKG2D ligand binding domain that is capable of binding any of the NKG2D ligands. Thus, as opposed to a ligand specific approach, the more broad-based approach enables therapies using the engineered NK cells disclosed herein to successfully target and eliminate tumor cells, even when only one particular marker is expressed in a robust fashion, or even when there is no singular highly expressed marker, but rather low levels of expression of several NKG2D ligands.

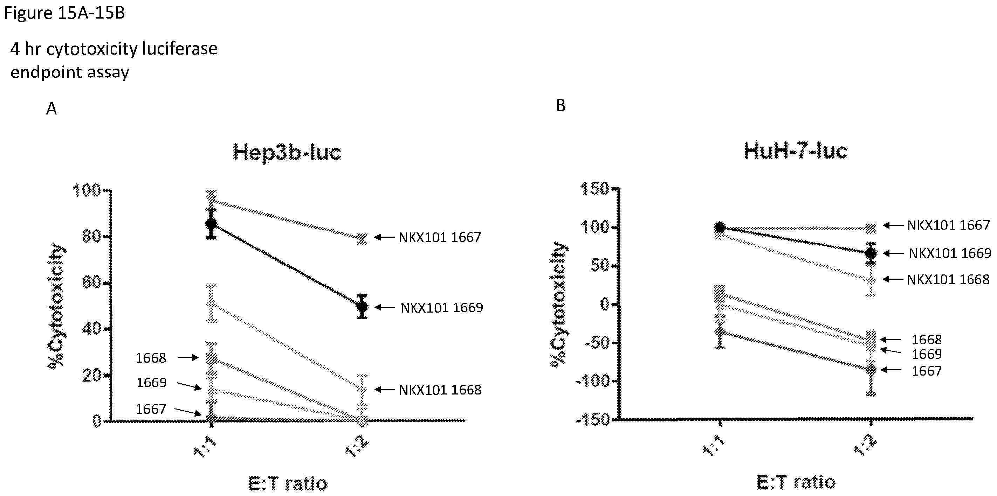

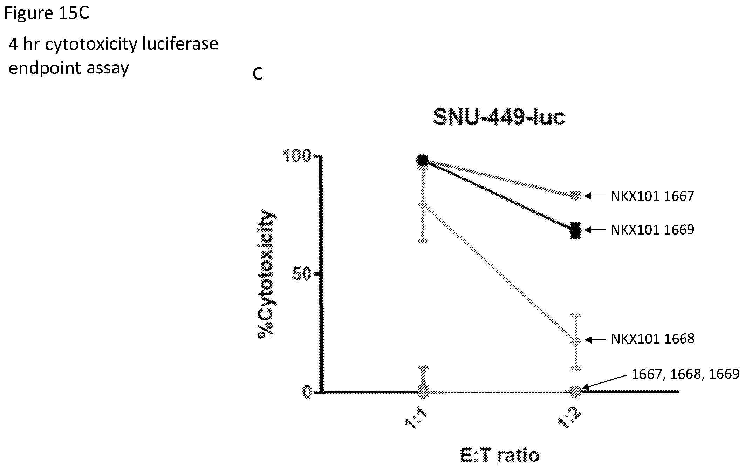

Figured 15A-15C show data related to the cytotoxicity of engineered NK cells against three hepatocellular carcinoma cell lines (Hep3B-luc, Huh-7-luc, and SNU-449-luc). As discussed above, each of these cell lines expressed various amounts of NKG2D ligands, but there was not a strong correlation indicating that any of these cell lines express a "dominant" marker. The data of Figures 15A-15C (using engineered NK cells from the indicated donors or non-transduced NK cells from the corresponding donor. Donors 1667 and 1668 have data from other prior experiments shown in earlier figures). Figures 15A-15C show results from a 4 hour cytotoxicity endpoint assay at E:T ratios of 1:1 and 1:2. As shown in each of Figures 15A-15C, the engineered NK cells from each donor exhibited enhanced cytotoxicity as compared to the corresponding non-transduced NK cells at both effector:target ratio is tested. For example, as shown in Figure 15A, at an E:T of 1:2, the non-transduced NK cells from all three donors showed essentially no cytotoxic effect against Hep3B cells. While the engineered NK cells from one donor showed up to about 20% cytotoxicity (NKX101 1668), cells from the other two donors exhibited about 50% and about 80% cytotoxicity (NKX101 1669 and NKX101 1667, respectively). At an E:T ratio of 1:1, the non-transduced NK cells showed various degrees of cytotoxicity, with the most efficacious cells achieving approximately 25% cytotoxicity. With the engineered NK cells, cytotoxicity was at least 50% (NKX101 1668), with cells from one of the remaining donors exhibiting over 85% cytotoxicity (NKX101 1169) and the other showing nearly 100% cytotoxicity (NKX 1667). Similar data pattern is shown for the cytotoxicity of the respective NK cells against the Huh-7 cell line (Figure 15B), except in this instance each of the engineered NK cells exhibited nearly 100% cytotoxicity against the hepatocellular carcinoma line. Likewise, similar data is shown in Figure 15C. Taken together, and in conjunction with the expression data showing variable NKG2D ligand expression across hepatocellular carcinoma cell lines, these data indicate that engineered NK cells according to embodiments disclosed herein still exhibit significant degree of cytotoxicity, despite the variability in ligand expression across target cells. These data therefore suggest that the engineered NK cells according to several embodiments disclosed herein should be effective against the anticipated heterogeneous tumor profiles that would be experienced across hepatocellular carcinoma patient population.

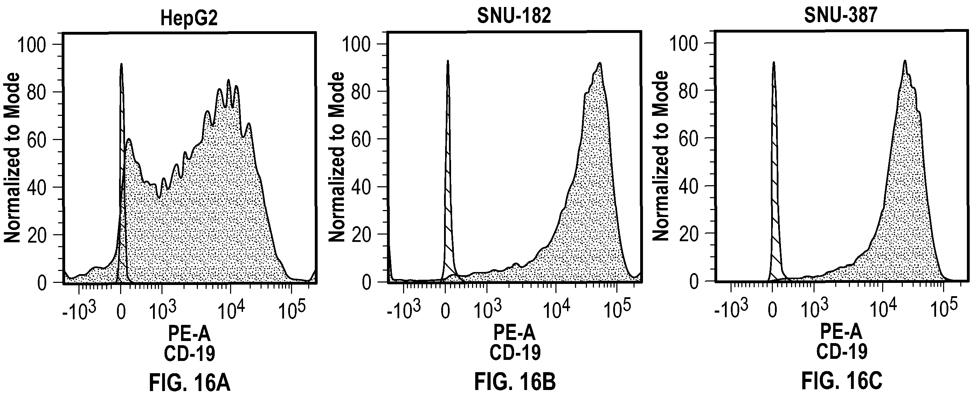

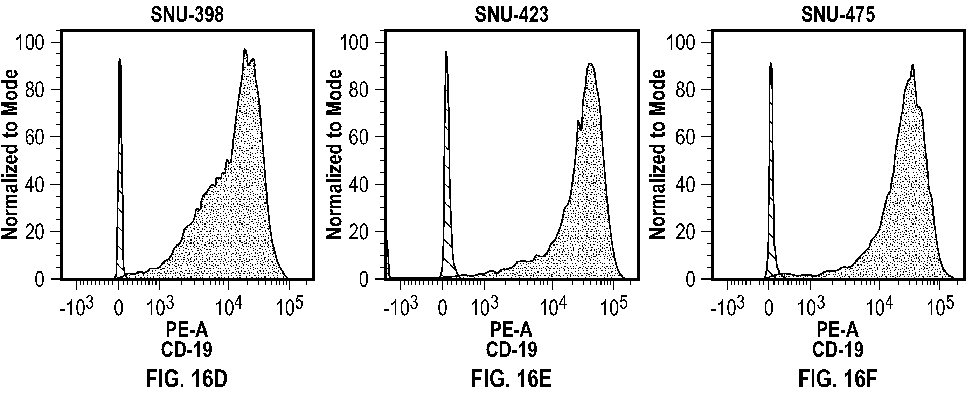

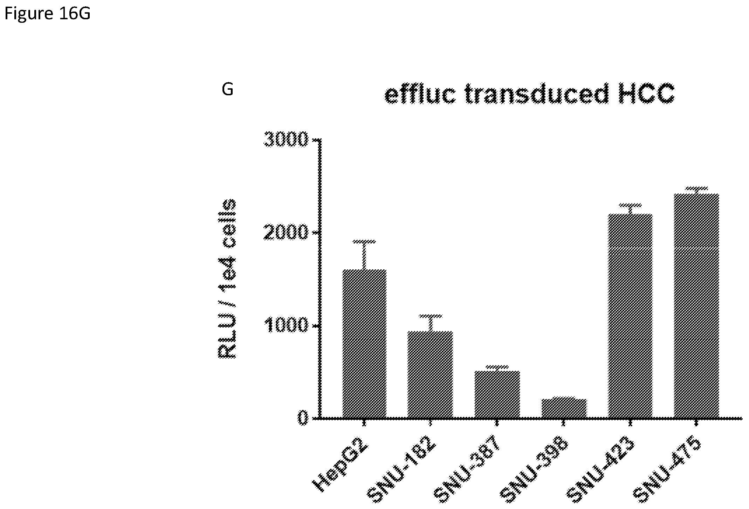

Figures 16A-16G relates to data collected when the indicated hepatocellular carcinoma lines were engineered to express luciferase, as is used to collect data in prior figures. Each of the indicated cell lines was transduced with a vector encoding the firefly luciferase gene as well as a CD19 marker that could be used to select for effectively transduced cells. Figures 16A-16F show flow cytometry data related to the degree of expression of CD19 of each of the engineered cell lines after magnetic activated cell sorting was used to select for transduced cells. Figure 16G depicts data related to the Relative Light Unit signal detected for each of cell lines engineered to express firefly luciferase.

To investigate a possible mechanism of action regarding the sensitivity of hepatocellular carcinoma cells to sorafenib, the expression of the total population of NKG2D ligands on each cell line was measured in response to various doses of sorafenib. The total population of NKG2D ligands was detected through the use of the NKG2D - Fc chimeric antibody. Figures 17A-17F show the flow cytometry data relating to NKG2D ligand expression. Figures 17G-17L show the corresponding flow cytometry data for expression of programmed death-ligand 1 (PD-L1) by the indicated hepatocellular carcinoma cells. Figures 17M and 17N are line plots that show the dose-response to the indicated doses of sorafenib in each of the cell lines tested. The data shown in Figure17M indicate that, at least at the doses tested, and the level of total NKG2D expression, sorafenib does not appear to have a substantial impact on the NKG2D ligand expression. Individual NKG2D ligands may have greater or lesser degrees of responsiveness to sorafenib treatment, which would not necessarily be identifiable based on this particular assay. Similarly, Figure 17N does not reveal a clear pattern in expression change of PD-L1 in response to sorafenib, though the data appear to suggest that at certain concentrations (e.g., 10 micromolar sorafenib) many of the cell lines show an increase in PD-L1 expression.

A similar investigation was undertaken with respect to determining the impact of interferon gamma on the expression of the total pool of NKG2D ligands as well as PD-L1. Interferon gamma is one of the inflammatory cytokines secreted by NK cells once activated, and thus plays a role in the cytotoxic effect of NK cells against the target. Figures 18A-18I show the flow cytometry data related to NKG2D ligand expression in nine hepatocellular carcinoma cell lines when exposed to the indicated concentrations of interferon gamma. Figures 18J-18R show the corresponding flow cytometry data for the same concentrations of interferon gamma and their impact on the expression of PD-L1. Figures 18S and 18T are line graphs that summarize the flow cytometry data for NKG2D ligand expression (18S) and PD-L1 expression (18T). Similar to those results shown in the prior figure after sorafenib administration, there does not appear to be a clear impact of interferon gamma on the expression of the total pool of NKG2D ligands. As mentioned above, however, individual specific NKG2D ligands may be upregulated (or down regulated) in response to interferon gamma exposure. While Figure 18T does not show a uniform expression response pattern to the increased doses of interferon gamma, several cell lines seem to show a trend towards increasing PD-L1 expression with the higher doses of interferon gamma. Despite lack of a clear induction of NKG2D ligand or PD-L 1 expression by sorafenib or interferon gamma, in several embodiments a combination treatment regime of an engineered NK cell according to several embodiments disclosed herein (e.g., NKX101 cells) in conjunction with an additional anticancer agent, such as sorafenib or other agent yield a synergistically enhanced cytotoxic effect. Sorafenib and/or IFNg may, in some embodiments, be upregulating other NK activation receptors (or downregulating inhibitory receptors) other than those studied here, which is in part responsible for synergistic activity seen in various embodiments.

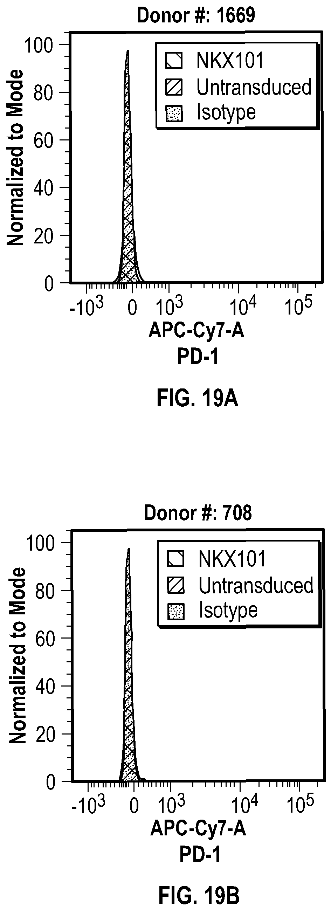

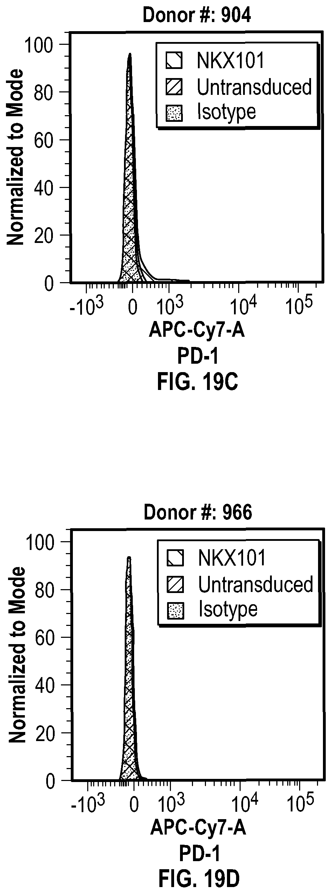

Figures 19A-19D show flow cytometry data related to expression of PD-1 by either non-transduced NK cells from four donors, engineered NK cells according to embodiments disclosed herein from those donors, or isotype control signals. Programmed Death receptor 1 (PD-1) is expressed on the surface of activated T cells, and it interacts with its ligand (PD-L1 or PD-L2) on target cells. The data for Figures 19A-19D indicate that untransduced NK cells express PD-1 levels that are not substantially distinguishable from controls, and also that the engineering of the NK cells to express a chimeric receptor as disclosed herein (e.g., NKX101) does not substantially alter the expression of PD-1. As PD-1 signaling is generally inhibitory in nature, the data suggest that induction of PD-L1 expression (discussed above) is not likely deleterious to activity of engineered NK cells expressing the chimeric constructs disclosed herein, since PD-1 expression is unchanged. In one embodiment, PD-L1 induction may inhibit an endogenous T cell response, and as such, engineered NK cells expressing chimeric receptors disclosed herein are administered in conjunction with an inhibitor of the PD-L1/PD-1 pathway.

### Colorectal Cancer

### Background:

Colorectal cancer is the third most common cancer diagnosed in both men and women in the United States. The American Cancer Society estimates for the number of new colorectal cancer cases in the United States for 2019 includes over 100,000 cases of colon cancer and over 40000 cases of rectal cancer. The liver is one of the most common sites of primary metastasis in patients with colorectal cancer. This is largely due to dissemination of the tumor through the portal circulation. While surgery can be a successful treatment for primary liver metastasis, many patients are not ideal candidates. Thus, as disclosed herein, in several embodiments, engineered NK cells can be administered, in some embodiments regionally/locally, to effectively treat liver metastases.

The experiments below relate to the investigation of engineered NK cells as disclosed herein, such as those expressing NKX101, in terms of cytotoxic activity against human colorectal carcinoma (CRC) cell line models. This includes measurement and analysis of data obtained from NKX101 in-vitro cytotoxicity and cytokine release in response to co-culture with target CRC cell lines. It also includes staining for NKG2D ligand expression on HCC cell lines for analysis of correlation with NKX101 cytotoxicity. Multiple ligands of the NKG2D receptor are highly expressed in CRC.

### Methods:

Transient retroviral supernatant was prepared by transfection of 293T cells. 293T cells were co-transfected with retroviral vectors (either encoding an NKX101 construct or luciferase with a CD19 tag (effluc.CD19)). The latter vector allows for selection of luciferase expressing cells by virtue of screening for CD19 expression.

For NK cell expansion, transduction and characterization, healthy donor buffy coat blood samples were obtained from Stanford Blood center. PBMCs were purified and banked. NK cells were expanded from banked PBMCs by co-culturing with K562-mbIL15-41BBL feeder cells at a feeder:PBMC ratio of 1: 1.

For EC50 cytotoxicity assays and cytokine analysis, day 5 expansions were depleted of T cells using anti-CD3 MACS beads.

NK cells were expanded using the modified K562 cells discussed above. The culture media was supplemented with 400 IU/mL IL2 at day 5 for 24 hr and the NK cells were transduced on day 6. In additional embodiments, other concentrations or timings are used to expand NK cells. In several embodiments, IL2 is not used, while other cytokines are used.

Surface expression of NKG2D on transduced NK cells (CD3- CD56+) was analyzed by flow cytometry using an anti-human NKG2D antibody (R&D system) at 4 days post transduction, and NK cell cultures continued at 40 IU/mL IL2. In several embodiments, other concentrations of IL2 (including no IL2) are used.

For transduction of CRC cell lines, 293FT cells were transfected with retroviral vector effluc.CD19 and viral supernatant was generated. Human HCC cell lines from ATCC (HepG2, SNU182, SNU387, SNU398, SNU423, SNU475) were transduced with effluc.CD19 viral supernatant by spinoculation and cells were seeded at a density of 2.5 x 10⁴ cells/ml (4 ml, 1e⁵/well) in a 6-well plate. Transduced cells were positively selected for CD19 cell surface expression using MACS anti-CD19 beads and CD19+ purity was analyzed by flow cytometry.

In-vitro cytotoxicity of NKX101 cells was measured against a panel of luciferase expressing CRC cell lines engineered as described above. Adherent CRC target cells were removed from cell culture flasks using Trypsin-EDTA (0.25%), counted, resuspended at 2 x 10⁵ cells/ml, and 100 ul (2 x 10⁴ cells/well) were seeded in clear 96-well U-bottom tissue culture plate. NK effector cells were counted and serially diluted (from a maximum E:T of 16: 1) two-fold for EC50 experiments. NK cells were added to wells with the CRC cells and plates were incubated for 4 hours at 37°C. After incubation, 100 ul of each cell suspension was transferred to a black-walled, clear bottom plate containing 100 ul/well Bright-Glo reagent (1:1 cell culture volume). Relative luminescence units (RLU) were read out on SpectraMax plate reader using SoftMax 6.2.2 CellTiter-Glo Luminescence assay protocol.

For NK cytokine release assays, NT NK (non-transduced NK) or NKX101 cells were co-cultured with CRC target cells at a ratio of 1:1 (1 x 10⁵ each cell type per well) in a total of 200uL media for 24 hr at 37C in a 96 well U bottom plate. At 24 hrs, plates were centrifuged at 300 g for 5 minutes, supernatants were collected and frozen at -80C until cytokine levels could be analyzed by Luminex. ProcartaPlex custom 5-plex assays were run per manufacturer protocol (Invitrogen) on Luminex MAGPIX instrument using xPONENT software.

### Results:

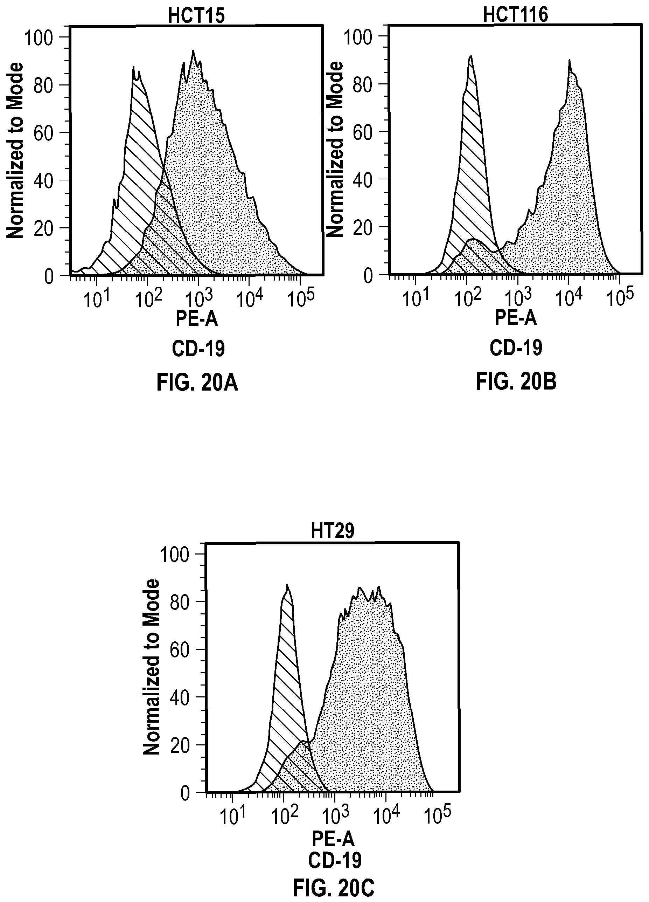

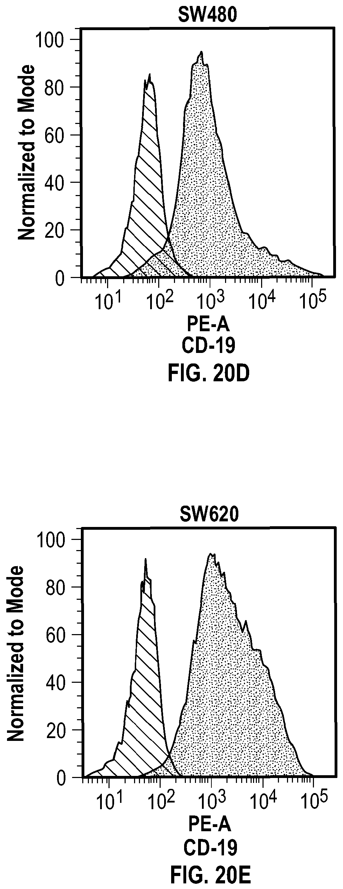

Figures 20A-20E show flow cytometry data related to engineering various colorectal cancer (CRC) cell lines to express luciferase. As described above, the vector encoding firefly luciferase also includes a CD19 marker that allows for MACS selection of cells that have been transduced. These data show staining of the resultant CRC cells after CD19 selection was performed. Data show CD19 staining (right shifted curve) as compared to the isotype control for the antibody used (left curve).

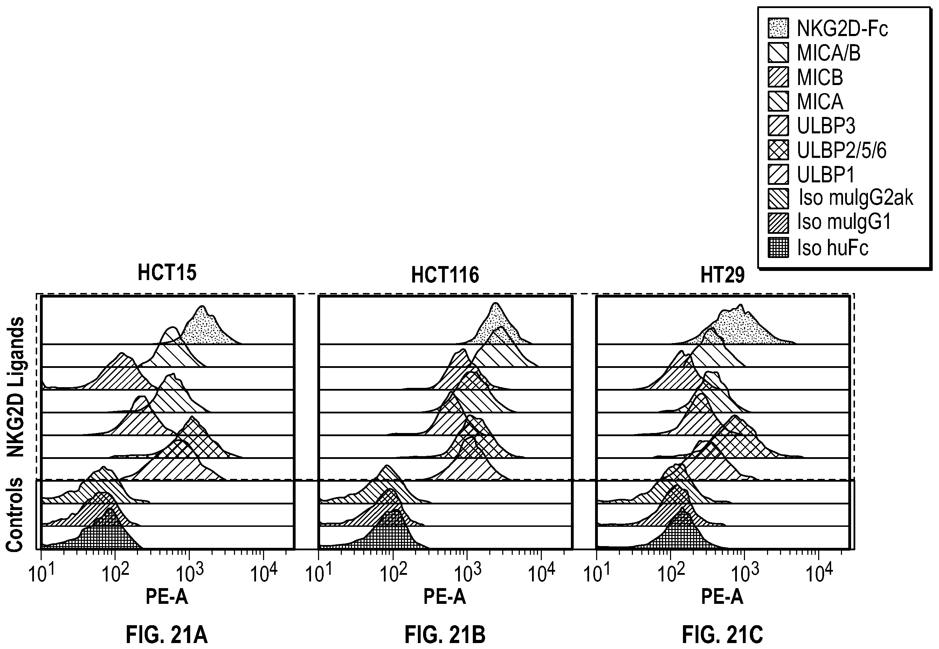

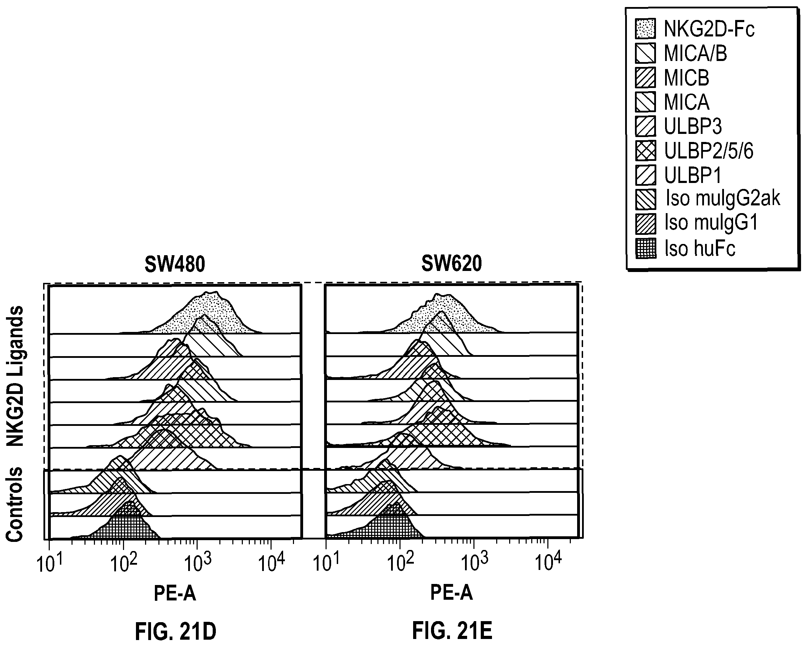

Figures 21A-21E show data related to the staining of the engineered colorectal cancer cells for either NKG2D ligands or isotype controls. NKG2D ligands are shown in the dashed box, while controls are shown in the solid box. The row labeled with NKG2D-Fc indicates the use of the NKG2D-Fc chimeric antibody that should bind all NKG2D ligands present, thus representing the total population of NKG2D ligands. Subsequent rows are staining for each specific indicated NKG2D ligand, bound with a specific antibody. In sum, these data confirm that multiple NKG2D ligands are expressed by CRC cells. According to several embodiments, the expression of the various NKG2D ligands by CRC cells allows NK cells engineered according to the disclosure herein to target and kill CRC cells, in particular those that have metastasized to the liver.

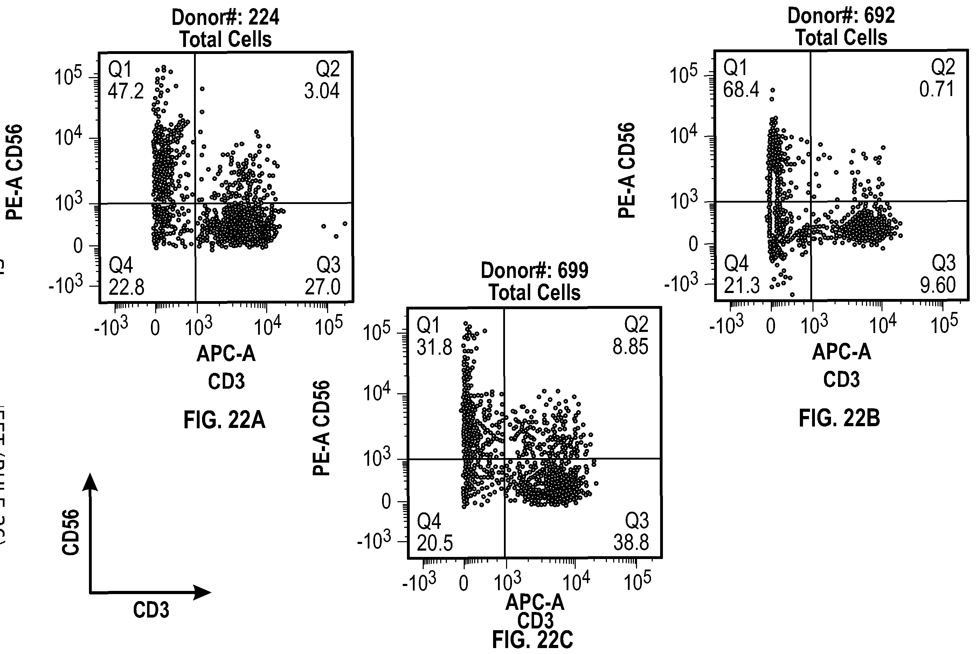

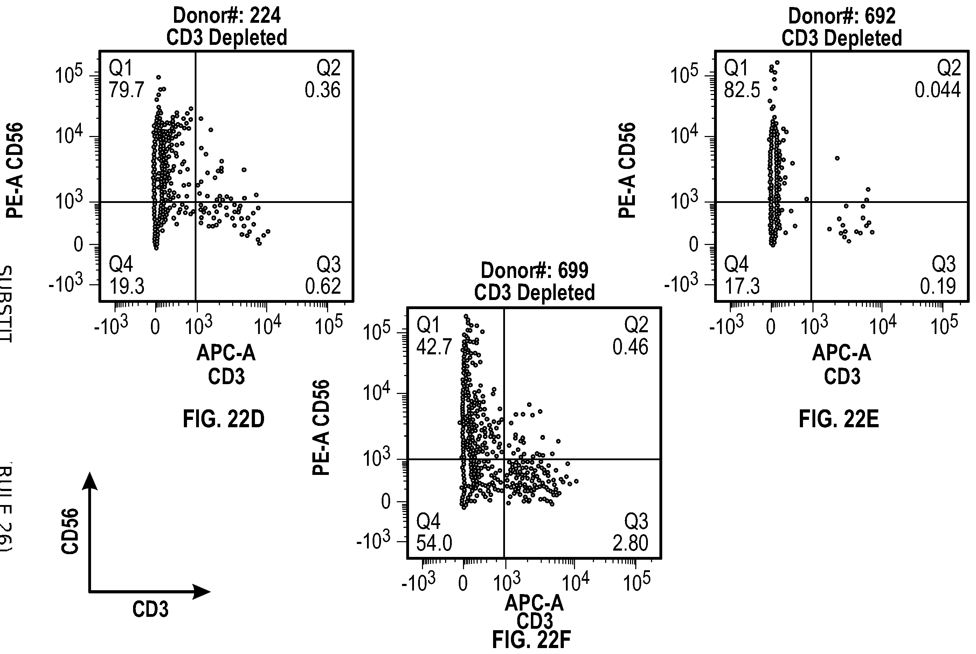

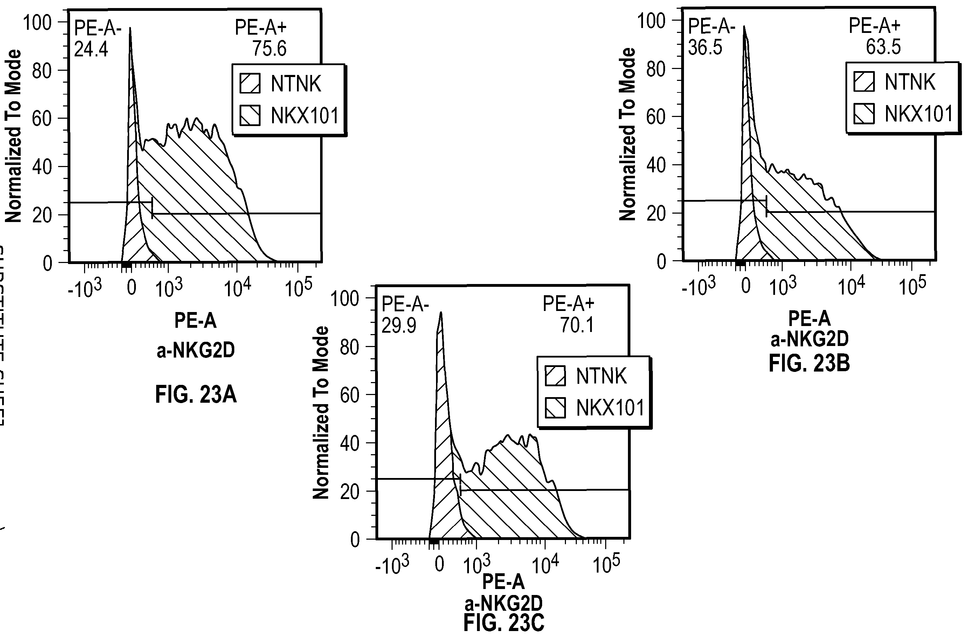

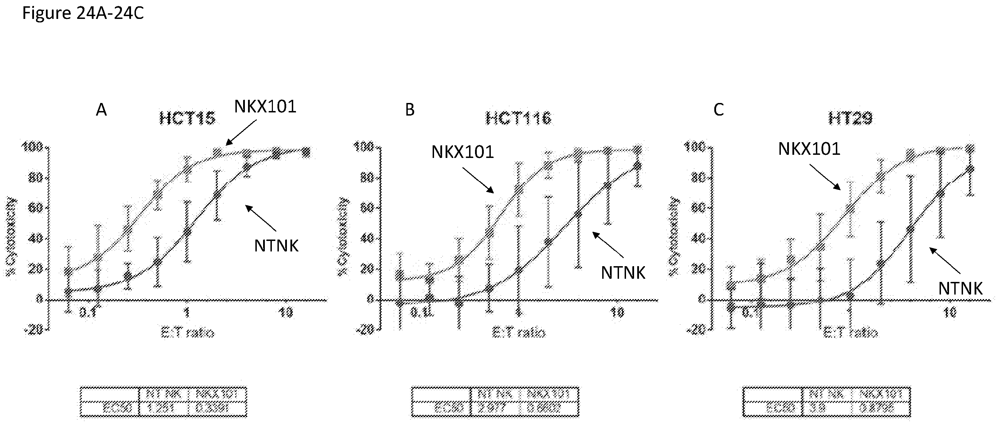

NK cells from 4 donors (224, 692, 699, 650) were expanded as described above. T cells were depleted by CD3 MACS isolation on expansion day 5, and cells were stained for CD3 and CD56 pre-CD3 depletion (Figures 22A-22C) and post- CD3 depletion (Figures 22D-22F). As discussed above, CD56 is a marker for human NK cells. Thus, the data in Figures 22D-22F indicate that the resultant population of NK cells after CD3 depletion is a highly pure NK cell population. Expanded NK cells were transduced on day 6 with a polynucleotide encoding a chimeric receptor construct as disclosed herein. By way of example, the present experiment utilized an NKG2D/OX40/CD3z cytotoxic receptor complex (see Figure 1D), though it shall be appreciated that other constructs disclosed herein can be used as well. The engineered NK cells expressing NKX101 were stained for NKG2D expression on day 4 post-transduction (Figures 23A-23C). As can be seen, as compared to control NTNK cells, NK cells engineered to express NKG2D-targeting chimeric receptors exhibit significantly higher signals for NKG2D receptor expression. These data confirm that the engineered NK cells are expressing the chimeric receptors. Cytotoxicity assays against CRC cell lines HCT15, HCT116 and HT29 were performed at day 14-21 post-expansion as described above, across an E:T ratio ranging from 16:1 down to 1:16. RLU signals were read at 4 hr endpoint, and percent cytotoxicity was used to calculate EC50 values using 4-parameter non-linear regression analysis in GraphPad Prism 7 (Figures 24A-24C). As shown, against each of the CRC cells tested in this experiment, the NK cells expressing the NKX101 chimeric receptor construct exhibited higher cytotoxicity than NTNK cells. In fact, the NKX101 cells exhibited enhanced cytotoxicity across all three cell lines at all E:T ratios (but for the similar effect at the highest E:T in HCT15 cells (Figure 24A). The calculated EC50 for NKX101 was 3-4 fold lower than for NT NK, thus reinforcing that several embodiments of engineered NK cells expressing NKG2D-directed chimeric receptors are highly effective against various tumor types.

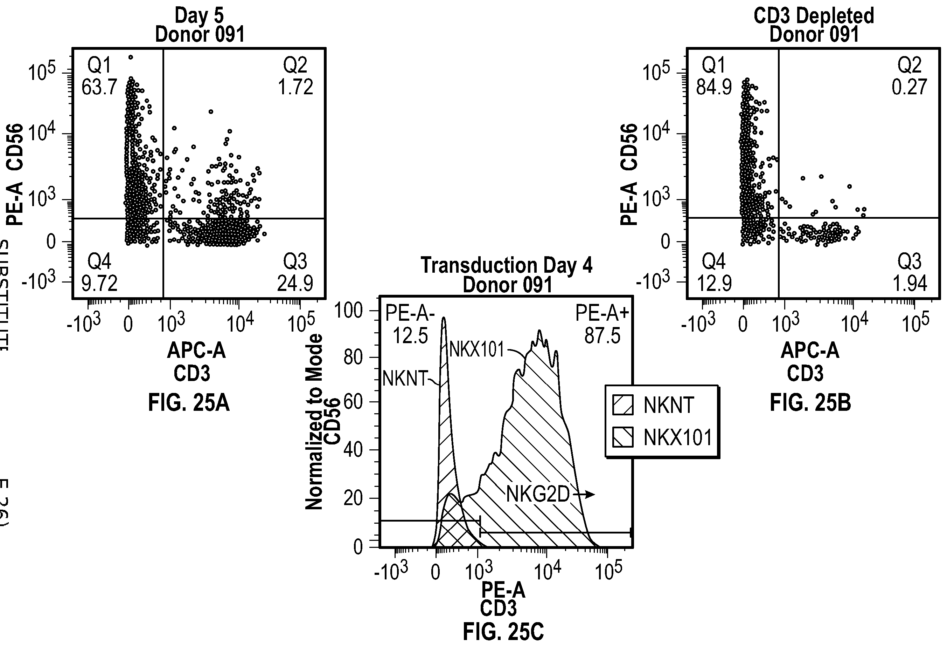

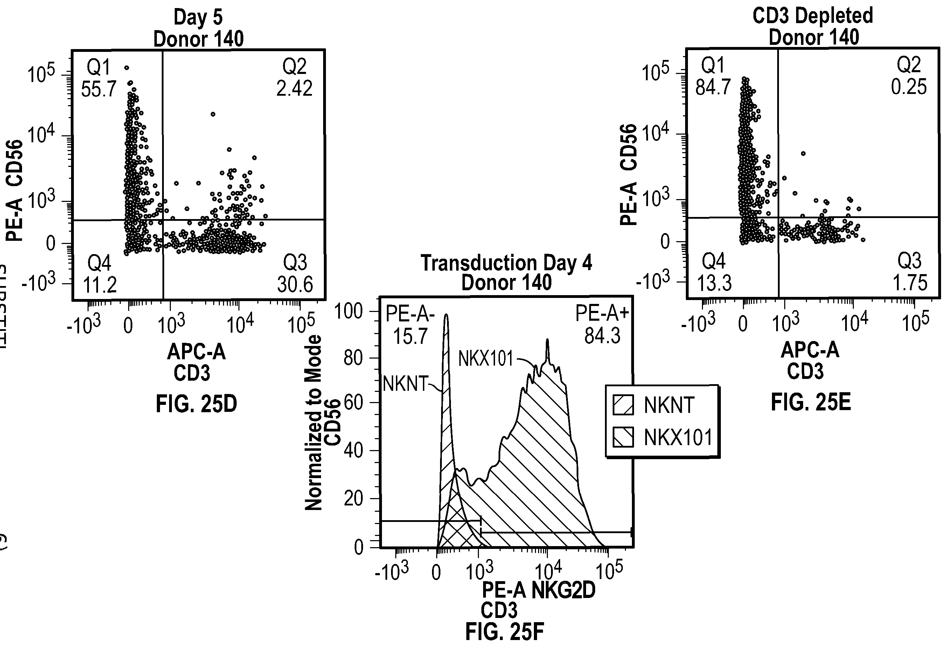

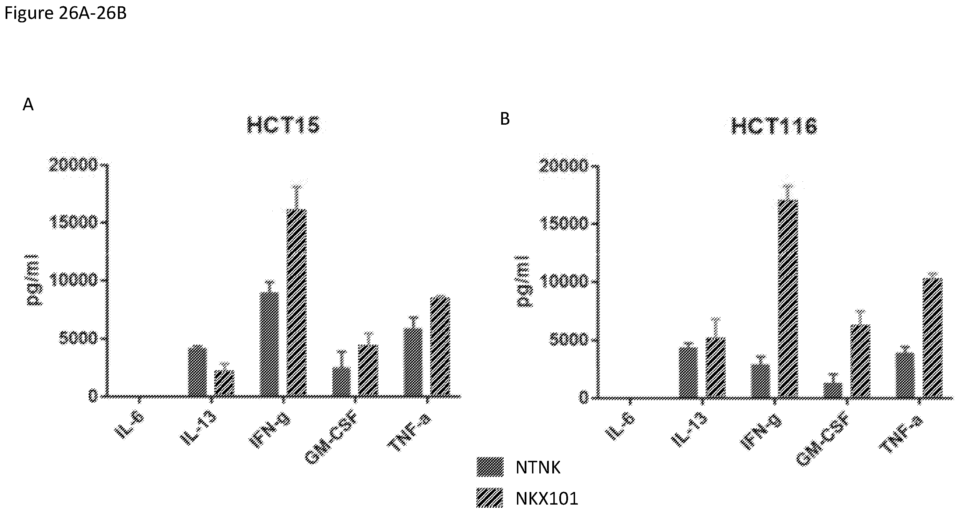

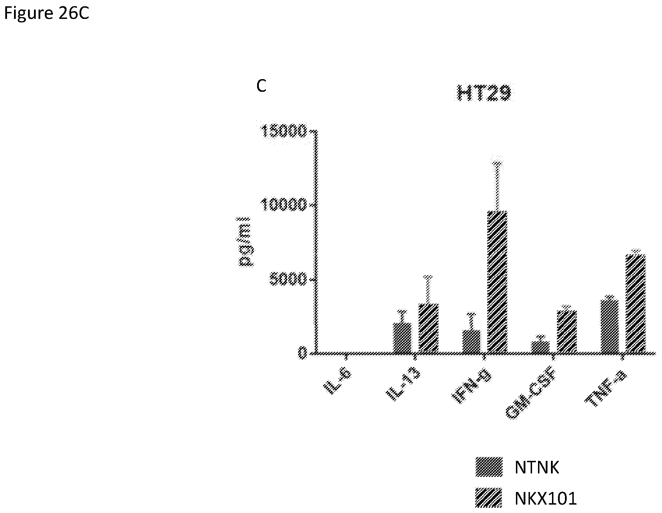

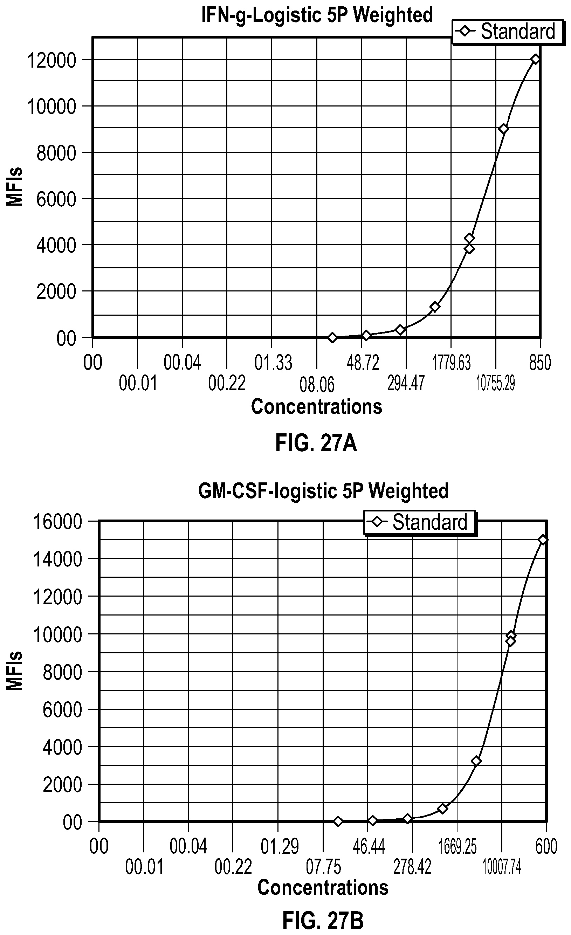

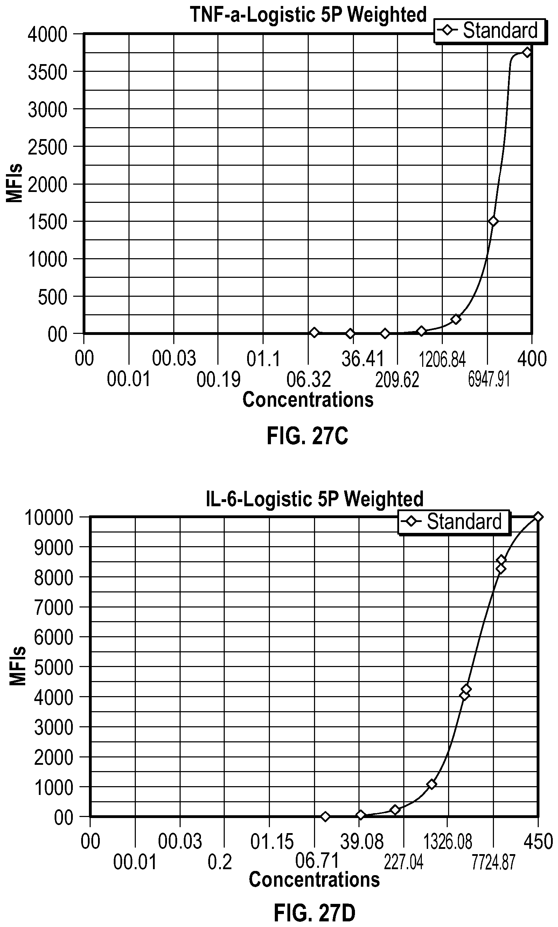

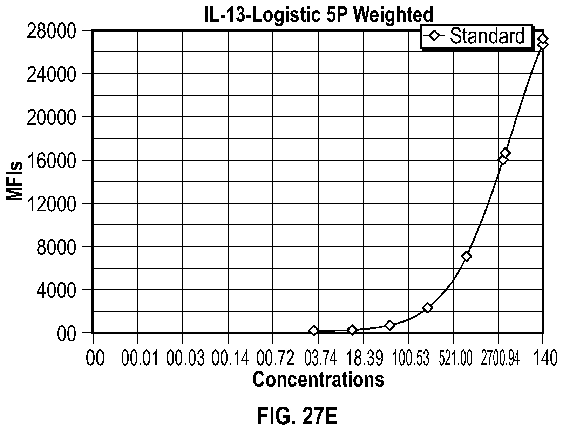

NK cells from two donors (091, 140) were expanded as described. T cells were depleted by CD3 MACS isolation on expansion day 5, and cells were stained for CD3 and CD56 pre-CD3 depletion (Figures 25A, 25D) and post-CD3 depletion (Figures 25B, 25D). Expanded NK cells were transduced on day 6 polynucleotide encoding a chimeric receptor construct as disclosed herein. By way of example, the present experiment utilized an NKG2D/OX40/CD3z cytotoxic receptor complex (see Figure 1D), though it shall be appreciated that other constructs disclosed herein can be used as well. The resulting cells were stained for NKG2D expression on day 4 post-transduction (Figures 25C, 25F). These data show that the NK cells expanded from the two donors show robust expression of NKG2D, as compared to NTNK cells. NKX101 or NT NK cells from both donors on expansion day 21 were co-cultured in-vitro with CRC target cells at a ratio of 1:1 for 24 hrs, and supernatants were analyzed for secreted cytokine levels by Luminex assay. Data for the cytokine release profile of each of the three cell lines are shown in Figures 26A-26C (standard curves for each analyte are shown in Figures 27A-27E). For each of the cell lines tested, co-culture of the CRC with NKX101-expressing NK cells induced enhanced pro-inflammatory cytokine release from the NKX101-expresing NK cells versus NT NK. For example, IFNg release by the NKX101-expressing NK cells was nearly double in the HCT15 group, with even more pronounced induction of NK cell cytokine release in the HCT116 and HT29 groups. These increased secretions of pro-inflammatory cytokines are responsible, at least in part for some embodiments, for the enhanced cytotoxicity of engineered NK cells as disclosed herein.

### Certain Characteristics of Engineered NK Cells

### Background:

To further evaluate the characteristics of the engineered NK cells as disclosed herein, additional experiments were performed to evaluate, for example, the marker profile of NK cells, ability of engineered NK cells to express embodiments of chimeric receptors disclosed herein, the cytotoxicity of such engineered NK cells and other characteristics of the engineered NK cells.

### Results:

As discussed herein, in several embodiments, a population of donor NK cells are expanded, optionally in connection with a feeder or stimulatory cell line. In several embodiments, modified K562 cells are used. Examples of such cells are provided in US Patent No. 7435596 or 8026097. In several embodiments, further modifications, such as those disclosed in PCT Application No. PCT/SG2018/050138, are used. The expanded NK cells are engineered, in several embodiments, to express a chimeric receptor and/or a molecule that promotes NK cell persistence (such as membrane bound IL15) and then optionally further expanded. According to several embodiments, the cells can then be cryopreserved and are ready for administration to a patient post-thawing. Depending on the embodiment, various degrees of expansion can be performed. For example, in some embodiments, NK cells for up to ~50 patients can be generated from a single donor. In several embodiments, NK cells for up to ~100 patients can be generated from a single donor. In several embodiments, expansion allows for still greater amounts of expansion, for example up to about 200, about 300, about 400, about 500 or more patient doses from a single donor.

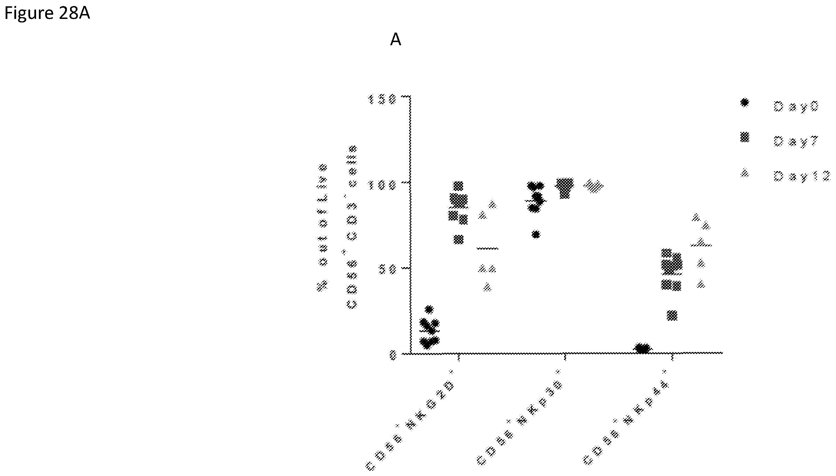

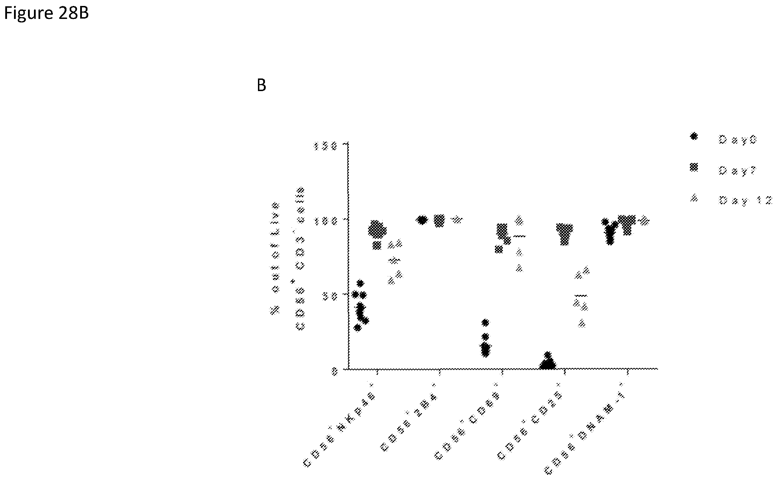

Experiments were performed to determine the characteristics of engineered NK cells according to embodiments disclosed herein. According to several embodiments, NK cells disclosed herein exhibit a broadly activated phenotype. Figure 28A shows flow cytometry expression data for NK cells at three time points during expansion (Day 0, circles; Day 7, squares, and Day 12, triangles). The Y-axis represents the percentage of the total number of live NK cells (CD56^{pos}/CD3^{neg} cells in the expansion culture). The x-axis relates to the detection of NK cells (CD56^{pos}) expressing either NKG2D (left groups), NKp30 (central groups), or NKp44 (right groups). As discussed above, each of NKG2D, NKp30 and NKp44 are activating receptors expressed by NK cells. At Day 0, only small numbers of NK cells expressed NKG2D (less than about 25%) or NKp44 (little to no expression). NKp30 expression was, in contrast, fairly high (~85%). After 7 days of expansion, however, NKG2D expression was significantly upregulated, with a mean of nearly 80% of the NK cells expressing NKG2D. Likewise, NKp44 was upregulated substantially as well, with nearly 50% of the cells expressing it. Despites its high initial expression level, even NKp30 expression was increased, now being present on nearly 100% of the NK cells. At day 12 of culture. NKG2D expression had decreased on average (still over 60%), yet still remained expressed on 80% of the NK cells from two donors. NKp44 expression continued to increase, with an average of 60% of the NK cells expressing NKp44. NKp30 was unchanged at Day 12, still present on nearly 100% of the NK cells. Figure 28B shows corresponding data for expression of NKp46, 2B4, CD69 CD25 and DNAM-1 as a percentage of the total number of NK cells. The NK cells were characterized by flow cytometry at day 0 and after 7 and 12 days after the start of expansion.NKp46, CD69 and CD25 expression were all relatively low at Day 0 (~40%, ~10%, and ~5%, respectively). In contrast, 2B4 and DNAM-1 were each highly expressed by NK cells at Day 0 (~100% and 90%, respectively). At Day 7 of expansion, each of NKp46, CD69, and CD25 exhibited significant prevalence on NK cells, each now being expressed by about 90% of the NK cells). 2B4 expression remained constant, on ~100% of NK cells, while DNAM-1 expression was increased to about 95% of NK cells. At Day 12 of expansion, NKp46 levels dropped on average to about 75% expression, and CD25 expression dropped to about 50%. CD69 expression remained high, nearly 85% of NK cells expressing it on average. 2B4 and DNAM-1 were both expressed by nearly all the NK cells. Taken together, these data shown that certain markers of NK cell activation and various activation receptors are broadly and relatively stably upregulated in expanded NK cells. According to several embodiments, expansion can be carried out for a certain time to maximize expression of a particular marker(s). In several embodiments, the broad expression of several different activation markers represents at least one of the reasons the engineered NK cells according to several embodiments disclosed herein exhibit such significant cytotoxic effects.

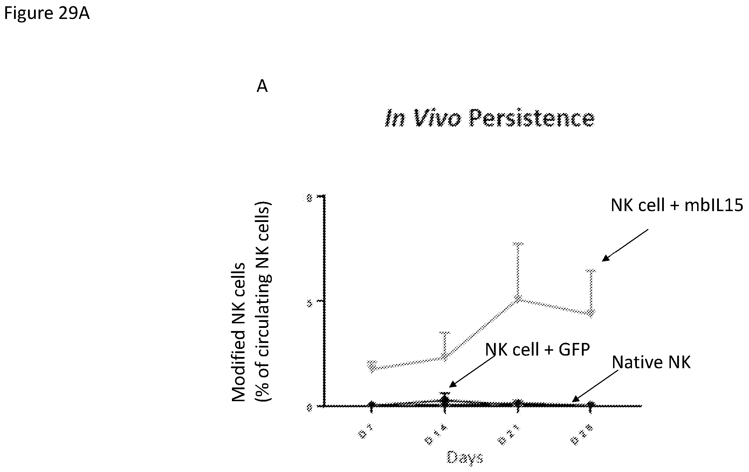

As discussed above, in several embodiments, cells are engineered to express NKG2D (or a portion thereof that binds ligands) as well as a IL15, for example as a membrane-bound form of IL-15 (mbIL-15) to enhance NK cell expansion and durability. Non-limiting information regarding membrane bound IL-15 can be found in US Patent Application No. 15/309362. In several embodiments, the expression of mbIL15 enhances the persistence of engineered NK cells. While that can be advantageous in an *in vitro* setting, according to embodiments disclosed herein, mbIL15 also enhances persistence *in vivo.* Figure 29A shows that mbIL15 expression enhances the persistence of engineered NK cells. Flow cytometry was used to measure NKG2D^{pos}/CD56^{pos}/CD3^{neg} cells in a sample of blood, with the result expressed as a percent of the total detected cells. As measured compared to the total number of circulating NK cells over time, only NK cells expressing mbIL15 as a result of expressing a non-limiting embodiment of a chimeric receptor construct (rather than GFP or non-modified NK cells) are still present in the circulation after 7 days. While the two control groups never show any increase in their numbers, mbIL 15 expressing NK cells are still present in the circulation until at least 28 days post-administration. These data indicate that , according to several embodiments, the engineered NK cells disclosed herein exhibit long-term durability, even *in vivo,* which allows for a prolonged cytotoxic effect in subject needing cancer immunotherapy treatment.

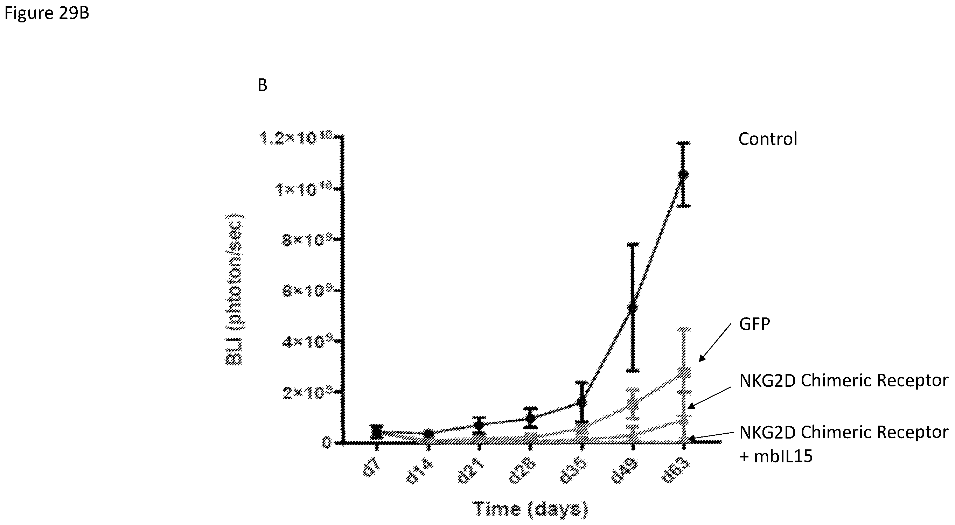

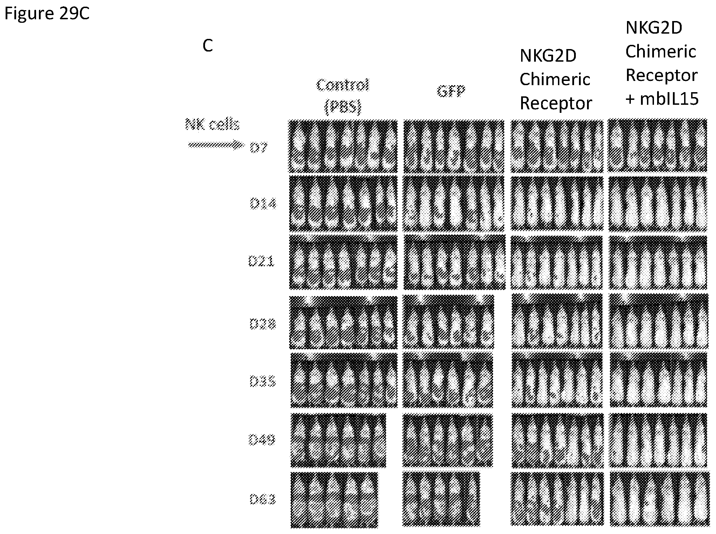

Further supporting the advantages of engineered NK cells disclosed herein, it is not only the presence of NK cells that is improved over time, but their activity as well. Figure 29B shows summary data related to the fluorescent signal detected in NSG mice after they were injected intraperitoneally with 2 x 10⁵ U2OS osteosarcoma cells. The mice received IP infusions of PBS, NK cells expressing GFP, NK cells expressing an NKG2D chimeric receptor disclosed herein (without mbIL15), or NK cells expressing an NKG2D chimeric receptor disclosed herein configured to also express mbIL15) (each NK cell infusion was 3 x 10⁶ cells, administered on Day 7). Three doses of IL2 were administered and imaging data was collected at Day 14, 21, 28, 35, 49, and 63 and are shown in Figure 29C. As shown, the mice receiving either NK cells expressing an NKG2D chimeric receptor or NK cells expressing an NKG2D chimeric receptor together with mbIL15 show significantly less tumor burden, even out to 63 days. This is in contrast to the rapidly increasing tumor burden over time in each of the two control groups (PBS and NK cells expressing GFP).

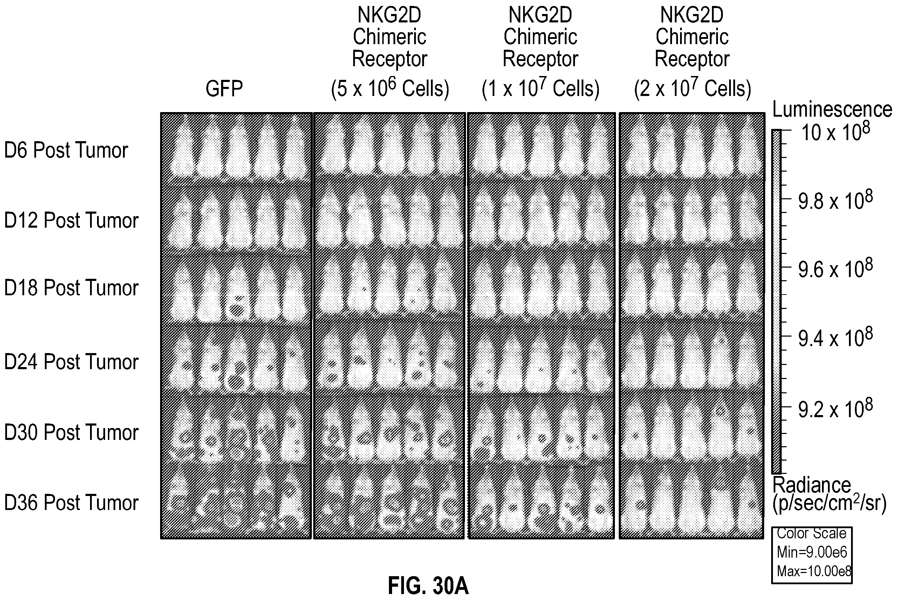

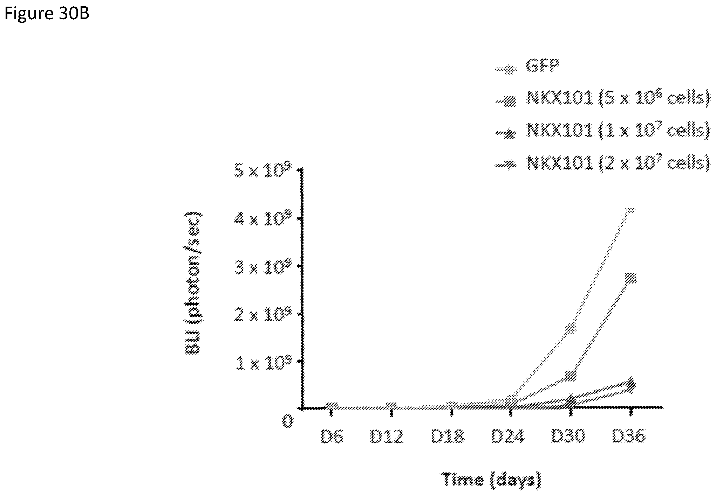

While data from experiments discussed above related to various liver tumor types or osteosarcoma cells, engineered NK cells according to several embodiments disclosed herein are effective against other tumor types as well. For example. NK cells expressing an NKG2D CAR as disclosed herein exhibit a dose dependent tumor activity in an AML model, the THP-1 xenograft model. In this model, rats were treated with a single dose of NK cells expressing an NKG2D-OX40-CD3Z-mbIL15 CAR 2 days after tumor injection. Imaging data are shown in Figure 30A and summary data are shown in Figure 30B. As with the U2OS xenograft model described above, tumor burden was significantly lower with the administration of NK cells expressing an NKG2D CAR. In addition, cytotoxic effects exhibited a clear a dose dependent response (tumor burden is decreased as cell dose increased).

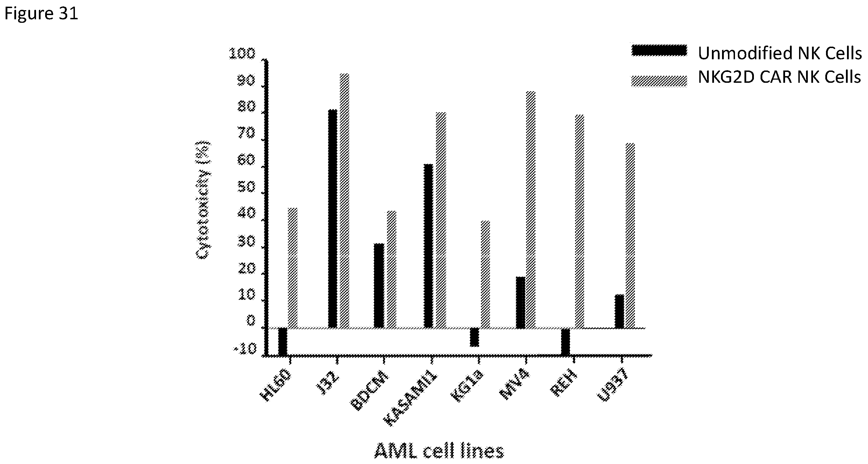

Further investigating the effectiveness of NKG2D CARs as disclosed herein, additional AML lines were tested. As shown in Figure 31, over eight different AML cell lines, an engineered NK cell expressing a chimeric receptor construct as disclosed herein yielded increased cytotoxic effects over unmodified NK cells. In some cell lines tested, the enhanced effect yielded nearly 90-95% cytotoxicity towards tumor cells, yet do not target resting PBMC. As mentioned, depending on the embodiment, a variety of engineered receptors can be used. For example in several embodiments, the activating receptor can comprise an NKG2D extracellular domain, a transmembrane domain, and a signaling domain. Each domain is optionally made up of multiple sub domains. For example, in several embodiments, the activating receptor comprises an NKG2D extracellular domain, a CD8alpha hinge region, a CD8 alpha transmembrane domain, an OX40 co-stimulatory domain, a CD3z signaling domain. In several embodiments, the polynucleotide encoding such a receptor is engineered to also express mbIL15. Other stimulatory domains can be used, such as CD28, 4-1BB, CD16, NKp80, 2B4, DAP10, DAP12, and combinations thereof. In several embodiments, various spacers, such as glycine-serine spacers are used to separate the various domains.

Advantageously, especially for allogeneic administration, NK cells can be cryopreserved with high viability, viable cell recovery and cytotoxic activity is retained upon thawing. This was assessed using the experimental protocol, schematically depicted in Figure 32A

After cryopreservation, the viability of NK cells expressing an NKG2D CAR according to embodiments disclosed here was compared to that of fresh (unfrozen) cells after 14 and 27 days of culture. Figure 32B shows that there is no substantial change in viability post-thaw - in other words engineered NK cells are still viable after cryopreservation. Importantly the percentage of viable cells was quite high post-thaw, indicating limited loss of viable cells from a given freeze-thaw cycle, meaning a greater percentage of a given batch of expanded cells can be administered post-thaw. Figure 32C shows upwards of 75% viability from 3 samples. Even after cryopreservation and thawing, engineered NK cell cytotoxicity was also retained to a degree that did not differ significantly from fresh cells (see Figure 32D). This is important because the ability of the engineered cells to exhibit anti-tumor activity is retained, which , according to several embodiments, enhances the ability to use cryopreserved cells effectively.

The expression levels of various additional markers were evaluated during expansion (in low IL2 media with characterization by flow cytometry at Day 0, 7, and 12). Figure 32E shows that expression of the inhibitory receptor CD158b on NK cells did not change significantly during expansion. In contrast, the percentage of NK cells expressing the inhibitory NKG2A receptor did increase at 7 days, but showed a small drop at 12 days of culture. The expression of the ILT2 inhibitory receptor was essentially unchanged during expansion. PD-1, a potential marker of NK cell exhaustion, did not change expression during expansion, essentially not being expressed. TIGIT and Tim3, which are markers of NK cell maturation were modestly more prevalent on NK cells as expansion progressed. Advantageously, as discussed herein, despite variable upregulation of certain inhibitory receptors, in several embodiments, activating receptors are also upregulated and allow for enhanced NK cell activity on balance.

Figure 32G shows data related to the baseline characterization of CD56 and CD16 expression on live NK cells in culture at Day 0. The two major subsets of NK cells are CD56^{high}/CD16^{dim} and CD56^{dim}/CD16^{high}. The presence/intensity of CD56 is directly correlated to the proliferative potential of an NK cell, that is, robust CD56 expression means an NK cell is configured to proliferate. This CD56 expression is associated with lower CD16 expression, which, as discussed below, is tied to cytotoxic activity. Thus CD56^{high}/CD16^{dim} are thought to proliferate and differentiate, giving rise to more cytotoxic CD56^{dim}/CD16^{high} NK cells. Figure 32G shows that the vast majority of live NK cells at Day 0 of culture are CD56^{dim}/CD16^{high} cytotoxically more potent NK cells. Figure 32H shows that there is a flux over expansion time in the CD16 and CD57 expression by NK cells, with CD16 temporarily decreasing at Day 7, then recovering to starting levels at Day 12. CD57 expression drops throughout expansion. Figure 32I shows data related to the marker intensity at Day 7 and Day 12 of culture, expressed as a ratio of that of Day 0. These data indicate that CD56 expression intensity is increased, while CD16 and CD57 expression intensity is unchanged. These data suggest that expansion according to embodiments disclosed herein trigger the engineered NK cells to "revert" or adopt a pro-proliferative phenotype. Figures 32J and 32K relate to the development of a memory NK cell like expression profile during expansion, with expression of certain memory-like markers being increased when engineered NK cells are expanded with feeder cells and additional cytokines. Further information on NK cell expansion methods can be found in US Patent No. 7,435,596 (issued October 14, 2008), 8,026,097 (issued September 27, 2011), International Patent Application No. PCT/SG2018/050138 (filed March 27, 2018) and US Provisional Patent Application No. 62/881,311 (filed July 21, 2019).

### Combination Therapies

### Background:

Without being bound by theory, cytotoxicity of the engineered NK cells in several embodiments disclosed herein can be enhanced by triggering antibody-dependent cellular cytotoxicity (ADCC). Antibodies mediate their anti-tumor effects on one hand in a direct fashion, by interfering with tumor cell growth. Antibodies may also act on tumors indirectly by inducing immune-mediated complement-dependent cytotoxicity (CDC) or ADCC. ADCC begins with recognition of an antigen expressed on the target cell surface by specific immunoglobulins. The Fc domain of these antibodies is then bound by Fcγ receptors (FcγRs) expressed on immune effector cells, such as the engineered NK cells disclosed herein, which then triggers the release of cytotoxic granules towards the target cell or upregulates death receptors expression on the cell surface.

### Results:

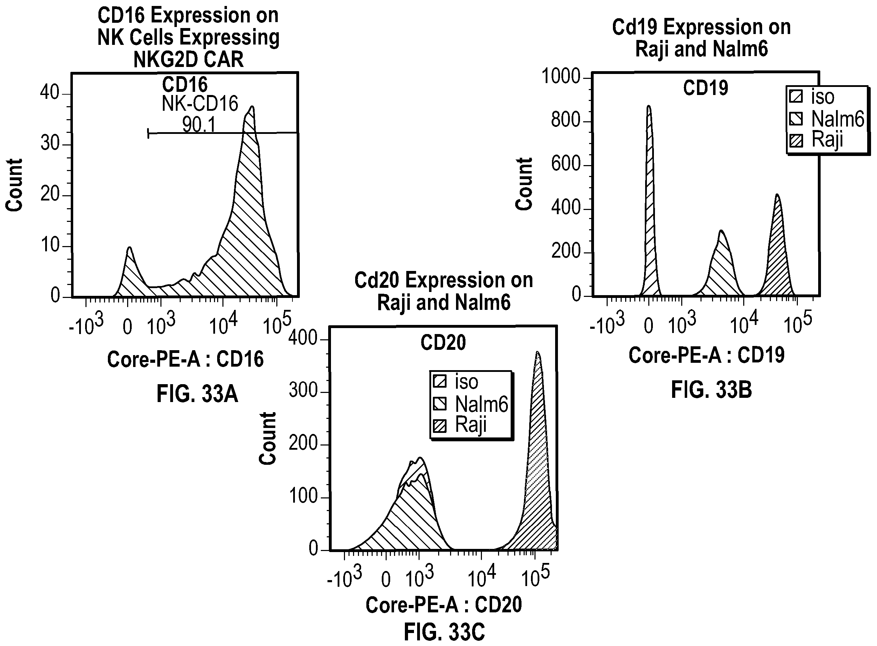

According to several embodiments, not only can the engineered NK cells bind ligands on the tumor cell surface and induce cytotoxicity through the expressed CAR, but they can also be recruited to induce further cytotoxic effects through being recruited into ADCC. With two distinct mechanisms at play, according to several embodiments, the use of engineered NK cells disclosed herein in conjunction with an antibody that can start the ADCC cascade results in synergistic anti-tumor effects. Figure 33A shows the expression of CD16 by NK cells expressing an NKG2D CAR according to a non-limiting embodiment disclosed herein (here the employed CAR was NKG2D-OX40-CD3z-mbIL15). CD16, the FcγRIIIA, is heavily involved in the recruitment of NK cells into ADCC. Interestingly, as shown in Figure 33A, 90% of the engineered NK cells expressing the NKG2D CAR also express CD16, suggesting that these engineered cells are primed for participating in ADCC. Figures 33B and 33C show data related to CD19 and CD20 expression (respectively) on Raji and Nalm6 cells. Raji cells are a B lymphocyte cell line and Nalm6 cells are a B cell precursor leukemia cell line initiated from an adolescent male. As shown in Figure 33B, both Raji and Nalm6 cells express CD19. As shown in Figure 33C, Raji cells are rich in CD20 expression, whereas Nalm6 cells are not.

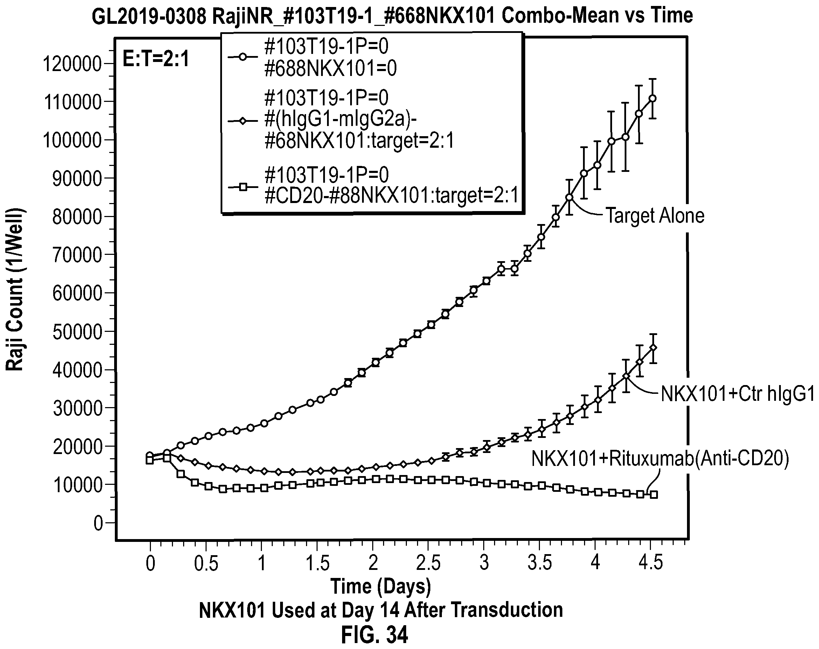

According to several embodiments, the use of a combination therapy, such as an antibody used to target cancers plus engineered cells according to embodiments disclosed herein, results in synergistic cytotoxicity. For example, in several embodiments, NK cells expressing an NKG2D CAR are co-administered with an anti-CD20 antibody (such as rituximab, by way of non-limiting example). Other embodiments employ other antibodies directed to other prevalent tumor markers, such as CD19, CD123, NY-ESO, MAGEA4, MAGEA1, PRAME, surviving, etc. Figure 34 shows data related to the expansion of Raji cells in a coculture alone, with NK cells expressing an NKG2D CAR plus a control IgG1 antibody, or with NK cells expressing an NKG2D CAR plus an anti-CD20 antibody. By way of non-limiting example, the NKG2D CAR used in this example was NKG2D-OX40-CD3z-mbIL15. Target:effector cell ratio was 2:1 and NK cells were added to the tumor cells 14 days after their transduction with the NKG2D CAR construct. As shown, the Raji cells alone increase in number over time (top "target alone" trace). When the Raji cells were exposed to NK cells expressing an NKG2D CAR according to embodiments disclosed herein and an IgG1, there was significant prohibition of Raji cell growth. While there was a trend towards increasing numbers at 4.5 days, tumor growth was clearly retarded as compared to control. The combination of NK cells expressing an NKG2D CAR according to embodiments disclosed herein and an anti-CD20 antibody appeared to prohibit all Raji cell growth. As discussed above, the phenotype of the engineered NK cells disclosed herein indicates their possible involvement in ADCC. As seen in this experiment, and according to several embodiments, the combination of NKG2D ligand binding and firing of the NKG2D CAR signaling domain and ADCC resulted in unexpectedly enhanced cytotoxicity.

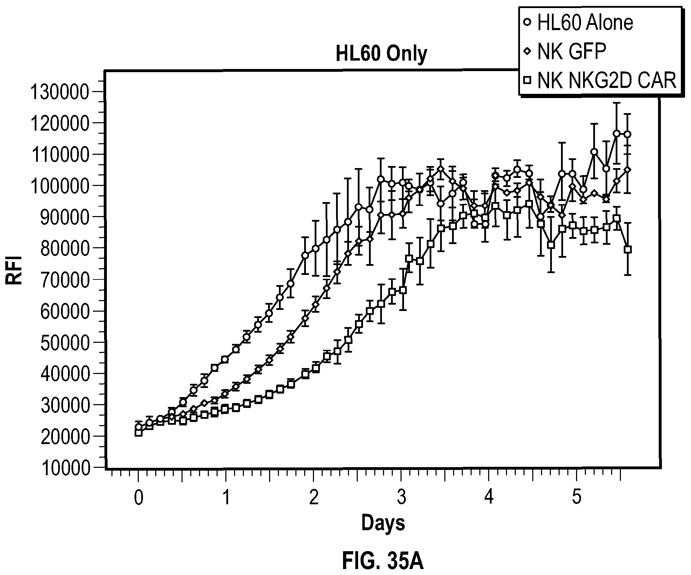

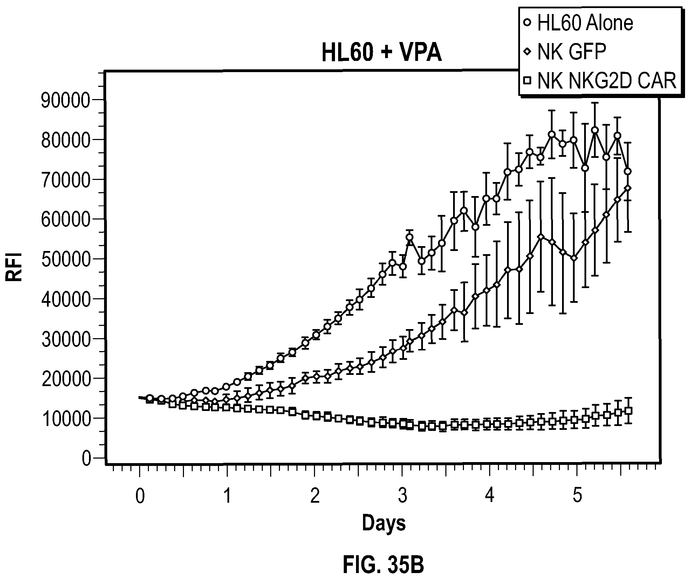

As discussed above, additional agents can be co-administered with engineered NK cells disclosed herein. For example, in several embodiments, histone deacetylase inhibitors are administered with engineered NK cells targeting an NKG2D ligand (or other tumor marker, such as CD123 or CD19). The leukemia cell line was grown alone as a control, co-cultured with NK cells expressing GFP, or co-cultured with NK cells expressing an NKG2D CAR as disclosed herein. By way of non-limiting example, the NKG2D CAR used in this example was NKG2D-OX40-CD3z-mbIL15. Figure 35A shows the increase in HL60 cell number and a modest reduction in that rate with NK GFP cells. Cellular growth was further reduced when HL60 cells were treated with NK cells expressing an NKG2D CAR. Figure 35B shows the same treatment groups, with the addition of the HDAC inhibitor valproic acid to the culture. As seen, the addition of valproic acid synergistically interacted with the NK cells expressing the NKG2D CAR dramatically suppress HL-60 cell growth. According to additional embodiments, other HDAC inhibitors are used, such as romidepsin (e.g. FR901228), entinostat (e.g., MS-275) phenylbutyrate, belinostat (PDX101), sodium valproate, suberoylanilide hydroxamic acid, or combinations thereof.

As mentioned above, in some embodiments NK cells are used. In some embodiments, T cells are used. In still additional embodiments, combinations of NK cells and T cells are used. In several embodiments, such a combination, wherein the NK and T cells are engineered to express an NKG2D activating receptor, results in synergistic cytotoxicity.

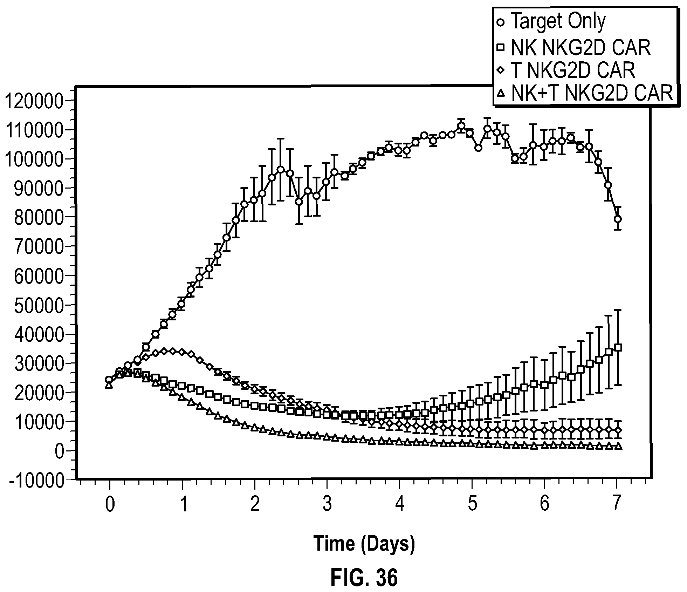

NK, T or NK + T cells were transduced with NK cells expressing an NKG2D CAR according to embodiments disclosed herein. By way of non-limiting example, the NKG2D CAR used in this example was NKG2D-OX40-CD3z-mbIL15. The cytotoxic activity of the various constructs was assessed against the HL60 AML line at a 1:4 E:T ratio (NK:T = 1: 1 ratio). Figure 36 shows the resultant data. HL60 cells increase in number when grown alone, as expected. NK cells expressing an NKG2D CAR significantly reduce the growth of HL60 cells. Likewise, T cells also decrease the growth of HL60 cells. Given the significant reduction with both NK NKGD CAR cells and T NKG2D CAR cells, it is unexpected that the combination of NK+T NKG2D CAR cells could still further reduce HL60 cell growth, effectively to zero. NK cells kill tumor cells with a rapid kinetics, but at high E:T ratios, tumor cells still proliferate. T cells kill with a slower kinetics, but expand in culture to provide more durable control. According to several embodiments, combinations of NK + T cells demonstrates a rapid onset and prolonged kinetic of cytotoxic activity, which is surprisingly advantageous, given the already high cytotoxic effects of the cell types alone. In accordance with several embodiments, a mixed NK+T cell population is used to treat tumors. In additional embodiments, either faster acting and/or longer duration effects are seen when an NK+T cell population is used with an additional agent, such as an anti-cancer antibody, kinase inhibitor, and/or and HDAC inhibitor.

It is contemplated that various combinations or subcombinations of the specific features and aspects of the embodiments disclosed above may be made.

Further, the disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another.

Moreover, while the disclosure is susceptible to various modifications, and alternative forms, specific examples thereof have been shown in the drawings and are herein described in detail. Any methods disclosed herein need not be performed in the order recited. The methods disclosed herein include certain actions taken by a practitioner; however, they can also include any third-party instruction of those actions, either expressly or by implication. For example, actions such as "administering a population of engineered NK cells locally to the liver" include "instructing the administration of a population of engineered NK cells locally to the liver." In addition, where features or aspects of the disclosure are described in terms of Markush groups, those skilled in the art will recognize that the disclosure is also thereby described in terms of any individual member or subgroup of members of the Markush group.

The ranges disclosed herein also encompass any and all overlap, sub-ranges, and combinations thereof. Language such as "up to," "at least," "greater than," "less than," "between," and the like includes the number recited. Numbers preceded by a term such as "about" or "approximately" include the recited numbers. For example, "about 90%" includes "90%." In some embodiments, at least 95% homologous includes 96%, 97%, 98%, 99%, and 100% homologous to the reference sequence. In addition, when a sequence is disclosed as "comprising" a nucleotide or amino acid sequence, such a reference shall also include, unless otherwise indicated, that the sequence "comprises", "consists of" or "consists essentially of" the recited sequence.

Any titles or subheadings used herein are for organization purposes and should not be used to limit the scope of embodiments disclosed herein.

## Claims

1. A combination therapy regimen for use in the treatment of a liver tumor, the therapy regimen comprising:
(i) a population of natural killer (NK) cells engineered to express a cytotoxic receptor complex,
wherein the cytotoxic receptor complex comprises:
a) a functional fragment of a C-type lectin-like receptor, wherein the C-type lectin-like receptor comprises a Natural Killer Group 2D (NKG2D) receptor,
wherein the functional fragment of the C-type lectin-like receptor comprises SEQ ID NO. 24; and
b) a cytotoxic signaling complex comprising a transmembrane domain, a co-stimulatory domain, and a signaling domain,
wherein the cytotoxic receptor complex comprises the amino acid sequence of SEQ ID NO: 8, and
wherein the therapy regimen is configured for the population of NK cells to be administered locally to the liver tumor;
(ii) at least one additional anti-cancer agent.

2. The combination therapy regimen for use of Claim 1, wherein the liver tumor results from hepatocellular carcinoma or wherein the liver tumor is a primary metastasis of colorectal carcinoma.

3. The combination therapy regimen for use according to any one of Claims 1 or 2, wherein the cytotoxic receptor complex is encoded by the sequence of SEQ ID NO: 7.

4. The combination therapy regimen for use according to any one of Claims 1-3, wherein the regimen is configured for the at least one additional anti-cancer agent to be administered prior to, concurrent with, or after the population of NK cells is administered.

5. The combination therapy regimen for use of any one of Claims 1-4, wherein the additional anti-cancer agent is:.
a tyrosine kinase inhibitor,
wherein the tyrosine kinase inhibitor is sorafenib, nilotinib, imantinib, gefitinib, erlotinib, sunitinib, adavosertib, lapatinib, or combinations thereof,
an antibody,
wherein the antibody is cetuximab, daratumumab, rituximab, obinutuzumab, trastuzumab, or combinations thereof,
a histone deacetylase (HDAC) inhibitor,
wherein the HDAC inhibitor is valproic acid, FR901228, MS-275, Phenylbutyrate, PDX101, Sodium valproate, Suberoylanilide hydroxamic acid, or combinations thereof,
a chemotherapeutic agent,
Cisplatin, Hydroxyurea, 5-Fluorouracil, Doxorubicin, Melphalan, Mitomycin C, Etoposide, or combinations thereof,
high dose ionizing radiation,
a proteasome inhibitor, wherein the proteasome inhibitor is optionally bortezomib,
all trans retinoic acid, sodium butyrate, or a combination thereof,
an inhibitor of a NK checkpoint pathway,
wherein the NK checkpoint inhibitor is an antagonist of CD73, an antagonist of CD39, an antagonist of an adenosine immunosuppressive signaling pathway, or combinations thereof,
an antibody directed to TIM3 and/or a TGF beta receptor, and/or
an inhibitor of indoleamine 2,3-dioxygenase

6. The combination therapy regimen for use according to any one of Claims 1-5, wherein the additional anti-cancer agent is tyrosine kinase inhibitor.

7. The combination therapy regimen for use according to any one of Claims 1-6, wherein the additional anti-cancer agent is sorafenib.

8. The combination therapy regimen for use according to any one of Claims 1-7, wherein the population of NK cells is autologous with respect to a recipient of the combination therapy.

9. The combination therapy regimen for use according to any one of Claims 1-8, wherein population of NK cells is allogeneic with respect to a recipient of the combination therapy.

10. A population of natural killer (NK) cells engineered to express a cytotoxic receptor complex for use in treatment of a liver tumor, the population comprising:
a plurality of NK cells engineered to express a cytotoxic receptor complex comprising:
a) a functional fragment of a C-type lectin-like receptor, wherein the C-type lectin-like receptor comprises a Natural Killer Group 2D (NKG2D) receptor,
wherein the functional fragment of the C-type lectin-like receptor comprises SEQ ID NO. 24; and
b) a cytotoxic signaling complex comprising a transmembrane domain, a co-stimulatory domain, and a signaling domain,
wherein the cytotoxic receptor complex comprises the amino acid sequence of SEQ ID NO: 8,
wherein the population of engineered NK cells is suitable for injection into the intra-hepatic artery of the patient, and
wherein the population of engineered NK cells are optionally administered with an additional therapy.

11. The population of natural killer (NK) cells for use of Claim 10, wherein the additional therapy comprises chemotherapy.

12. The combination therapy regimen for use of any one of Claims 1-7, wherein the additional anti-cancer agent is sorafenib.

13. The combination therapy regimen for use of any one of Claims 1-7, wherein the additional anti-cancer agent is a chemotherapeutic agent, which is cisplatin, hydroxyurea, 5-fluorouracil, doxorubicin, melphalan, mitomycin C, etoposide, or combinations thereof.

## Patentansprüche

1. Kombinations-Therapieregime zur Verwendung bei der Behandlung eines Lebertumors, wobei das Therapieregime folgendes aufweist:
(i) eine Population von natürlichen Killerzellen (NK-Zellen), die so gentechnisch so verändert sind, dass sie einen zytotoxischen Rezeptorkomplex exprimieren,
wobei der zytotoxische Rezeptorkomplex aufweist:
a) ein funktionelles Fragment eines C-Typ-Lektin-ähnlichen Rezeptors, wobei der C-Typ-Lektin-ähnliche Rezeptor einen Natural Killer Group 2D (NKG2D)-Rezeptor aufweist,
wobei das funktionelle Fragment des C-Typ-Lektin-ähnlichen Rezeptors die SEQ ID NO: 24 aufweist; und
b) einen zytotoxischen Signalkomplex, der eine Transmembrandomäne, eine kostimulatorische Domäne und eine Signaldomäne aufweist,
wobei der zytotoxische Rezeptorkomplex die Aminosäuresequenz mit der SEQ ID NO: 8 aufweist, und
wobei das Therapieregime so konfiguriert ist, dass die Population von NK-Zellen lokal in den Lebertumor verabreicht wird;
(ii) mindestens ein zusätzliches Antikrebsmittel.

2. Kombinationstherapieregime zur Verwendung nach Anspruch 1, wobei der Lebertumor aus einem hepatozellulären Karzinom resultiert oder wobei der Lebertumor eine primäre Metastase eines kolorektalen Karzinoms ist.

3. Kombinationstherapiesregime zur Verwendung nach Anspruch 1 oder 2, wobei der zytotoxische Rezeptorkomplex durch die Sequenz von SEQ ID NO: 7 kodiert wird.

4. Kombinationstherapieregime zur Verwendung nach einem der Ansprüche 1 bis 3, wobei das Regime so konfiguriert ist, dass das mindestens eine zusätzliche Antikrebsmittel vor, gleichzeitig mit oder nach der Verabreichung der NK-Zellpopulation verabreicht wird.

5. Kombinationstherapieregime zur Verwendung nach einem der Ansprüche 1 bis 4, wobei das zusätzliche Antikrebsmittelist:
ein Tyrosinkinase-Inhibitor,
wobei der Tyrosinkinase-Inhibitor Sorafenib, Nilotinib, Imantinib, Gefitinib, Erlotinib, Sunitinib, Adavosertib, Lapatinib oder Kombinationen davon ist,
ein Antikörper,
wobei der Antikörper Cetuximab, Daratumumab, Rituximab, Obinutuzumab, Trastuzumab oder Kombinationen davon ist,
ein Histon-Deacetylase (HDAC)-Inhibitor,
wobei der HDAC-Inhibitor Valproinsäure, FR901228, MS-275, Phenylbutyrat, PDX101, Natriumvalproat, Suberoylanilidhydroxamsäure oder Kombinationen davon ist,
ein chemotherapeutisches Mittel,
Cisplatin, Hydroxyharnstoff, 5-Fluorouracil, Doxorubicin, Melphalan, Mitomycin C, Etoposid oder Kombinationen davon,
hochdosierte ionisierende Strahlung,
ein Proteasom-Inhibitor, wobei der Proteasom-Inhibitor optional Bortezomib ist,
All-trans-Retinsäure, Natriumbutyrat oder eine Kombination davon,
ein Inhibitor eines NK-Checkpoint-Signalwegs,
wobei der NK-Checkpoint-Inhibitor ein Antagonist von CD73, ein Antagonist von CD39, ein Antagonist eines Adenosin-Immunsuppressions-Signalwegs oder Kombinationen davon ist,
ein Antikörper, der gegen TIM3 und/oder einen TGF-beta-Rezeptor gerichtet ist, und/oder
ein Inhibitor von Indolamin-2,3-Dioxygenase.

6. Kombinationstherapieregime zur Verwendung nach einem der Ansprüche 1 bis 5, wobei das zusätzliche Antikrebsmittel ein Tyrosinkinase-Inhibitor ist.

7. Kombinationstherapieregime zur Verwendung nach einem der Ansprüche 1 bis 6, wobei das zusätzliche Antikrebsmittel Sorafenib ist.

8. Kombinationstherapieregime zur Verwendung nach einem der Ansprüche 1 bis 7, wobei die Population von NK-Zellen in Bezug auf einen Empfänger des Kombinationstherapieregimes autolog ist.

9. Kombinationstherapieregime zur Verwendung nach einem der Ansprüche 1 bis 8, wobei die Population von NK-Zellen in Bezug auf einen Empfänger des Kombinationstherapieregimes allogen ist.

10. Population von natürlichen Killerzellen (NK-Zellen), die gentechnisch so verändert sind, dass sie einen zytotoxischen Rezeptorkomplex exprimieren, zur Verwendung bei der Behandlung eines Lebertumors, wobei die Population aufweist:
eine Vielzahl von NK-Zellen, die gentechnisch so verändert sind, dass sie einen zytotoxischen Rezeptorkomplex exprimieren, der aufweist:
a) ein funktionelles Fragment eines C-Typ-Lektin-ähnlichen Rezeptors, wobei der C-Typ-Lektin-ähnliche Rezeptor einen Natural Killer Group 2D (NKG2D)-Rezeptor aufweist,
wobei das funktionelle Fragment des C-Typ-Lektin-ähnlichen Rezeptors die SEQ ID NO: 24 aufweist; und
b) einen zytotoxischen Signalkomplex, der eine Transmembrandomäne, eine kostimulatorische Domäne und eine Signaldomäne aufweist,
wobei der zytotoxische Rezeptorkomplex die Aminosäuresequenz von SEQ ID NO: 8 aufweist,
wobei die Population der gentechnisch veränderten NK-Zellen zur Injektion in die intrahepatische Arterie des Patienten geeignet ist, und
wobei die Population der gentechnisch veränderten NK-Zellen optional zusammen mit einer zusätzlichen Therapie verabreicht wird.

11. Population von natürlichen Killerzellen (NK-Zellen) zur Verwendung nach Anspruch 10, wobei die zusätzliche Therapie eine Chemotherapie umfasst.

12. Kombinationstherapieregime zur Verwendung nach einem der Ansprüche 1 bis 7, wobei das zusätzliche Antikrebsmittel Sorafenib aufweist.

13. Kombinationstherapieregime zur Verwendung nach einem der Ansprüche 1 bis 7, wobei das zusätzliche Antikrebsmittel ein Chemotherapeutikum ist, das Cisplatin, Hydroxyharnstoff, 5-Fluorouracil, Doxorubicin, Melphalan, Mitomycin C, Etoposid oder Kombinationen davon ist.

## Revendications

1. Schéma de traitement combiné pour une utilisation dans le traitement d'une tumeur du foie, le schéma de traitement comprenant :
(i) une population de cellules tueuses naturelles (NK) modifiées pour exprimer un complexe récepteur cytotoxique,
dans lequel le complexe récepteur cytotoxique comprend :
a) un fragment fonctionnel d'un récepteur de type lectine de type C, dans lequel le récepteur de type lectine de type C comprend un récepteur de groupe tueur naturel 2D (NKG2D),
dans lequel le fragment fonctionnel du récepteur de type lectine de type C comprend SEQ ID NO. 24 ; et
b) un complexe de signalisation cytotoxique comprenant un domaine transmembranaire, un domaine de co-stimulation et un domaine de signalisation,
dans lequel le complexe récepteur cytotoxique comprend la séquence d'acides aminés de SEQ ID NO : 8, et
dans lequel le schéma de traitement est conçu pour que la population de cellules NK soit administrée localement à la tumeur du foie ;
(ii) au moins un agent anticancéreux supplémentaire.

2. Schéma de traitement combiné pour une utilisation selon la revendication 1, dans lequel la tumeur du foie résulte d'un carcinome hépatocellulaire ou dans lequel la tumeur du foie est une métastase primaire d'un carcinome colorectal.

3. Schéma de traitement combiné pour une utilisation selon l'une quelconque des revendications 1 ou 2, dans lequel le complexe récepteur cytotoxique est codé par la séquence SEQ ID NO : 7.

4. Schéma de traitement combiné pour une utilisation selon l'une quelconque des revendications 1 à 3, dans lequel le schéma est conçu pour l'administration de l'au moins un agent anticancéreux supplémentaire avant, en même temps que ou après l'administration de la population de cellules NK.

5. Schéma de traitement combiné pour une utilisation selon l'une quelconque des revendications 1 à 4, dans lequel l'agent anticancéreux supplémentaire est :
un inhibiteur de tyrosine kinase,
dans lequel l'inhibiteur de tyrosine kinase est le sorafenib, le nilotinib, l'imantinib, le gefitinib, l'erlotinib, le sunitinib, l'adavosertib, le lapatinib, ou leurs combinaisons,
un anticorps,
dans lequel l'anticorps est le cetuximab, le daratumumab, le rituximab, l'obinutuzumab, le trastuzumab, ou leurs combinaisons,
un inhibiteur d'histone-désacétylase (HDAC),
dans lequel l'inhibiteur HDAC est l'acide valproïque, FR901228, MS-275, le phénylbutyrate, PDX101, le valproate de sodium, l'acide subéroylanilide hydroxamique, ou leurs combinaisons,
un agent chimiothérapeutique,
cisplatine, hydroxyurée, 5-fluorouracile, doxorubicine, melphalan, mitomycine C, étoposide, ou leurs combinaisons,
un rayonnement ionisant à forte dose,
un inhibiteur de protéasome, dans lequel l'inhibiteur de protéasome est éventuellement du bortézomib,
tout acide trans rétinoïque, du butyrate de sodium, ou leur combinaison, un inhibiteur d'une voie de point de contrôle NK,
dans lequel l'inhibiteur de point de contrôle NK est un antagoniste de CD73, un antagoniste de CD39, un antagoniste d'une voie de signalisation immunosuppressive d'adénosine, ou leurs combinaisons,
un anticorps dirigé contre TIM3 et/ou un récepteur bêta de TGF, et/ou un inhibiteur d'indoleamine 2,3-dioxygénase

6. Schéma de traitement combiné pour une utilisation selon l'une quelconque des revendications 1 à 5, dans lequel l'agent anticancéreux supplémentaire est un inhibiteur de tyrosine kinase.

7. Schéma de traitement combiné pour une utilisation selon l'une quelconque des revendications 1 à 6, dans lequel l'agent anticancéreux supplémentaire est le sorafénib.

8. Schéma de traitement combiné pour une utilisation selon l'une quelconque des revendications 1 à 7, dans lequel la population de cellules NK est autologue par rapport à un receveur du traitement combiné.

9. Schéma de traitement combiné pour une utilisation selon l'une quelconque des revendications 1 à 8, dans lequel une population de cellules NK est allogénique par rapport à un receveur du traitement combiné.

10. Population de cellules tueuses naturelles (NK) modifiées pour exprimer un complexe récepteur cytotoxique pour une utilisation dans le traitement d'une tumeur du foie, la population comprenant :
une pluralité de cellules NK modifiées pour exprimer un complexe récepteur cytotoxique comprenant :
a) un fragment fonctionnel d'un récepteur de type lectine de type C, dans lequel le récepteur de type lectine de type C comprend un récepteur de groupe tueur naturel 2D (NKG2D),
dans lequel le fragment fonctionnel du récepteur de type lectine de type C comprend SEQ ID NO. 24 ; et
b) un complexe de signalisation cytotoxique comprenant un domaine transmembranaire, un domaine de co-stimulation et un domaine de signalisation,
dans lequel le complexe récepteur cytotoxique comprend la séquence d'acides aminés de SEQ ID NO : 8,
dans lequel la population de cellules NK modifiées peut être injectée dans l'artère intrahépatique du patient, et
dans lequel la population de cellules NK modifiées est éventuellement administrée avec un traitement supplémentaire.

11. Population de cellules tueuses naturelles (NK) pour une utilisation selon la revendication 10, dans laquelle le traitement supplémentaire comprend une chimiothérapie.

12. Schéma de traitement combiné pour une utilisation selon l'une quelconque des revendications 1 à 7, dans lequel l'agent anticancéreux supplémentaire est le sorafénib.

13. Schéma de traitement combiné pour une utilisation selon l'une quelconque des revendications 1 à 7, dans lequel l'agent anticancéreux supplémentaire est un agent chimiothérapeutique, qui est cisplatine, hydroxyurée, 5-fluorouracile, doxorubicine, melphalan, mitomycine C, étoposide, ou leurs combinaisons.
